# EUROPEAN PATENT APPLICATION

(11) **EP 4 643 883 A2**
(43) Date of publication of application: **05.11.2025**
(21) Application number: 23910359.1
(22) Date of filing: 22.12.2023
(51) Int. Cl.: A61K 47/68, A61K 47/65, A61K 45/00, A61K 31/4745, A61P 35/00

(54) **ANTIBODY-DRUG CONJUGATE CONTAINING N-METHYLENE AMIDE LINKER**

(30) Priority: 29.12.2022 CN 202211743640
(71) Applicant: Hangzhou Adcoris Biopharma Co., Ltd, Hangzhou, Zhejiang 310056 (CN)
(72) Inventor: HUANG, Yunsheng, Hangzhou, Zhejiang 310056 (CN); LI, Yaowu, Hangzhou, Zhejiang 310056 (CN)
(74) Representative: Elzaburu S.L.P.
(86) International application number: PCT/CN2023/140834
(87) International publication number: WO 2024/140433

(57) **Abstract**

The present invention relates to a linking structure capable of being used for linking an antibody and a drug that is toxic to malignant cells, and an antibody-drug conjugate as represented by formula (I) formed by means of linking an antibody and a cytotoxic drug via the linking structure, wherein each group in the formula is as defined in the description. The antibody-drug conjugate has a significant anti-tumor effect.

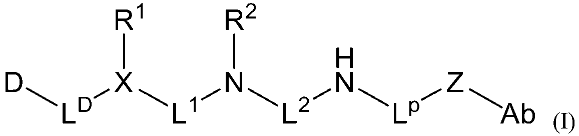

## Description

### TECHNICAL FIELD

The present disclosure relates to the biomedical field. Specifically, the present disclosure provides an antibody-drug conjugate comprising a methylene amide and analogous structural linkers thereof, preparation method thereof and use thereof.

### BACKGROUND

Antibody-targeted conjugates are a class of highly effective therapeutic drugs, and their therapeutic effects on tumor diseases are especially obvious. The conjugation of drugs and antibodies requires linkers. The role of the linkers is not only to serve as a connecting function, but more importantly to control the release mode and release rate of drugs within cells, thereby relating to the efficacy of the drugs.

Existing linker release technologies include p-aminobenzyl alcohol formate amide (PAB-carbonyl fragment), hemiaminal, cleavable peptide chains, non-cleavable peptide chains, etc. Developing more antibody-drug conjugates containing linkers cleavable within cells is of great significance.

### SUMMARY

The purpose of the present disclosure is to provide a linking structure capable of being used for linking an antibody and a drug that is toxic to malignant cells, and an antibody-drug conjugate formed by means of linking an antibody and a cytotoxic drug via the linking structure. The antibody-drug conjugate has a significant anti-tumor effect.

One aspect of the present disclosure provides an antibody-drug conjugate represented by Formula (I): 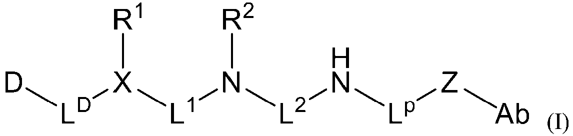 in the Formula,
D is a cytotoxic drug, Ab is an antibody;
L^{D} is selected from nonexistence or C₁-C₃ alkylene;
X is selected from N, O or S;
R¹ is selected from nonexistence, hydrogen, deuterium, C₁-C₆ alkyl or halogen-substituted C₁-C₆ alkyl;
R² is selected from hydrogen, deuterium, C₁-C₆ alkyl, C₁-C₆ alkoxyl, acyl, sulfonyl;
L¹ is selected from -L¹¹-L¹²-L¹³-; wherein, L¹¹, L¹², L¹³ are each independently selected from nonexistence, -C=O-, C₁-C₆ alkylene or -O-C₁-C₆ alkylene;
L² is selected from C₁-C₆ alkylene or C₁-C₆ acyl, the C₁-C₆ alkylene or C₁-C₆ acyl is optionally substituted with one or more R³;
R³ is selected from phenyl-substituted or unsubstituted C₁-C₆ alkyl, C₁-C₆ alkoxyl;
L^{P} is a peptide residue consisting of 2 to 7 amino acids;
Z is selected from -L^{z}-L^{j}-, wherein, L^{z} is selected from -C(=O)-C₁-C₈ alkylene or -C(=O)-(CH₂CH₂O)₂₋₆-CH₂CH₂NH-, L^{j} is a linker that can conjugate to an antibody.
In the antibody-drug conjugate represented by Formula (I), the drug antibody ratio (DAR) thereof is selected from 2-8, further can be 6-8 or 6.2-7.8, for example can be 6.2, 6.3, 6.5, 6.6, 6.8, 7.0, 7.1, 7.2, 7.3, 7.4, 7.5, 7.6, 7.7, 7.78 or 7.8.

The antibody-drug conjugate represented by Formula (I) is the antibody-drug conjugate represented by Formula (I'): 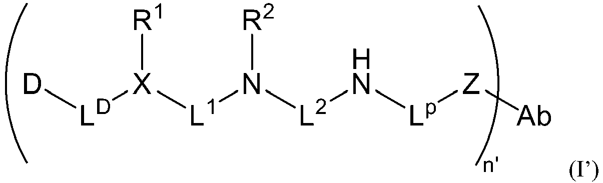 n' is selected from 2-8, further can be 6-8 or 6.2-7.8, for example can be 6.2, 6.3, 6.5, 6.6, 6.8, 7.0, 7.1, 7.2, 7.3, 7.4, 7.5, 7.6, 7.7, 7.78 or 7.8.

In the Formula, each group is defined the same as above.

In some specific embodiments, R¹ is selected from nonexistence, hydrogen, deuterium, C₁-C₃ alkyl or halogen-substituted C₁-C₃ alkyl.

In some specific embodiments, R¹ is selected from nonexistence, hydrogen, deuterium or halogen-substituted C₁-C₃ alkyl.

In some specific embodiments, R¹ is selected from nonexistence, hydrogen, deuterium or a C₁-C₃ alkyl substituted by F, Cl, Br, I.

In some specific embodiments, R¹ is selected from nonexistence, hydrogen, deuterium or F-substituted C₁-C₃ alkyl.

In some specific embodiments, R¹ is selected from nonexistence, hydrogen, deuterium, fluoromethyl, difluoromethyl, trifluoromethyl, fluoroethyl, difluoroethyl or trifluoroethyl.

In some specific embodiments, R¹ is selected from nonexistence, hydrogen, difluoroethyl or trifluoroethyl.

In some specific embodiments, R² is selected from hydrogen, deuterium, C₁-C₆ alkyl, C₁-C₆ alkoxyl, -C(=O)C₁-C₆ alkyl or -S(=O)₂C₁-C₆ alkyl.

In some specific embodiments, R² is selected from hydrogen, deuterium, C₁-C₆ alkyl, C₁-C₆ alkoxyl, -C(=O)C₁-C₆ alkyl or -S(=O)₂C₁-C₃ alkyl.

In some specific embodiments, R² is selected from hydrogen, deuterium, C₁-C₃ alkyl, C₁-C₃ alkoxyl, -C(=O)C₁-C₃ alkyl or -S(=O)₂C₁-C₃ alkyl.

In some specific embodiments, R² is selected from hydrogen, deuterium, methyl, ethyl, methoxy, ethoxy, formyl, acetyl, methanesulfonyl or ethylsulfonyl.

In some specific embodiments, R² is selected from hydrogen, methyl, methoxy, formyl or methanesulfonyl.

In some specific embodiments, R² is selected from hydrogen, methyl, formyl or methanesulfonyl.

In some specific embodiments, L¹¹, L¹², L¹³ are each independently selected from nonexistence, - C=O-, C₁-C₂ alkylene or C₁-C₂ alkylene-O-, provided that, when L¹¹ is -C=O-, R² is not hydrogen.

In some specific embodiments, L¹¹, L¹² , L¹³ are each independently selected from nonexistence, - C=O-, -CH₂- or -OCH₂-.

In some specific embodiments, L¹ is selected from -C(=O)CH₂OCH₂-, -C(=O)CH₂O- or -CH₂-.

In some specific embodiments, L² is selected from C₁-C₃ alkylene or C₁-C₃ acyl, the C₁-C₃ alkylene or C₁-C₃ acyl is optionally substituted with one or more R³.

In some specific embodiments, L² is selected from methylene, ethylene or -C(=O)CH₂-, the methylene, ethylene or -C(=O)CH₂-is optionally substituted with one or more R³.

In some specific embodiments, R³ is selected from C₁-C₆ alkyl, C₁-C₆ alkoxyl or phenyl-substituted C₁-C₆ alkyl.

In some specific embodiments, R³ is selected from C₁-C₆ alkyl or phenyl-substituted C₁-C₆ alkyl. In some specific embodiments, R³ is selected from C₁-C₄ alkyl or phenyl-substituted C₁-C₃ alkyl.

In some specific embodiments, R³ is selected from methyl, ethyl, propyl, isopropyl, butyl, isobutyl, phenylmethyl or phenylethyl.

In some specific embodiments, R³ is selected from methyl, isopropyl, isobutyl or phenylmethyl.

In some specific embodiments, L² is selected from -CH₂-, -CH₂CH₂-,

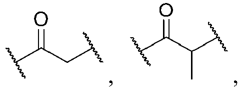

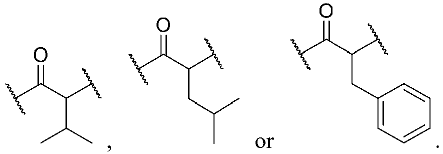

In some specific embodiments, L² is selected from -CH₂-, -CH₂CH₂-,

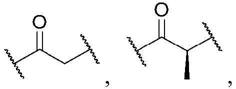

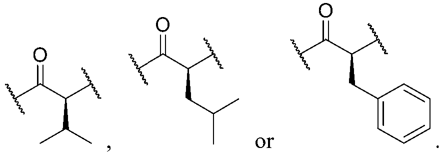

In some specific embodiments, L² is selected from -CH₂-, -CH₂CH₂-,

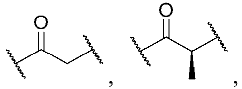

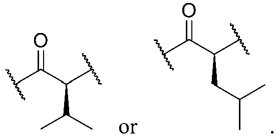

In some specific embodiments, the amino acid is selected from phenylalanine (Phe), glycine (Gly), valine (Val), alanine (Ala), leucine (Leu), lysine (Lys) or glutamate (Glu).

In some specific embodiments, the amino acid is selected from phenylalanine (Phe), glycine (Gly), valine (Val), alanine (Ala) or leucine (Leu).

In some specific embodiments, L^{p} is selected from peptide residues consisting of 2-5 amino acids selected from phenylalanine (Phe), glycine (Gly), valine (Val), alanine (Ala) or leucine (Leu).

In some specific embodiments, L^{p} is selected from peptide residues consisting of 2, 3 or 4 amino acids selected from phenylalanine (Phe), glycine (Gly), valine (Val), alanine (Ala) or leucine (Leu).

In some specific embodiments, L^{p} is selected from -Val-Cit-, -Gly-Lys-, -Gly-Leu-, -Val-Ala-, - Gly-Phe-, -GLy-Gly-Lys-, -Gly-Gly-Phe-, -Gly-Val-Ala-, -Gly-Gly-Val-, -Gly-Leu-Val-, -Gly-Phe-Gly- or -Gly-Gly-Leu-.

In some specific embodiments, L^{p} is selected from -Gly-Leu-, -Gly-Phe-, -Gly-Gly-Phe-, -Gly-Val-Ala-, -Gly-Gly-Val-, -Gly-Leu-Val-, -Gly-Phe-Gly- or -Gly-Gly-Leu-.

In some specific embodiments, L^{p} is selected from

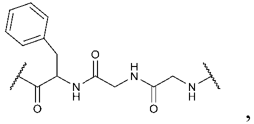

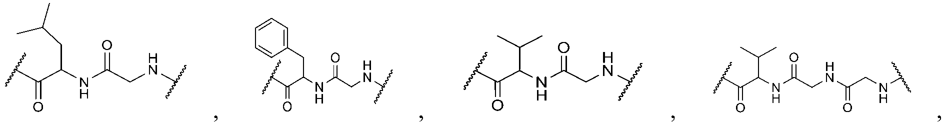

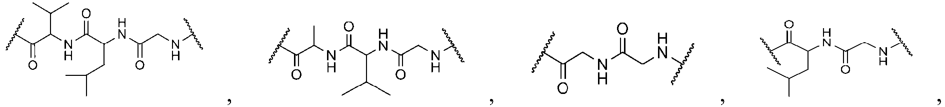

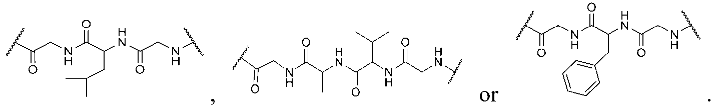

In some specific embodiments, L^{p} is selected from

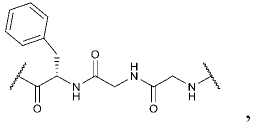

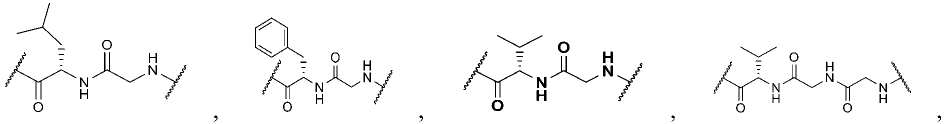

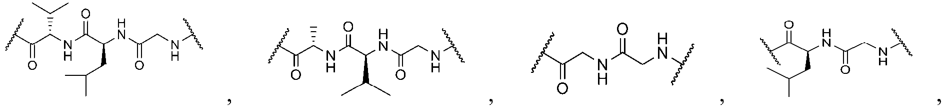

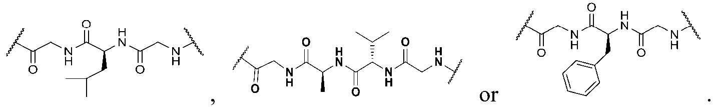

In some specific embodiments, L^{j} is selected from 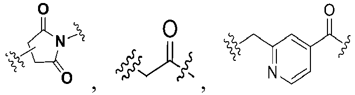 and 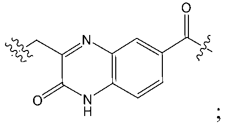 the position represented by 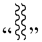 is linked to the antibody, and the position represented by 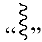 is linked to the L^{Z} group.

In some specific embodiments, L^{z} is selected from -C(=O)-C₁-C₈ alkylene or -C(=O)-(CH₂CH₂O)₂₋₆-CH₂CH₂NH-.

In some specific embodiments, L^{z} is selected from -C(=O)-(CH₂CH₂O)₂₋₆-CH₂CH₂NH-.

In some specific embodiments, L^{z} is selected from -C(=O)-(CH₂CH₂O)₂₋₄-CH₂CH₂NH-.

In some specific embodiments, L^{z} is selected from -C(=O)-(CH₂CH₂O)₂-CH₂CH₂NH-.

In some specific embodiments, Z is selected from

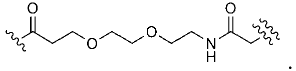

In some specific embodiments, the cytotoxic drug is selected from Camptothecin and deritives thereof.

In some specific embodiments, the cytotoxic drug is selected from

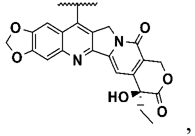

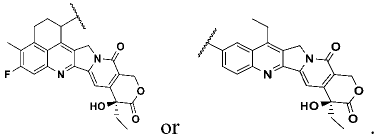

In some specific embodiments, the antibody is an antibody against tumor-associated antigen.

In some specific embodiments, the antibody against tumor-associated antigen is selected from anti-Her2 antibody, anti-Trop2 antibody, anti-B7H3 antibody, anti-5T4 antibody, anti-Nectin-4 antibody, anti-CD20 antibody, and anti-ROR1 antibody.

In some specific embodiments, the compound structure represented by Formula (I) is as represented by Formula (II) or Formula (II'): 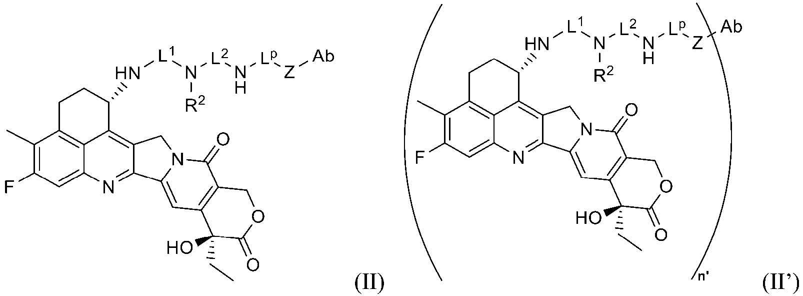 in the Formula, R², L¹, L², L^{p}, Z, n', Ab are each defined the same as the antibody-drug conjugate of Formula (I) or Formula (I').

In some specific embodiments, L¹ is selected from -C(=O)CH₂OCH₂- or -C(=O)CH₂O-.

In some specific embodiments, L² is selected from -CH₂- or -CH₂CH₂-.

In some specific embodiments, 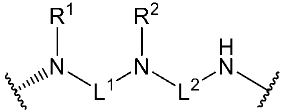 is selected from

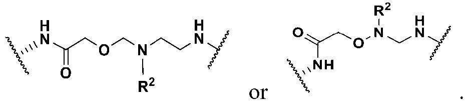

In some specific embodiments, the structure of the antibody-drug conjugate represented by Formula (I) is as represented by Formula (III) or Formula (III'): 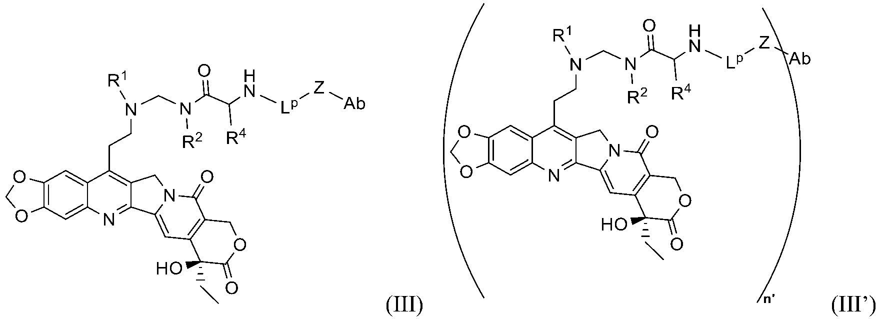 in the Formula, R¹, R², R⁴, L^{p}, Z, n', Ab are each defined the same as the antibody-drug conjugate of Formula (I) or Formula (I').

In some specific embodiments, R⁴ is selected from hydrogen, phenyl-substituted or unsubstituted C₁-C₆ alkyl, C₁-C₆ alkoxyl.

In some specific embodiments, R⁴ is selected from hydrogen, C₁-C₆ alkyl, C₁-C₆ alkoxyl or phenyl-substituted C₁-C₆ alkyl.

In some specific embodiments, R⁴ is selected from hydrogen, C₁-C₆ alkyl or phenyl-substituted C₁-C₆ alkyl.

In some specific embodiments, R⁴ is selected from hydrogen, C₁-C₄ alkyl or phenyl-substituted C₁-C₃ alkyl.

In some specific embodiments, R⁴ is selected from hydrogen, methyl, ethyl, propyl, isopropyl, butyl, isobutyl, phenylmethyl or phenylethyl.

In some specific embodiments, R⁴ is selected from hydrogen, methyl, isopropyl, isobutyl or phenylmethyl.

In some specific embodiments, R⁴ is selected from hydrogen, methyl or isopropyl.

In some specific embodiments, the structure of the antibody-drug conjugate represented by Formula (I) is as represented by Formula (IV) or Formula (IV') : 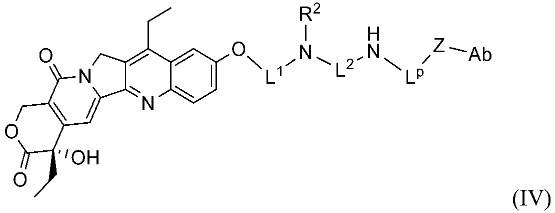 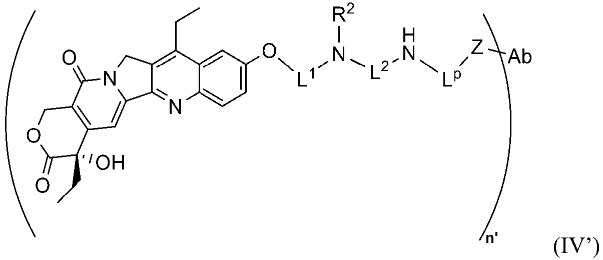 in the Formula, R², L¹, L², L^{p}, Z, n', Ab are each defined the same as the antibody-drug conjugate of Formula (I) or Formula (I').

In some specific embodiments, L¹, L² are each independently selected from C₁-C₃ alkylene.

In some specific embodiments, L¹ is -CH₂-, L² is -CH₂CH₂-.

In some specific embodiments, structure: 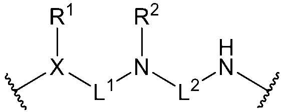 is selected from the following

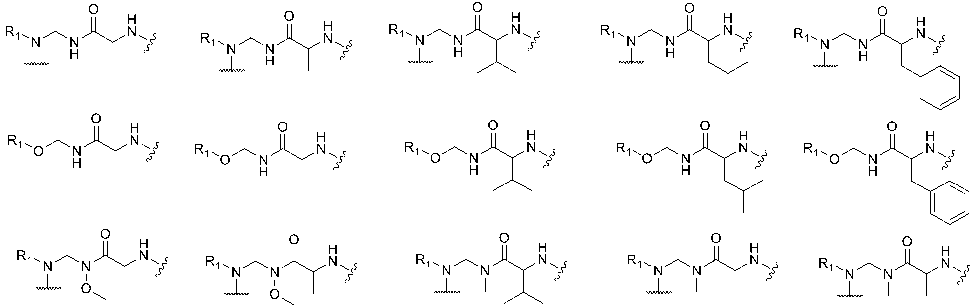

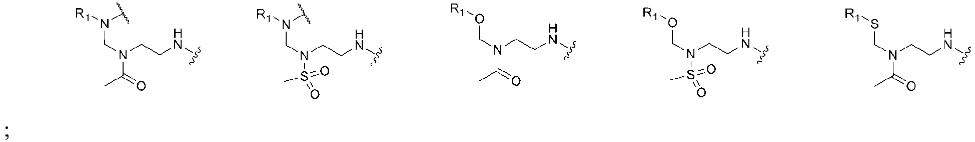

In some specific embodiments, 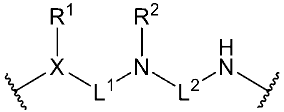 is selected from the following structure: 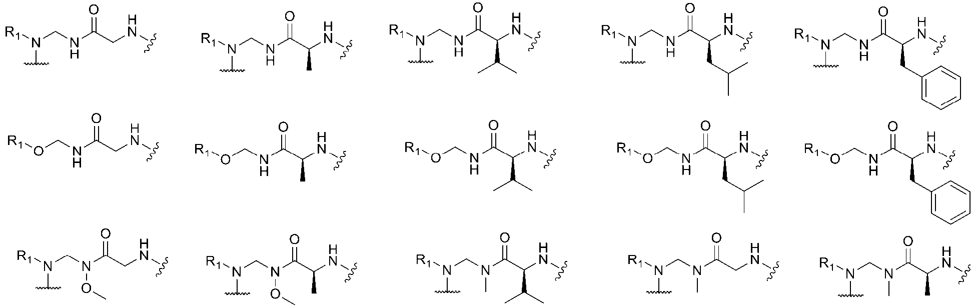 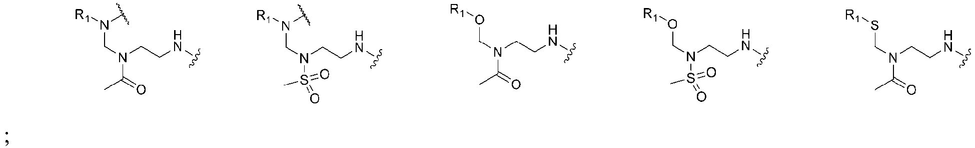 wherein, R¹ is defined the same as the antibody-drug conjugate of Formula (I).

The antibody-drug conjugate (ADC) afforded by conjugating the following compound with an antibody,

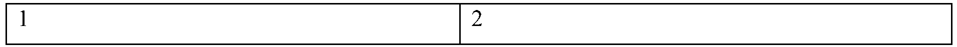

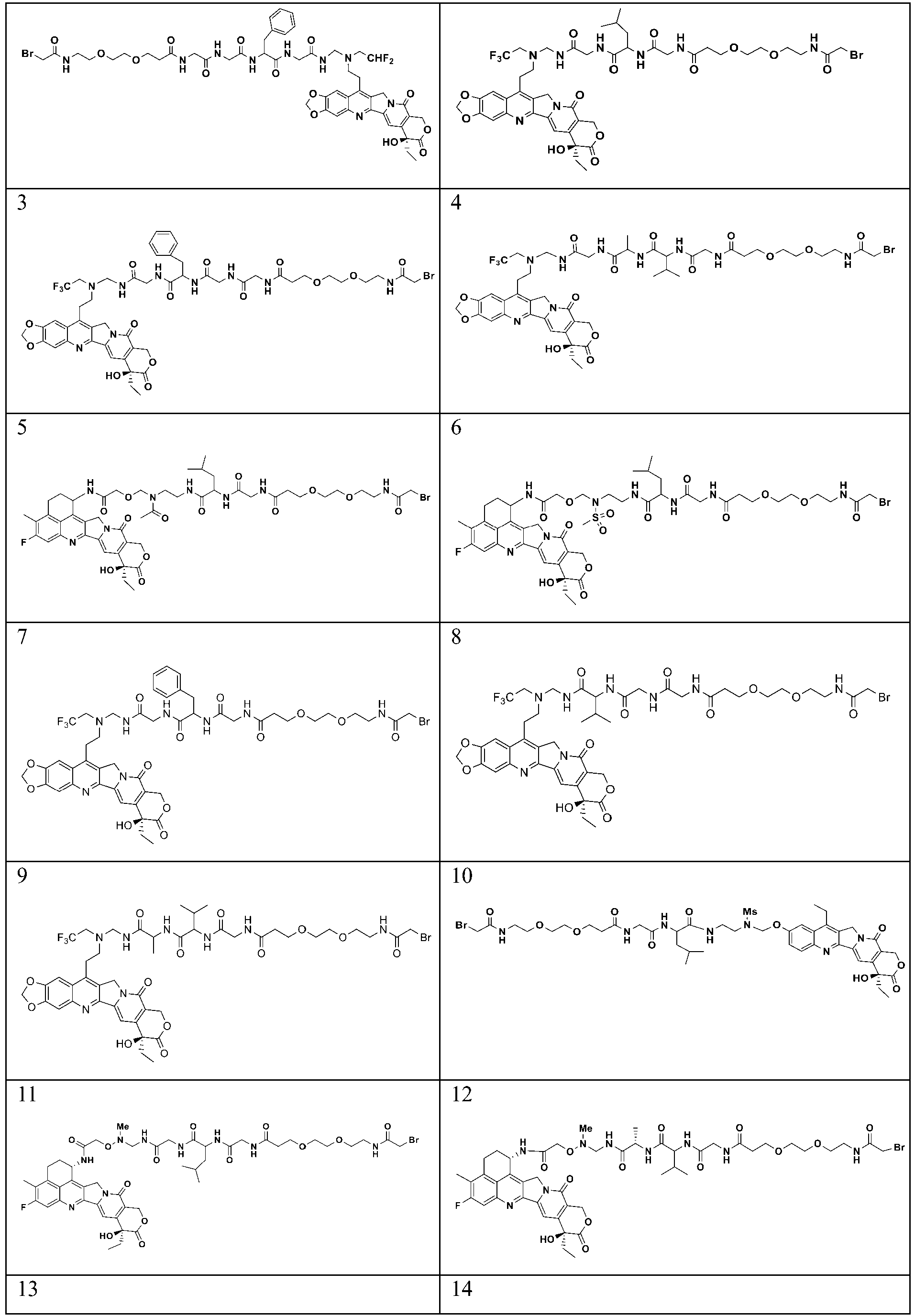

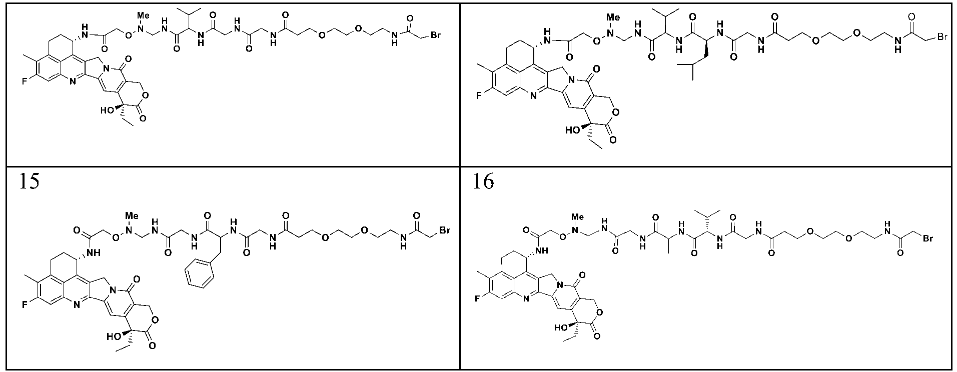

.

The antibody-drug conjugate afforded by linking the following Compound with an antibody:

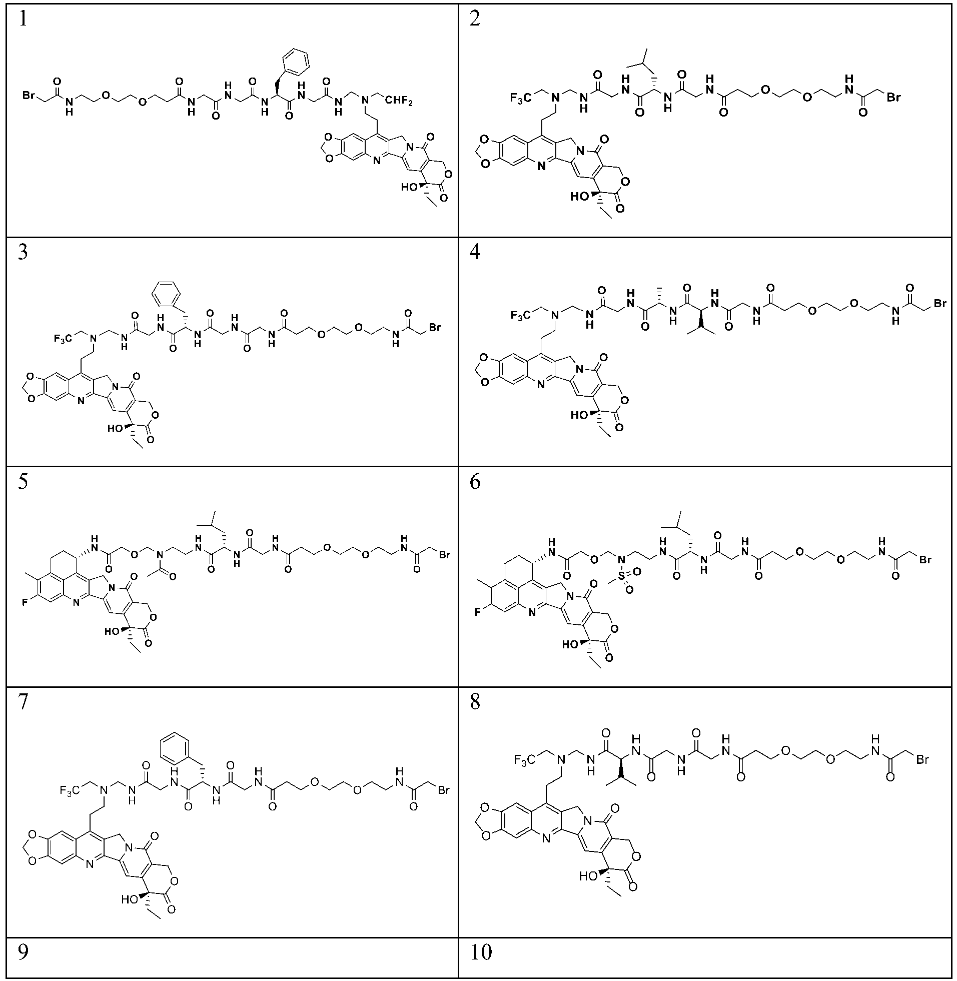

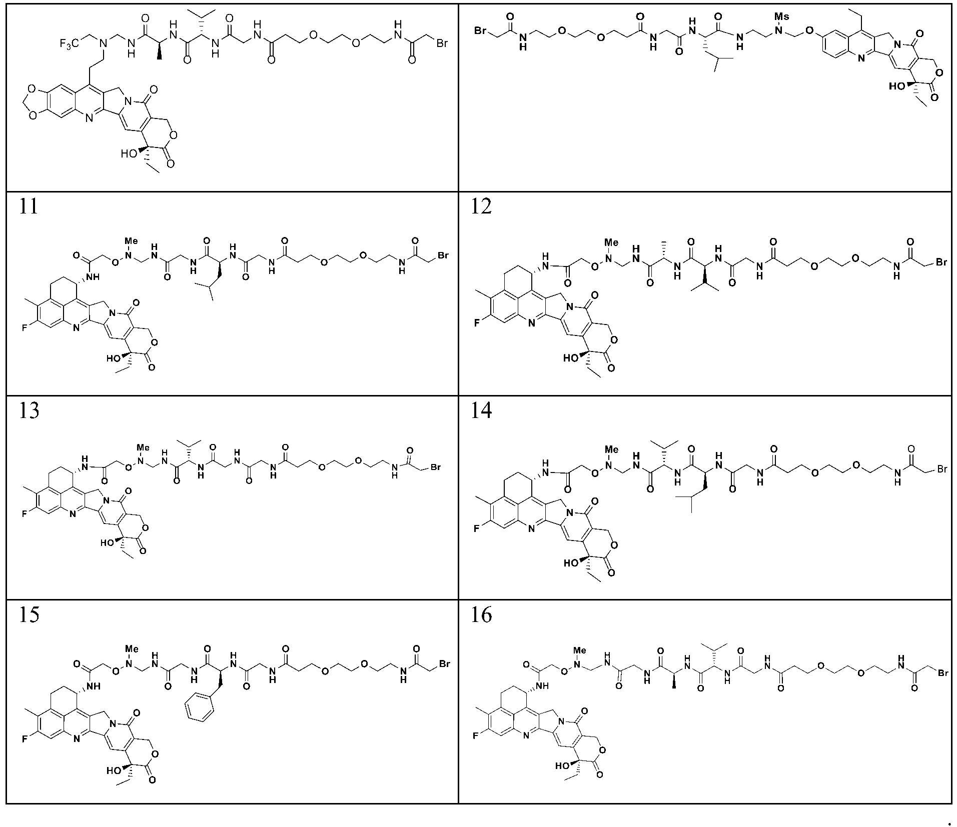

In some specific embodiments, the antibody is HS627 antibody or IP140B antibody, the heavy chain amino acid sequence of HS627 is as set forth in SEQ ID NO: 1, and the light chain amino acid sequence thereof is as set forth in SEQ ID NO: 2; the heavy chain amino acid sequence of the IP140B antibody is as set forth in SEQ ID NO: 3, and the light chain amino acid sequence thereof is as set forth in SEQ ID NO: 4.

The following antibody-drug conjugate,

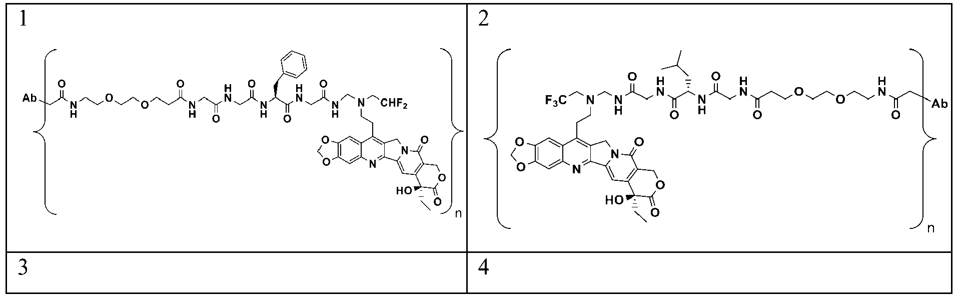

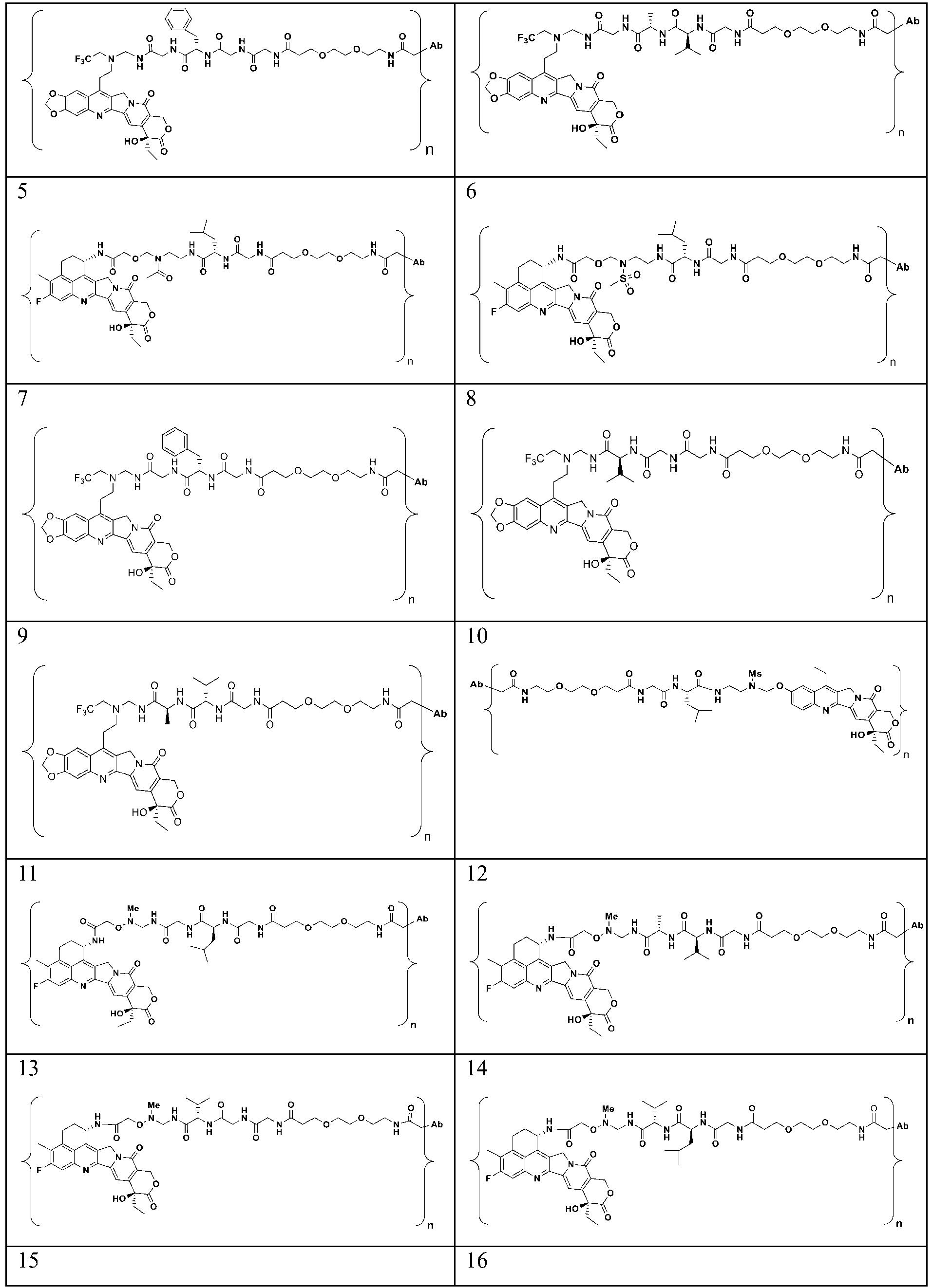

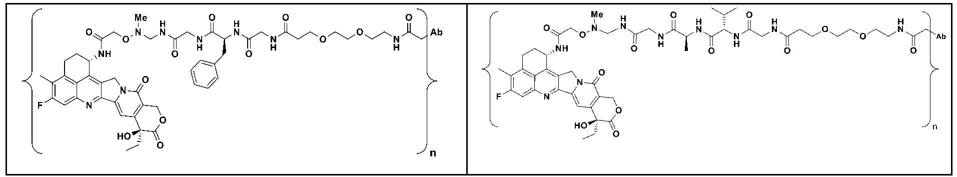

wherein, 2<n<8.

In some embodiments, n is selected from 6-8 or 6.2-7.8, for example can be 6.2, 6.3, 6.5, 6.6, 6.8, 7.0, 7.1, 7.2, 7.3, 7.4, 7.5 or 7.6, 7.7, 7.78 or 7.8.

In some specific embodiments, Ab is selected from an antibody against tumor-associated antigen, further selected from anti-Her2 antibody, anti-Trop2 antibody, anti-B7H3 antibody, anti-5T4 antibody, anti-Nectin-4 antibody, anti-CD20 antibody, and anti-ROR1 antibody.

Another aspect of the present disclosure provides a method for preparing the above antibody-drug conjugate, which is selected from the following synthetic routes:

### Synthetic route 1:

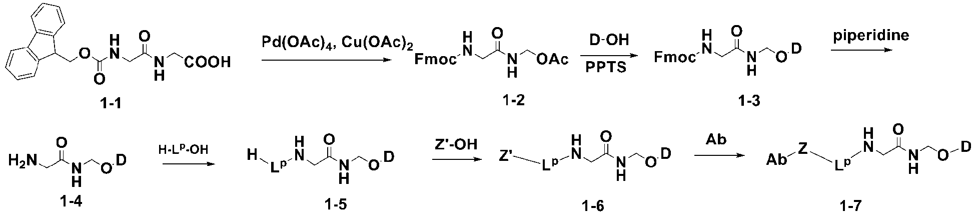

The compound of Formula 1-1 was reacted to afford the compound of Formula 1-2; the compound of Formula 1-2 was reacted with D-OH to afford the compound of Formula 1-3; the compound of Formula 1-3 was deprotected by removing the Fmoc protecting group to afford the compound of Formula 1-4; the compound of Formula 1-4 was condensed with H-L^{p}-OH to afford the compound of Formula 1-5; the compound of Formula 1-5 was condensed with Z'-OH to afford the compound of Formula 1-6; the compound of Formula 1-6 was conjugated with an antibody to afford the compound of Formula 1-7.

### Synthetic route 2:

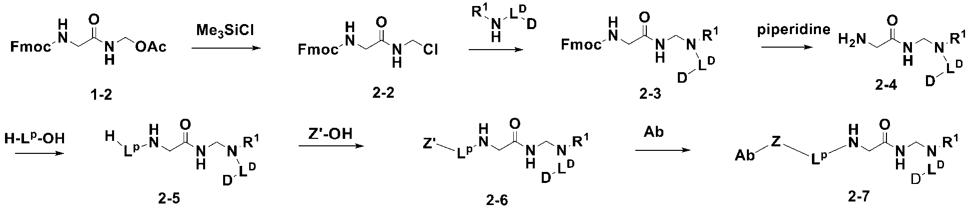

The compound of Formula 1-2 was reacted to afford the compound of Formula 2-2; the compound of Formula 2-2 was reacted with Compound R¹-NH-L^{D}-D to afford the compound of Formula 2-3; the compound of Formula 2-3 was deprotected by removing the Fmoc protecting group to afford the compound of Formula 2-4; the compound of Formula 2-4 was condensed with H-L^{p}-OH to afford the compound of Formula 2-5; the compound of Formula 2-5 was condensed with Z'-OH to afford the compound of Formula 2-6; the compound of Formula 2-6 was linked with an antibody to afford the compound of Formula 2-7.

### Synthetic route 3:

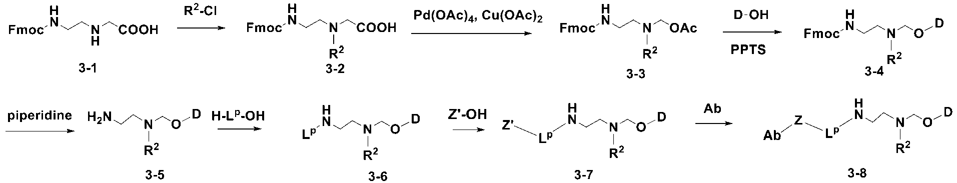

The compound of Formula 3-1 was reacted with R²-Cl to afford the compound of Formula 3-2; the compound of Formula 3-2 was reacted to afford the compound of Formula 3-3; the compound of Formula 3-3 was reacted with D-OH to afford the compound of Formula 3-4; the compound of Formula 3-4 was deprotected by removing the Fmoc protecting group to afford the compound of Formula 3-5; the compound of Formula 3-5 was condensed with H-L^{p}-OH to afford the compound of Formula 3-6; the compound of Formula 3-6 was condensed with Z'-OH to afford the compound of Formula 3-7; the compound of Formula 3-7 was linked with an antibody to afford the compound of Formula 3-8.

### Synthetic route 4:

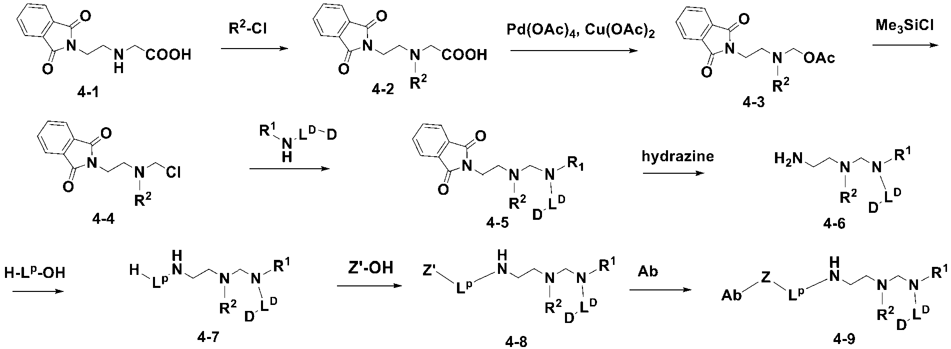

The compound of Formula 4-1 was reacted with R²-Cl to afford the compound of Formula 4-2; the compound of Formula 4-2 was reacted to afford the compound of Formula 4-3; the compound of Formula 4-3 was further reacted to afford the compound of Formula 4-4; the compound of Formula 4-4 was reacted with R¹-NH-L^{D}-D to afford the compound of Formula 4-5; the compound of Formula 4-5 was reacted to afford the compound of Formula 4-6; the compound of Formula 4-6 was condensed with H-L^{p}-OH to afford the compound of Formula 4-7; the compound of Formula 4-7 was condensed with Z'-OH to afford the compound of Formula 4-8; the compound of Formula 4-8 was linked with an antibody to afford the compound of Formula 4-9.

### Synthetic route 5:

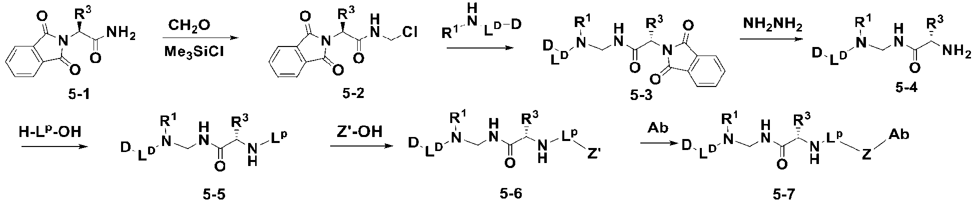

The compound of Formula 5-1 was reacted to afford the compound of Formula 5-2; the compound of Formula 5-2 was reacted with R¹-NH-L^{D}-D to afford the compound of Formula 5-3; the compound of Formula 5-3 was reacted to afford the compound of Formula 5-4; the compound of Formula 5-4 was condensed with H-L^{p}-OH to afford the compound of Formula 5-5; the compound of Formula 5-5 was condensed with Z'-OH to afford the compound of Formula 5-6; the compound of Formula 5-6 was linked with an antibody to afford the compound of Formula 5-7.

In the above synthetic routes, R¹, R², R³, L^{D}, D, L^{p}, Z, Ab are each defined the same as above.

Z' is selected from -L^{z}-L^{j'}, wherein, L^{z} is defined the same as above, L^{j}' is selected from

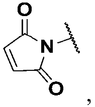

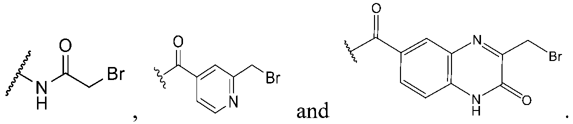

Another aspect of the present disclosure provides a pharmaceutical composition comprising the above antibody-drug conjugate and a pharmaceutically acceptable carrier.

Another aspect of the present disclosure provides a use of the above antibody-drug conjugate, or pharmaceutical composition in the manufacture of an anti-tumor medicament.

Another aspect of the present disclosure provides a method for preventing or treating a tumor disease in a patient in need thereof, comprising administering to the patient the above antibody-drug conjugate or pharmaceutical composition.

In some specific embodiments, the dosage of the above antibody-drug conjugate or pharmaceutical composition is a therapeutically effective amount.

In some specific embodiments, the tumor is selected from a solid tumor.

In some specific embodiments, the tumor is selected from lung cancer, non-small cell lung cancer, esophageal squamous cell carcinoma, ovarian cancer, esophageal adenocarcinoma or breast cancer.

In some specific embodiments, the lung cancer is non-small cell lung cancer.

### Description of Drawings

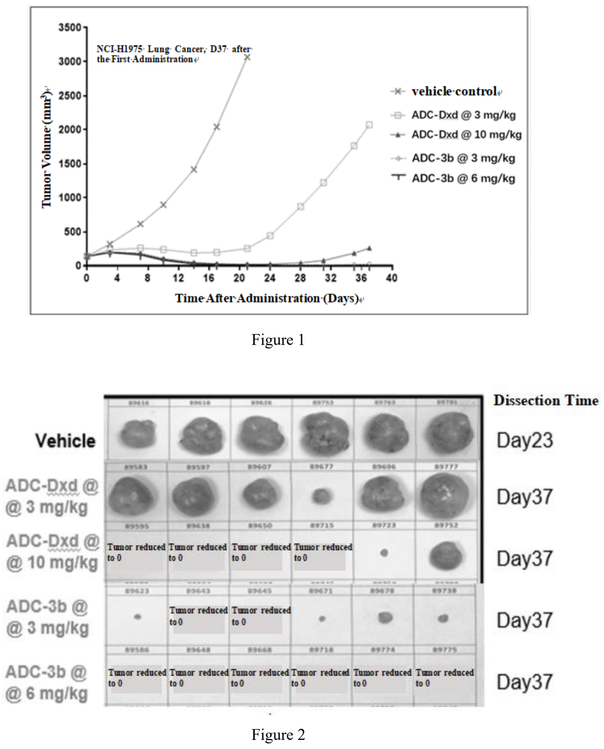

Figure 1 is a graph of the *in vivo* tumor growth curve for the NCI-H1975 lung cancer CDX model inhibited by ADC3b in Assay 1.
Figure 2 is a photograph of tumors after dissection from the NCI-H1975 lung cancer CDX model inhibited by ADC3b in Assay 1.

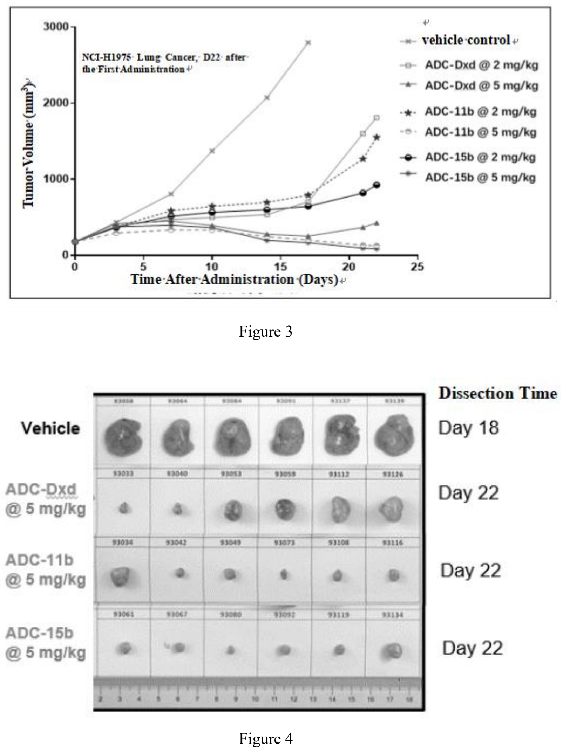

Figure 3 is a graph of the *in vivo* tumor growth curve for the NCI-H1975 lung cancer CDX model inhibited by ADC11b and 15b in Assay 1.
Figure 4 is a photograph of tumors after dissection from the NCI-H1975 lung cancer CDX model inhibited by ADC11b and 15b in Assay 1.

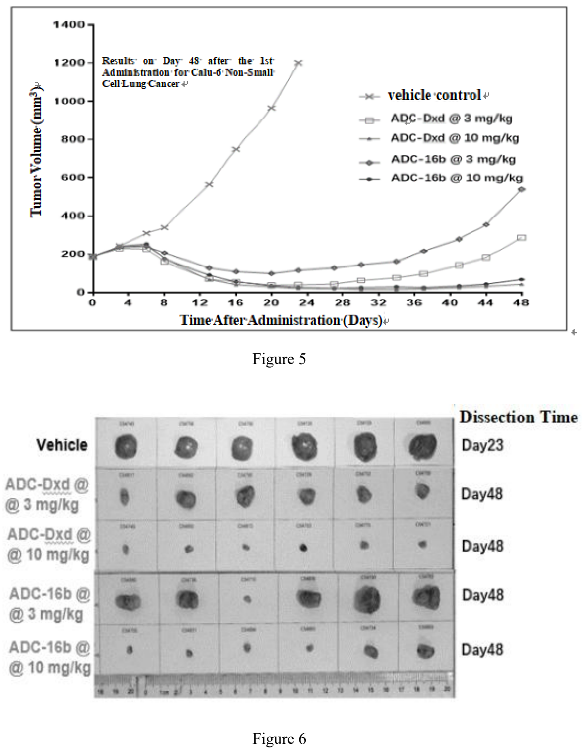

Figure 5 is a graph of the *in vivo* tumor growth curve for the Calu-6 non-small cell lung cancer CDX model inhibited by ADC16b in Assay 1.
Figure 6 is a photograph of tumors after dissection from the Calu-6 non-small cell lung cancer CDX model inhibited by ADC16b in Assay 1.

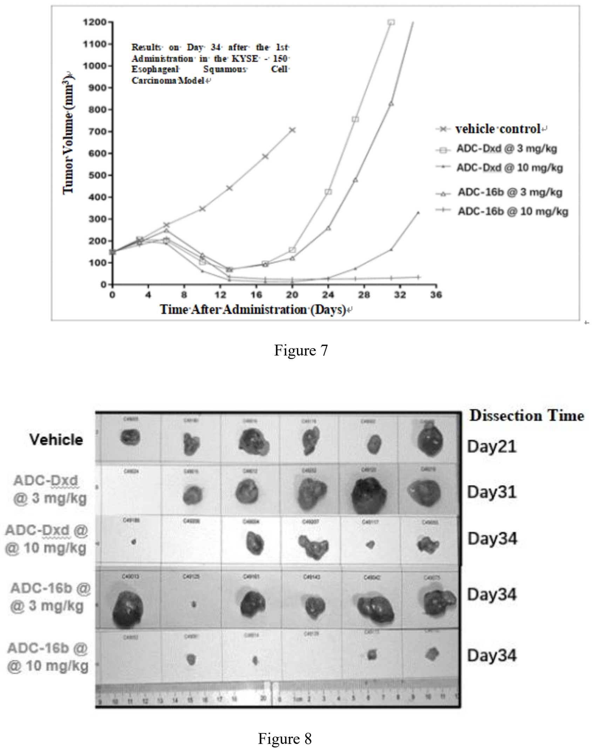

Figure 7 is a graph of the *in vivo* tumor growth curve for the KYSE-150 esophageal squamous cell carcinoma CDX model inhibited by ADC16b in Assay 1.
Figure 8 is a photograph of tumors after dissection from the KYSE-150 esophageal squamous cell carcinoma CDX model inhibited by ADC16b in Assay 1.

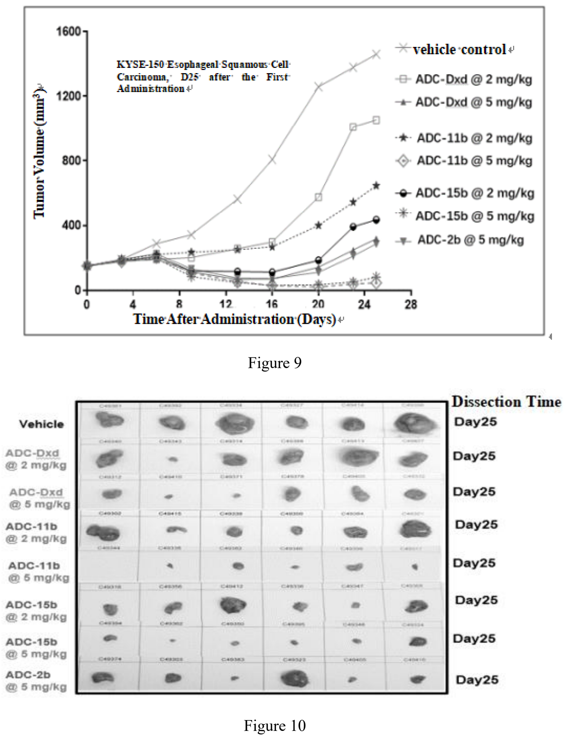

Figure 9 is a graph of the *in vivo* tumor growth curve for the KYSE-150 esophageal squamous cell carcinoma CDX model inhibited by ADC11b, 15b, and 2b in Assay 1.
Figure 10 is a photograph of tumors after dissection from the KYSE-150 esophageal squamous cell carcinoma CDX model inhibited by ADC11b, 15b, and 2b in Assay 1.

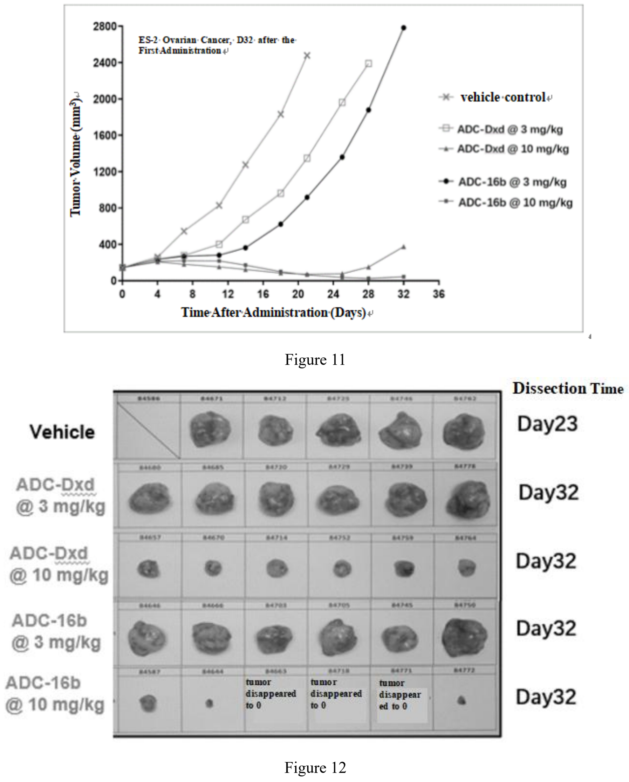

Figure 11 is a graph of the *in vivo* tumor growth curve for the ES-2 ovarian cancer CDX model inhibited by ADC16b in Assay 1.
Figure 12 is a photograph of tumors after dissection from the ES-2 ovarian cancer CDX model inhibited by ADC16b in Assay 1.

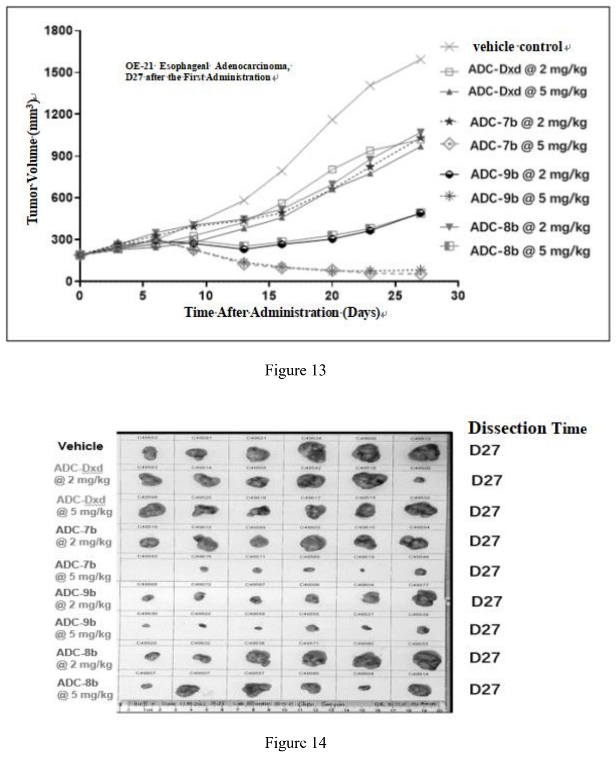

Figure 13 is a graph of the *in vivo* tumor growth curve for the OE-21 esophageal adenocarcinoma CDX model inhibited by ADC7b, 9b, and 8b in Assay 1.
Figure 14 is a photograph of tumors after dissection from the OE-21 esophageal adenocarcinoma CDX model inhibited by ADC7b, 9b, and 8b in Assay 1.

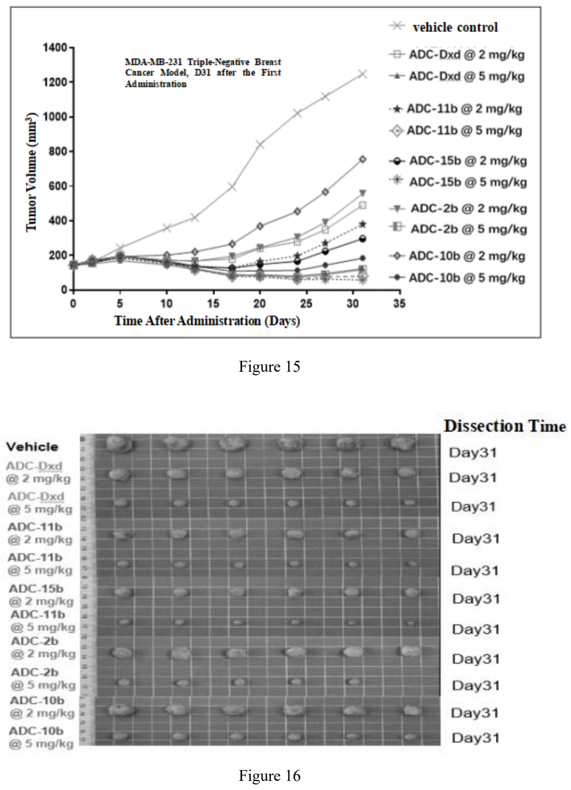

Figure 15 is a graph of the *in vivo* tumor growth curve for the MDA-MB-231 triple-negative human breast cancer CDX model inhibited by ADC11b, 15b, 2b, and 10b in Assay 1.
Figure 16 is a photograph of tumors after dissection from the MDA-MB-231 triple-negative human breast cancer CDX model inhibited by ADC11b, 15b, 2b, and 10b in Assay 1.

### DETAILED DESCRIPTION OF THE EMBODIMENTS

### I. Definition

In the present disclosure, unless otherwise specified, scientific and technical terms used herein have the meanings commonly understood by those skilled in the art. Also, the relevant terms and laboratory procedures used herein are terms and conventional procedures widely used in the corresponding fields. Meanwhile, for a better understanding of the present disclosure, definitions and explanations of related terms are provided below.

As used herein and unless otherwise specified, the terms "comprising", "including", "having", "containing", including grammatical equivalents thereof, should generally be understood to be openended and non-limiting, for example, not excluding other unrecited elements or steps.

The compound of the present disclosure can be asymmetric, for example, having one or more stereoisomers. Unless otherwise specified, all stereoisomers are included, for example, enantiomers and diastereomers. The stereoisomers include geometric isomers (such as cis and trans structures) and optical isomers (such as enantiomers), therapeutic substances consisting of monomers, racemates, racemic mixtures and pharmaceutically acceptable salts thereof. The compound of the present disclosure containing asymmetric carbon atoms can be isolated in optically active pure form or racemic form. Optically active pure forms can be resolved from racemic mixtures or synthesized by using chiral starting materials or chiral reagents. Racemates, diastereomers, enantiomers are all included within the scope of the present disclosure.

A numerical range as used herein refers to each integer within the given range. For example,"C1-C6" refers to the group that can have 1 carbon atom, 2 carbon atoms, 3 carbon atoms, 4 carbon atoms, 5 carbon atoms or 6 carbon atoms.

When any variable (such as Rn) appears more than once in the composition or structure of a compound, the definition thereof is independent in each case. Therefore, for example, if a group is substituted with 1-5 R's, then the group can optionally be substituted with up to 5 R's, and each R has independent options in every case. In addition, combinations of substituents and/or variants thereof are only allowed if such combinations result in the formation of stable compounds.

The term "C₁₋C₆ alkyl" refers to straight or branched alkyl groups having 1 to 6 carbon atoms. Specific examples of alkyl groups include, but are not limited to, methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and various branched isomers thereof, etc.

The term "alkoxyl" refers to -O-(alkyl) and -O-(unsubstituted cycloalkyl), wherein the definition of alkyl is as described above. Non-limiting examples of alkoxyl include: methoxy, ethoxy, propoxy, butoxy, cyclopropoxy, cyclobutoxy, cyclopentoxy, cyclohexoxy. Alkoxyl can be optionally substituted or unsubstituted. When substituted, the substituents are preferably one or more of the following groups, which are independently selected from alkyl, alkenyl, alkynyl, alkoxyl, alkylthio, alkylamino, halogen, mercapto, cyano, nitro, chloro group, cycloalkyl, heterocycloalkyl, aryl, heteroaryl, cycloalkoxyl, heterocycloalkoxyl, cycloalkylthio, heterocycloalkylthio, carboxyl, or carboxylate.

The term "acyl" means -C(=O)R, where R is a substituted or unsubstituted alkyl, a substituted or unsubstituted cycloalkyl, or a substituted or unsubstituted heteroalkyl.

The term "sulfonyl" means -S(=O)₂R, where R is a substituted or unsubstituted alkyl, a substituted or unsubstituted cycloalkyl, or a substituted or unsubstituted heteroalkyl.

" 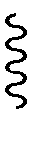 " refers to the chemical bond junction. It should be noted that the structural fragments described in the disclosure (such as -L¹¹-L¹²-L¹³- or -L^{z}-L^{j}-), unless otherwise specified, represent the sequential linking of corresponding groups from left to right. For example, when -L¹²- is C₁-C₂ alkylene-O-, it means the left side of C₁-C₂ alkylene-O- is linked to -L¹¹-, and the right end is linked to -L¹³-.

Understandably, in the antibody-drug conjugates of the present disclosure, such as in formulas (I), (II), (III), (IV), the "-" between the drug-linker fragment and the antibody is intended to signify the linking relationship between the antibody and the fragment, and is not intended to limit the conjugate to one antibody linked to one drug-linker fragment. As is known in the art, due to the presence of multiple interchain disulfide bonds on an antibody, one antibody can be linked to one or more drugs.

Medicament or pharmaceutical composition

The drugs or drug compositions of the present disclosure can be administered orally, topically, parenterally, or mucosally (e.g., sublingually, by inhalation, or rectally) in dose unit formulations comprising conventional non-toxic pharmaceutically acceptable carriers. An oral route is generally preferred. The active agents can be administered orally in the form of capsules, tablets, etc.

The term "treatment" includes inhibiting, alleviating, preventing, or eliminating one or more symptoms or side effects associated with the disease, condition, or disorder being treated.

The term "inhibition" is used in relation to controls. Those skilled in the art will readily determine the appropriate control for each experiment. For example, a reduced response in subjects or cells treated with the compound is compared to the response in subjects or cells not treated with the compound.

The term "patient" refers to an animal, preferably a mammal, and more preferably a human.

The term "pharmaceutical composition" refers to a composition that includes the compound or pharmaceutically acceptable salt thereof of the present disclosure, as well as at least one pharmaceutically acceptable component selected from the following, depending on the administration route and the nature of dosage form, including but not limited to: carriers, diluents, adjuvants, excipients, preservatives, fillers, disintegrants, wetting agents, emulsifiers, suspension agents, sweeteners, flavoring agents, fragrances, antimicrobials, antifungals, lubricants, dispersing agents, temperature-sensitive materials, temperature regulators, adhesives, stabilizers, and suspending agents.

The term "effective amount" or "therapeutically effective amount" refers to a sufficient amount of a drug or medication that is non-toxic but can achieve the desired effect. In embodiments of the present disclosure, when treating a patient in accordance with the present disclosure, the amount of drugs administered depends on many factors, such as the specific dosing regimen, the type of disease or condition and the severity thereof, the uniqueness (for example, body weight) of the treated person or host to be treated, but the amount of drug administered depends on specific surrounding circumstances, including, for example, the specific drug that has been employed, the route of administration, the treated condition, and the treated person or host to be treated, the administered dose may be routinely determined by methods known in the art. Generally, regarding the dosage used for adult therapy, the administered dose is typically within the range of 0.02-5000mg/day, for example, the range of about 1-1500mg/day. The required dose can be conveniently administered as a single dose, or as doses given simultaneously (or in a short period of time) or in divided doses at appropriate intervals, such as two, three, four doses per day or more. It can be understood by those skilled in the art that, although the above dose ranges are provided, the specific effective amount can be appropriately adjusted based on the patient's condition and in conjunction with a physician's diagnosis.

The term "antibody-drug conjugate (ADC)" refers to a biologically active small molecule drug linked to a monoclonal antibody via a chemical linker. The monoclonal antibody serves as a carrier to target and deliver the small molecule drug to specific target cells. As used herein, the term "antibody-drug conjugate" has the same meaning as "antibody-drug conjugate".

The term "Drug Antibody Ratio (DAR)" refers to the average number of payload molecules (antitumor compounds or drugs) attached to a single monoclonal antibody.

Abbreviations:
MS: methanesulfonyl;
AC: acetyl;
Fmoc: 9-fluorenylmethoxycarbonyl;
amino acids constituting the peptide residue L^{P}: Val: valine, whose structural formula is 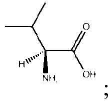 Ala: alanine, whose structural formula is 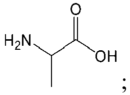 Gly: glycine, whose structural formula is 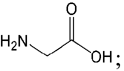 Phe: phenylalanine, whose structural formula is 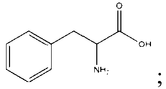 Leu: leucine, whose structural formula is 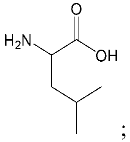
in the present disclosure, the peptide residue L^{P} can be obtained by amino acid condensation methods known in the art, which is not particularly limited in the present disclosure.

In the present disclosure, the antibody and the linker L^{j} for connecting to the antibody can be linked by methods known in the art. For example, when the structure of L^{j} is 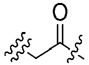 (or 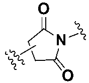 ), it can be linked to a reactive group such as a thiol on the antibody through 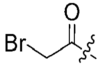 ( or 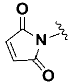 ) to afford, which is not particularly limited in the present disclosure.

### II. Example

In the example, the preparation of ADC uses, but not limited to, pertuzumab and IP 140B antibodies. The heavy chain amino acid sequence of pertuzumab (HS627, PERTUZUMAB) is as follows (SEQ ID NO: 1):

EVQLVESGGGLVQPGGSLRLSCAASGFTFTDYTMDWVRQAPGKGLEWVADVNPNSGGS
IYNQRFKGRFTLSVDRSKNTLYLQMNSLRAEDTAVYYCARNLGPSFYFDYWGQGTLVTVSSA
STKGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVLT
VLHQDWLNGKEYKCKVSNKALPAPIEKTISKAKGQPREPQVYTLPPSREEMTKNQVSLTCLV
KGFYPSDIAVEWESNGQPENNYKTTPPVLDSDGSFFLYSKLTVDKSRWQQGNVFSCSVMHEA
LHNHYTQKSLSLSPG

The light chain amino acid sequence is as follows (SEQ ID NO: 2):

DIQMTQSPSSLSASVGDRVTITCKASQDVSIGVAWYQQKPGKAPKLLIYSASYRYTGVPSR
FSGSGSGTDFTLTISSLQPEDFATYYCQQYYIYPYTFGQGTKVEIKRTVAAPSVFIFPPSDEQLKS
GTASVVCLLNNFYPREAKVQWKVDNALQSGNSQESVTEQDSKDSTYSLSSTLTLSKADYEKH
KVYACEVTHQGLSSPVTKSFNRGEC

The heavy chain amino acid sequence of the IP140B antibody is as follows (SEQ ID NO: 3):

QVQLVQSGGGLVQPGGSLRLSCAASGFTFSSYEMNWVRQAPGKGLEWVSYISSSGSTIY
YADSVKGRFTISRDNAKNSLYLQMNSLRAEDTAVYYCAREMQFGWELLGAFDIWGQGTMVT
VSSASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKRVEPKSCDKTHTCPPCPAPELLGGPSVFL
FPPKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSRDELTKNQVSLTC
LVKGFYPSDIAVEWESNGQPENNYKTTPPVLDSDGSFFLYSKLTVDKSRWQQGNVFSCSVMH
EALHNHYTQKSLSLSPGK

The light chain amino acid sequence is as follows (SEQ ID NO: 4):

EIVLTQSPGTLSLSPGERATLSCRASQSVSSSYLAWYQQKPGQAPRLLIYGASSRATGIPDR
FSGSGSGTDFTLTISRLEPEDFAVYYCQQYGSSFGQGTKLEIKRTVAAPSVFIFPPSDEQLKSGTA
SVVCLLNNFYPREAKVQWKVDNALQSGNSQESVTEQDSKDSTYSLSSTLTLSKADYEKHKV
YACEVTHQGLSSPVTKSFNRGEC.

Unless otherwise specified, the raw materials and equipment used in the specific embodiments of the present disclosure are all known products, obtained by purchasing commercially available products.

### Preparation Example 1: Preparation of Intermediates 1-5

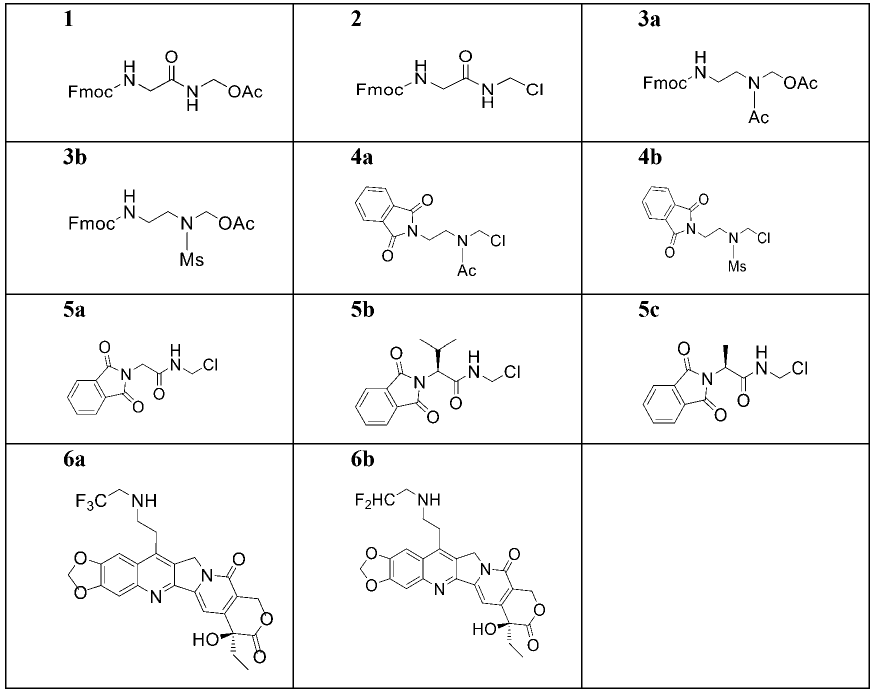

### Synthesis of Intermediate 1

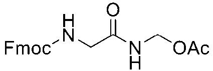

Intermediate 1 was synthesized according to the method in literature (WO2019195665A1): In a 250 mL reaction flask, Fmoc-Gly-Gly-OH (5 g, 14.11 mol), DMF (50 mL) were added, and the mixture was stirred to dissolve. Cupric acetate (768 mg, 4.23 mmol), acetic acid (1.69 g, 28.22 mmol), and lead tetraacetate (6.872 g, 15.5 mmol) were added in sequence. The mixture was heated to 60 °C and stirred to react for 20 min. The reaction solution was poured into ice water, extracted with ethyl acetate, dried, and recrystallized to afford the product Intermediate 1 as an off-white solid (3.2 g, yield 66%); LCMS: (M+1)⁺369.21 (theoretical value: 368.14).

### Synthesis of Intermediate 2

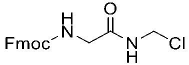

Intermediate 2 was synthesized according to the method in literature (WO2019195665A1): In a 100 mL three-necked flask, Intermediate 1 (1 g, 2.7 mmol), DCM (10 mL), and TMSC1 (8 mL) were added. The mixture was stirred at room temperature for 2 h under argon protection and concentrated under reduced pressure to afford the Intermediate 2 as a white solid (yield ~100%), which was directly used in the next step without purification; LCMS: (M+1)+ 344.80 (theoretical value: 344.09). LCMS: (M+1)⁺344.80 (theoretical value: 344.09).

### Synthesis of Intermediates 3a and 3b

### (1) Synthesis of Intermediate 3a

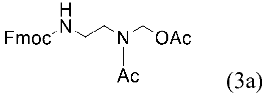

In a 250 mL reaction flask, Fmoc-NHCH₂CH₂NHCH₂COOH (5 g, 14.7 mmol), DCM (70 mL), TEA (4.46 g, 44.07 mmol), and acetyl chloride (2.31 g, 29.38 mmol) were added. The mixture was stirred to react at room temperature for 4 h, and the solvent was removed under reduced pressure to afford the product Fmoc-NHCH₂CH₂N(Ac)CH₂COOH as a solid. (5.62 g, yield 100%); LCMS: (M+1)⁺ 383.16 (theoretical value: 382.15).

In a reaction flask, Fmoc-NHCH₂CH₂N(Ac)CH₂COOH (2 g, 5.23 mmol), THF (30 mL), Cu(OAc)₂ (351.5 mg, 1.94 mmol), Pb(OAc)₄ (3.48 g, 7.84 mmol), and acetic acid (691 mg, 11.51 mmol) were added. The reaction solution was stirred at room temperature for 0.5 h, and the solvent was removed under reduced pressure. The residue was recrystallized to afford the product Intermediate 3a Fmoc-NHCH₂CH₂N(Ac)CH₂OAc as a solid. (1.95 g, yield 94.1%); ¹H NMR (500 MHz, DMSO-d₆, ppm): δ 7.89 (d, *J*=7.5 Hz, 2H), 7.68 (t, *J*=6.3 Hz, 2H), 7.42 (t, *J*=7.5 Hz, 2H), 7.34 (t, *J*=7.5 Hz, 2H), 5.31 (d, *J*=18.7 Hz, 2H), 4.34 (dd, *J*=20.0, 6.9 Hz, 2H), 4.23 (d, *J*=6.9 Hz, 1H), 3.46-3.39 (m, 2H), 3.12-3.20 (m, 2H), 2.11 (s, 3H), 2.04 (s, 3H); ¹³C NMR (126 MHz, DMSO-d₆, ppm) δ 171.67, 170.74, 156.65, 144.37, 141.22, 128.08, 127.53, 125.62, 120.57, 74.41, 65.81, 47.21, 46.01, 39.08, 21.66, 21.19; LCMS: (M+1)⁺ 397.17 (calculated value: 396.17).

### (2) Synthesis of Intermediate 3b

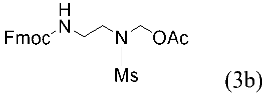

In a 250 mL reaction flask, Fmoc-NHCH₂CH₂NHCH₂COOH (2 g, 5.88 mmol), DCM (70 mL), TEA (1.78 g, 17.7 mmol), and MsCl (1.35 g, 11.8 mmol) were added. The reaction solution was stirred at room temperature for 4 h, and the solvent was removed under reduced pressure to afford the product Fmoc-NHCH₂CH₂N(Ms)CH₂COOH as a solid. (2.46 g, yield 100%); LCMS: (M+1)⁺ 419.12 (theoretical value: 418.12).

In a reaction flask, Fmoc-NHCH₂CH₂N(Ms)CH₂COOH (2 g, 4.78 mmol), THF (30 mL), Cu(OAc)₂ (321.5 mg, 1.77 mmol), Pb(OAc)₄ (3.18 g, 7.17 mmol), and acetic acid (631 mg, 10.51 mmol) were added. The mixture was stirred at room temperature for 0.5 h, and the solvent was removed under reduced pressure to afford the Intermediate 3b Fmoc-NHCH₂CH₂N(Ms)CH₂OAc as a solid. (1.95g, yield 94.3%); ¹H NMR (500 MHz, DMSO-d₆, ppm): δ 7.89 (d, *J*=7.5 Hz, 2H), 7.69 (d, *J*=7.5 Hz, 2H), 7.42 (t, *J*=7.6 Hz, 2H), 7.33 (t, *J*=7.1 Hz, 2H), 5.30 (s, 2H), 4.32 (d, *J*=6.7 Hz, 2H), 4.23 (d, *J*=6.8 Hz, 1H), 3.32 (t, *J*=6.1 Hz, 2H), 3.23 (t, *J*=6.1 Hz, 2H), 3.02 (s, 3H), 2.05 (s, 3H); ¹³C NMR (126 MHz, DMSO-d6, ppm) δ 170.74, 156.67, 144.34, 141.21, 128.08, 127.54, 125.61, 120.57, 72.53, 65.90, 47.17, 46.31, 39.97, 36.24; LCMS: (M+1)⁺ 433.14 (calculated value: 432.14).

### Synthesis of Intermediates 4a and 4b

### (1) Synthesis of Intermediate 4a

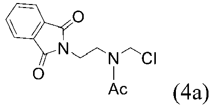

To a reaction flask, N-(aminoethyl)phthalimide (0.6 g, 2.65 mmol), DCM (50 mL), DIEA (0.684 g, 5.29 mmol), and AcCl (0.25 g, 3.18 mmol) were added. The mixture was stirred to react at room temperature for 4 h. Saturated brine (50 mL) was added, and the DCM layer was separated. The aqueous phase was extracted twice with dichloromethane (50 mL x 2). The combined organic phases were dried, and the solvent was removed under reduced pressure to afford the Compound N-(2-acetamidoethyl)phthalimide. (550 mg, yield 89.4%); LCMS: (M+1)⁺ 233.02 (theoretical value: 232.08).

To a reaction flask, the above N-(2-acetamidoethyl)phthalimide (0.55 g, 2.37 mmol), paraformaldehyde (95.4 mg, 3.18 mmol), DCM (12 mL), and TMSCl (3 mL) were added. The mixture was stirred to react at 50 °C for 12 h, and the solvent was removed under reduced pressure to afford Intermediate 4a (585 mg, yield 88%); ¹H NMR (600 MHz, DMSO-d₆, ppm): δ7.88-7.71 (m, 4H), 4.76-4.51 (m, 2H), 3.77-3.42 (m, 4H), 1.91 (s, 3H); LCMS: (M+1)⁺ 280.95 (theoretical value: 280.06).

### (2) Synthesis of Intermediate 4b

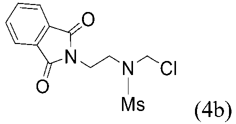

To a reaction flask, N-(2-aminoethyl)phthalimide (0.6 g, 2.65 mmol), DCM (50 mL), DIEA (0.684 g, 5.29 mmol), and MsCl (0.364 g, 3.18 mmol) were added. The mixture was stirred to react at room temperature for 4 h. Saturated brine (50 mL) was added, and the DCM layer was separated. The aqueous phase was extracted twice with dichloromethane (50 mL x 2). The combined organic phases were dried and the solvent was removed under reduced pressure to afford the Compound N-(2-(methylsulfonamidoethyl)phthalimide. (700 mg, yield 98%); LCMS: (M+1)⁺ 269.05 (theoretical value: 268.05).

To a reaction flask, the above N-(2-(methylsulfonamidoethyl)phthalimide (0.71 g, 2.65 mmol), paraformaldehyde (95.4 mg, 3.18 mmol), DCM (12 mL), and TMSCl (3 mL) were added. The mixture was stirred to react at 50 °C for 12 h, and the solvent was removed under reduced pressure to afford Intermediate **4b.** (838.5 mg, yield 100%); ¹H NMR (600 MHz, DMSO-d₆, ppm): δ 7.98-7.65 (m, 4H), 4.85-4.54 (m, 2H), 3.85-3.64 (m, 2H), 3.50-3.30 (m, 2H), 2.84 (s, 3H); ¹³C NMR (150 MHz, DMSO-d₆, ppm) δ 168.2, 168.2, 134.8, 134.8, 132.1, 132.1, 123.5, 123.5, 70.0, 42.5, 40.1, 36.2; LCMS: (M+1)⁺ 318.03 (theoretical value: 316.76).

### Synthesis of Intermediates 5a-c

### (1) Synthesis of Intermediate 5a

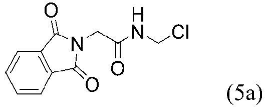

In a 500 mL single-neck flask, phthalylglycine (5 g, 24.39 mmol) was added and dissolved with DCM (175 mL). DMF (100 µL) was added, and the reaction solution was cooled to 0 °C with an ice bath. Oxalyl chloride (6.15 g, 48.78 mmol) was added dropwise under an argon atmosphere, and the reaction solution was continued to stir at 0 °C for 1.5 h. The mixture was concentrated under reduced pressure, and the concentrate was dissolved in DCM (90 mL). The solution was cooled to 0 °C, and a solution of 7 M ammonia in methanol (10.45 mL, 73 mmol) was added dropwise. The mixture was continued to stir to react at room temperature for 2 h, n-hexane was added, and the mixture was filtered. The filter cake was dried to afford phthalylglycinamide. (4.05 g, yield 81% ); ¹H NMR (600 MHz, DMSO-d₆):δ 7.87-7.86 (m, 4H), 7.84 (s, 1H), 7.26 (s, 1H), 4.17 (s, 2H); ¹³C NMR (151 MHz, DMSO-d₆):δ 168.54, 168.05, 162.96, 134.98, 132.15, 45.79; LCMS: (M+1)⁺204.87 (theoretical value: 204.05).

To a reaction flask, 5 mL of anhydrous DCM, Compound 2-(phthalimido)acetamide (200 mg, 979.5 µmol), paraformaldehyde (470.15 mg, 3.92 mmol), and TMSCl (1.25 mL) were added. The reaction solution was stirred at 50 °C for 2 h, and the solvent was removed under reduced pressure. The residue was purified by silica gel column chromatography to afford Intermediate **5a** (R = H) (220mg, yield 89%, HPLC 95%); ¹H NMR (500 MHz, CDCl₃, ppm): δ 7.91-7.89 (m, 2H), 7.77-7.75 (m, 2H), 5.44 (d, 2H), 4.73 (s, 2H); ¹³C NMR (151 MHz, DMSO-d₆): δ 170.20, 167.84, 134.86, 132.25, 123.52, 48.57, 15.24; LCMS: (M+1)⁺ 253.15 (theoretical value: 252.15).

### (2) Synthesis of Intermediate 5b

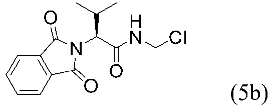

In a 500-mL single-neck flask, phthalylvaline (5 g, 20.22 mmol), dry DCM (175 mL), and DMF (100 µL) were added. The reaction solution was cooled to 0 °C with an ice bath, and oxalyl chloride (5.13 g, 40.44 mmol) was added dropwise under an argon atmosphere. The mixture was stirred at 0 °C for 1.5 h, and the solvent was removed under reduced pressure. The concentrate was dissolved in DCM (90 mL), cooled to 0 °C, and a solution of 7 M ammonia in methanol (8.66 mL, 60.6 mmol) was added dropwise. The reaction was continued to stir to react at room temperature for 2 h. N-hexane was added, and the mixture was filtered. The filter cake was dried to afford phthalylvalinamide. (2.98 g, yield 61% ); LCMS: (M+1)⁺ 247.02 (theoretical value: 246.10).

Phthalylvalinamide (1 g, 4.06 mmol), prepared as described above was dissolved in DCM (10 mL). TMSC1 (5 mL) and paraformaldehyde (550.4 mg, 18 mmol) were added, the tube was sealed, and the reaction was heated to 40°C and reacted for 1.5 h. The mixture was cooled to room temperature, filtered and concentrated to afford **5b** (1.19 g, yield 100% ); ¹H NMR (600 MHz, DMSO-d₆, ppm): δ 8.01-7.70 (m, 4H), 4.75-4.42 (m, 2H), 4.42-4.15 (m, 1H), 2.82-2.60 (m, 1H), 1.08-0.83 (m, 6H); LCMS: (M+1)⁺ 294.97 (theoretical value: 294.08).

### (3) Synthesis of Intermediate 5c

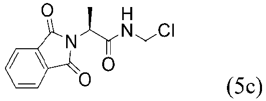

In a 500 mL single-neck flask, phthalylalanine (5 g, 22.8 mmol), DCM (175 mL), and DMF (100 µL) were added. The reaction solution was cooled to 0 °C with an ice bath, and oxalyl chloride (5.79 g, 45.6 mmol) was added dropwise under an argon atmosphere. The mixture was stirred at 0 °C for 1.5 h, and the solvent was removed under reduced pressure. The concentrate was dissolved in DCM (90 mL), cooled to 0 °C, and a solution of 7 M ammonia in methanol (9.78 mL, 68.5 mmol) was added dropwise. The mixture was continued to stir to react at room temperature for 2 h, n-hexane was added, and the mixture was filtered. The filter cake was dried to afford phthalylalaninamide (4.5 g, yield 92% ); ¹H NMR (600 MHz, DMSO-d₆, ppm): δ 7.84 (d, *J*=4.9 Hz, 3H), 7.63 (s, 1H), 7.45 (s, 1H), 7.19 (d, *J*=10.6 Hz, 1H), 4.69 (q, *J*=7.2 Hz, 1H), 1.56 (d, *J*=7.3 Hz, 3H); ¹³C NMR (151 MHz, DMSO-d₆, ppm):δ 171.23, 167.96, 134.81, 132.25, 123.45, 48.58, 15.42; LCMS: (M+1)⁺219.05 (theoretical value: 218.07).

Phthalylalaninamide (1 g, 4.5 mmol), prepared as described above was dissolved in DCM (10 mL), TMSCl (5 mL) and paraformaldehyde (550.4 mg, 18 mmol) were added, the tube was sealed, and the reaction was heated to 40°C and reacted for 1.5 h. The mixture was cooled to room temperature, filtered and concentrated to afford **5c** (1.22 g, yield 100%); ¹H NMR (500 MHz, CDCl₃, ppm): δ 7.85 (dd, *J*=5.4, 3.1 Hz, 2H), 7.78-7.72 (m, 2H), 7.19 (s, 1H), 5.23-5.13 (m, 2H), 5.03-4.74 (m, 2H), 1.69 (d, *J*=7.3 Hz, 3H); LCMS: (M+1)⁺266.98 (theoretical value: 266.05).

### Synthesis of Intermediates 6a and 6b

### (1) Synthesis of Intermediate 6a

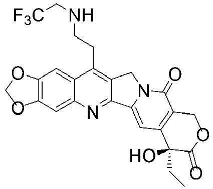

Trifluoroethylamine hydrochloride (100 mg, 0.74 mmol) and 7-methyl-10,11-methylenedioxycamptothecin (100 mg, 0.25 mmol) were dissolved in DMSO (3 mL). The mixture was stirred and heated to 120 °C for 1 h. After cooling, methyl tert-butyl ether was added, and the mixture was filtered and purified by silica gel column chromatography to afford the product 7-(N-(2,2,2,-trifluoroethyl)amino)ethyl-10,11-methylenedioxycamptothecin, **6a** (49mg, yield 38%, HPLC 99%); ¹H NMR (500 MHz, DMSO-d₆) δ 7.71 (s, 1H), 7.54 (s, 1H), 7.29 (s, 1H), 6.55 (s, 1H), 6.34 (d, *J*=2.0 Hz, 2H), 5.48 (d, *J*=3.0 Hz, 2H), 5.30 (s, 2H), 3.42 (s, 2H), 3.42 (s, 2H), 3.29 (s, 2H), 1.92 (dd, *J*=14.4, 7.3 Hz, 2H), 0.94 (t, *J*=7.3 Hz, 4H); LC-MS (M+H)⁺ 518.34 (theoretical value 517.15).

### (2) Synthesis of Intermediate 6b

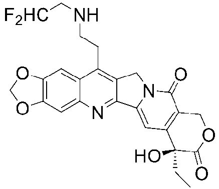

To a reaction flask, difluoroethylamine (1.0 g, 12 mmol), hydrochloric acid (1.4 mL, 16 mmol), 7-methyl-10,11-methylenedioxycamptothecin (1.0 g, 2.46 mmol), and DMSO (5 mL) were added. The mixture was stirred and heated to 120°C, and reacted for 1 h, then cooled. Isopropanol was added, and the mixture was filtered and purified by silica gel column chromatography to afford the product 7-(N-(2,2,-difluoroethyl)amino)ethyl-10,11-methylenedioxycamptothecin, **6b** (393 mg, yield 32%, HPLC 95%); LC-MS (M+H) + 500.13 (theoretical value 499.16). ¹H NMR (500 MHz, DMSO-d₆) δ 7.68 (s, 1H), 7.52 (s, 1H), 7.28 (s, 1H), 6.58 (s, 1H), 6.33 (s, 2H), 6.02 (t, *J*=56.6 Hz, 1H), 5.47 (s, 2H), 5.26 (s, 2H), 3.26 (s, 3H), 2.94 (s, 4H), 1.92 (s, 2H), 0.93 (s, 3H); LC-MS (M+H)⁺ 500.13 (theoretical value 499.16).

### Example 1: 7-(N-(2,2-difluoroethyl)-N-(bromoacetamidoethoxyethoxypropionyl-Gly-Gly-Phe-Gly-methylene)amino)ethyl-10,11-methylenedioxycamptothecin (1)

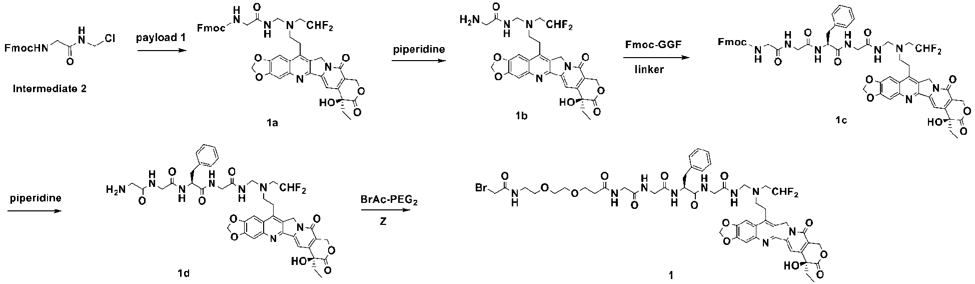

To a reaction flask, 7-(N-(2,2-difluoroethyl)amino)ethyl-10,11-methylenedioxycamptothecin (payload 1, 100 mg, 0.2 mmol), DCM (5 mL), triethylamine, and Intermediate 2 (0.93 g, 2.7 mmol) were added. The reaction solution was reacted at room temperature for 2 hours, then water (10 mL) was added. The mixture was extracted with DCM (20 mL*3), the organic phase was concentrated, and purified by silica gel column chromatography to afford Compound **10a** (121 mg, 75%); LCMS: (M+1)⁺ 808.13 (theoretical value: 807.28).

In a reaction flask, **1a** (121 mg, 0.149 mmol), DMF (1 mL), and piperidine (100 µL) were added. The reaction solution was stirred at room temperature for 1 h. Methyl tert-butyl ether (20 mL) was added, the mixture was centrifuged, the supernatant was removed, and the residue was dried to afford **1b** (68 mg, yield 78%); LCMS: (M+1)⁺ 586.21 (theoretical value: 585.20).

In a reaction flask, **1b** (68 mg, 0.11 mmol), DMF (2 mL), and DIPEA (15 mg, 0.11 mmol) were added. The reaction solution was stirred at room temperature, and Fmoc-Gly-Gly-Phe-OH (56 mg, 0.11 mmol) and HATU (42 mg, 0.11 mmol) were added in sequence. The reaction solution was continued to stir overnight at room temperature, the solvent was removed under reduced pressure, and purified by silica gel column chromatography afforded Product **1c** (107 mg, yield 86%); LCMS: (M+1)⁺ 1069.09 (theoretical value: 1068.38).

In a reaction flask, **1c** (107 mg, 0.1 mmol), DMF (1 mL), and piperidine (100 µL) were added. The reaction solution was stirred at room temperature for 1 h. Methyl tert-butyl ether (20 mL) was added, the mixture was centrifuged, the supernatant was removed, and the residue was dried to afford **1d** (56 mg, yield 66%); LCMS: (M+1)⁺ 847.23 (theoretical value: 846.31).

In a reaction flask, DCM (3 mL), **1d** (56 mg, 0.066 mmol), bromoacetamidoethoxyethoxypropionic acid (19.2 mg, 0.066 mmol), and DIC (8.4 mg, 0.066 mmol) were added. The reaction solution was stirred at room temperature for 90 min, concentrated, and purified by silica gel column chromatography to afford **1** as a yellow solid (12 mg, yield 16%); LCMS: (M+1)⁺ 1126.24 (theoretical value: 1125.33).

ADC-1a: Compound **1** and HER2 monoclonal antibody HS627 form an antibody-drug conjugate

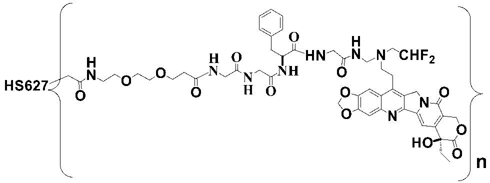

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **1** (0.90mg, 0.8mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose, 0.2g/L polysorbate 20, to afford ADC-1a (3.4mg/mL, 2mL).
UV-HPLC calculated average value: n=7.5

ADC-1b: Compound **1** and 5T4 monoclonal antibody IP140B form an antibody-drug conjugate

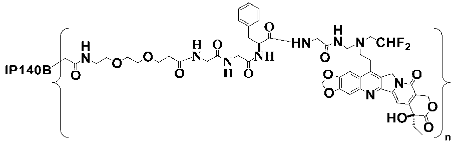

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **1** (0.90mg, 0.8mmol) was dissolved in 0.09 mL of DMA, and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH5.7, to afford ADC-1b (3.1mg/mL, 2mL).
UV-HPLC calculated average value: n=7.6

### Example 2: 7-(N-(2,2,2-trifluoroethyl)-N-(bromoacetamidoethoxyethoxypropionyl-Gly-Leu-Gly-methylene)amino)ethyl-10,11-methylenedioxycamptothecin (2)

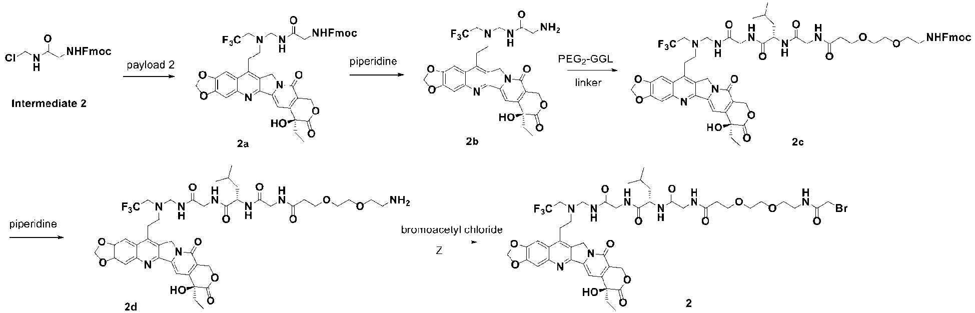

To a reaction flask, 7-(N-(2,2,2,-trifluoroethyl)amino)ethyl-10,11-methylenedioxycamptothecin (payload 2, 100mg, 0.19 mmol), DCM (5 mL), Intermediate 2 (665.3 mg, 1.9 mmol) were added. The reaction solution was stirred at room temperature for 2 hours, the reaction solution was concentrated, and purified by silica gel column chromatography to afford Compound **2a** (47 mg, yield 30%); LCMS: (M+1)⁺ 826.19 (theoretical value: 825.26).

In a reaction flask, **2a** (47 mg, 0.056 mmol), DMF (1 mL), and piperidine (100 µL) were added. The mixture was stirred at room temperature for 1 h, methyl tert-butyl ether (20 mL) was added, and the mixture was centrifuged. The supernatant was removed, and the residue was dried to afford **2b** (21 mg, yield 60%); LCMS: (M+1)⁺ 604.03 (theoretical value: 603.19).

In a reaction flask, **2b** (21 mg, 0.034 mmol), DMF (2 mL), and DIPEA (4.49 mg, 0.034 mmol) were added. The reaction solution was stirred at room temperature until dissolved. Fmoc-aminoethoxyethoxypropionyl-Gly-Leu (19.8 mg, 0.034 mmol) and HATU (13.23 mg, 0.034 mmol) were added in sequence, and the reaction solution was stirred at room temperature for 1 h. The mixture was concentrated and purified by silica gel column chromatography to afford Compound **2c** (35 mg, yield 87.5%); LCMS: (M+1)⁺ 1155.22 (theoretical value: 1154.46).

In a reaction flask, **2c** (35 mg, 0.03 mmol), DMF (1 mL), and piperidine (100 µL) were added. The mixture was stirred at room temperature for 1 h, methyl tert-butyl ether (20 mL) was added, and the mixture was centrifuged. The supernatant was removed, and the residue was dried to afford **2d** (22 mg, yield 78.9%); LCMS: (M+1)⁺933.65 (theoretical value: 932.95).

In a reaction flask, DCM (3 mL), **2d** (22 mg, 0.023 mmol), bromoacetic acid (7 mg, 0.023 mmol), and DIC (2.97 mg, 0.023 mmol) were added in sequence. The mixture was stirred at room temperature for 90 min, concentrated, and purified by silica gel column chromatography to afford **2** as a yellow solid (19 mg, yield 77%); LCMS: (M+1)⁺ 1053.27 (theoretical value: 1052.31).

ADC-2a: Compound **2** and HER2 monoclonal antibody HS627 form an antibody-drug conjugate

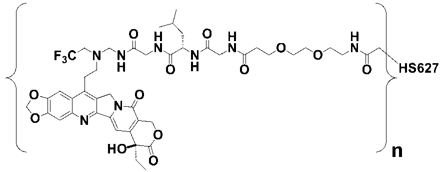

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **2** (0.84mg, 0.8mmol) was dissolved in 0.08 mL of DMA, and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose, 0.2g/L polysorbate 20, to afford ADC-2a (3.3mg/mL, 2mL).
UV-HPLC calculated average value: n=7.1

ADC-2b: Compound **2** and 5T4 monoclonal antibody IP140B form an antibody-drug conjugate

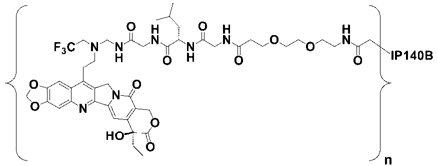

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **2** (0.84mg, 0.8mmol) was dissolved in 0.08 mL of DMA, and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH5.7, to affordADC-2b (3.1mg/mL, 2mL).

UV-HPLC calculated average value: n=7.0

### Example 3: 7-(N-(2,2,2-trifluoroethyl)-N-(bromoacetamidoethoxyethoxypropionyl-Gly-Gly-Phe-Gly-methylene)amino)ethyl-10,11-methylenedioxycamptothecin (3)

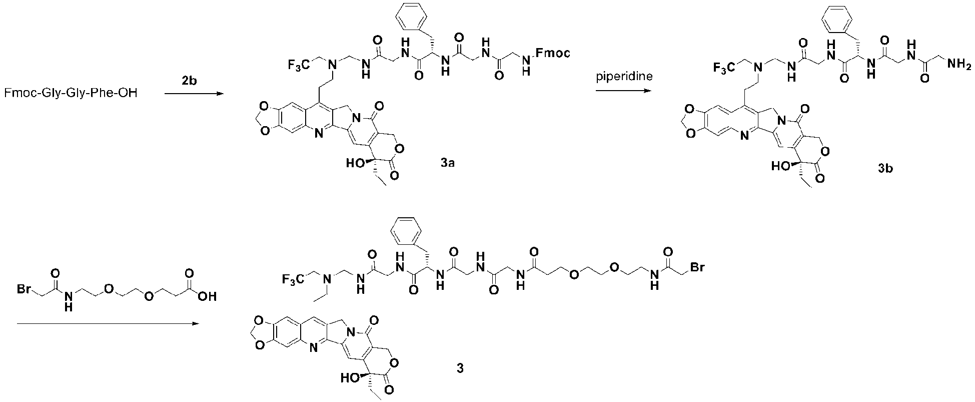

To a reaction flask, **2b** (70 mg, 0.11 mmol), DMF (2 mL), HATU (41.8 mg, 0.11 mmol), DIPEA (14.19 mg, 0.11 mmol), and Fmoc-Gly-Gly-Phe-OH (55.11 mg, 0.11 mmol) were added. The reaction solution was stirred at room temperature for 6 h, DCM (200 mL) was added, and the mixture was washed with saturated aqueous sodium chloride solution, dried over anhydrous sodium sulfate, and purified by silica gel column chromatography to afford **3a** as a yellow solid (108 mg, yield 85%); LCMS: (M+1)⁺ 1087.25 (theoretical value: 1086.37).

In a reaction flask, **3a** (108 mg, 0.099 mmol), DMF (1 mL), and piperidine (77 mg, 0.99 mmol) were added. The mixture was stirred at room temperature for 1 h, methyl tert-butyl ether (30 mL) was added, and the mixture was centrifuged. The supernatant was removed, and the solvent was removed under reduced pressure to afford **3b** as a solid (43 mg, yield 50%), which was directly used in the next step. LCMS: (M+1)⁺865.29 (theoretical value: 864.31).

In a reaction flask, DCM (2 mL), 3b (43 mg, 0.049 mmol), bromoacetamidoethoxyethoxypropionic acid (15 mg, 0.049 mmol), and DIC (6.27 mg, 0.049 mmol) were added in sequence. The mixture was stirred at room temperature for 90 min. The reaction solution was injected into a 25 g C18 pre-column (pre-equilibrated with acetonitrile, then with water, the water phase contains 0.1% TFA), then eluted via medium-pressure reverse-phase C18 chromatography (gradient: 5% to 40% acetonitrile in water over 30 min), lyophilized to afford **3** as a yellow solid (21 mg, yield 37.5%); LCMS: (M+1)⁺ 1144.28 (theoretical value: 1143.32).

ADC-3a: Compound **3** and HER2 monoclonal antibody HS627 form an antibody-drug conjugate

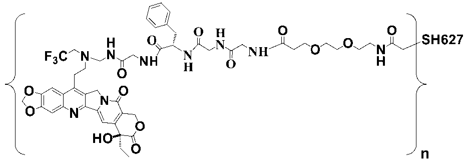

HS627 antibody (20.0mg/mL, 10mg, 0.066mmol), was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **3** (0.92mg, 0.8mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose, 0.2g/L polysorbate 20, to affordADC-3a (3.2mg/mL, 2mL).

UV-HPLC calculated average value: n=6.2

ADC-3b: Compound **3** and 5T4 monoclonal antibody IP140B form an antibody-drug conjugate

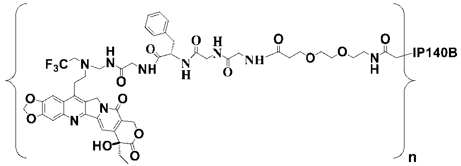

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **3** (0.92mg, 0.8mmol) was dissolved in 0.09 mL of DMA, and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH5.7, to affordADC-3b (3.3mg/mL, 2mL).

UV-HPLC calculated average value: n=6.5

### Example 4: 7-(N-(2,2,2-trifluoroethyl)-N-(bromoacetamidoethoxyethoxypropionyl-Gly-Val-Ala-Gly-methylene)amino)ethyl-10,11-methylenedioxycamptothecin (4)

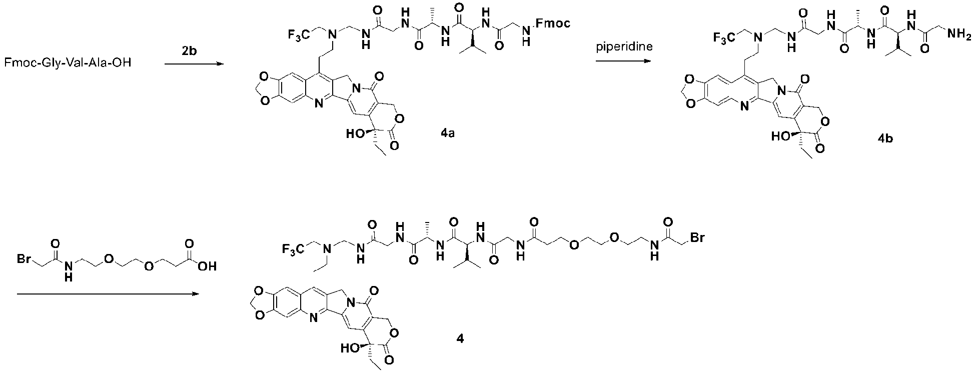

To a reaction flask, **2b** (70 mg, 0.11 mmol), DMF (2 mL), HATU (41.8 mg, 0.11 mmol), DIPEA (14.19 mg, 0.11 mmol), and Fmoc-Gly-Val-Ala-OH (51.37 mg, 0.11 mmol) were added. The mixture was stirred at room temperature for 6 h, DCM (200 mL) was added, and the mixture was washed with saturated sodium chloride solution, dried over anhydrous sodium sulfate, and purified by silica gel column chromatography to afford **13a** as a yellow solid (100.1 mg, yield 87%); LCMS: (M+1)⁺ 1053.06 (theoretical value: 1052.39).

In a reaction flask, **4a** (100.1 mg, 0.095 mmol), DMF (0.5 mL), and piperidine (74 mg, 0.95 mmol) were added. The mixture was stirred at room temperature for 1 h, methyl tert-butyl ether (30 mL) was added, and the mixture was centrifuged. The supernatant was removed, and the solvent was removed under reduced pressure to afford **4b** as a solid (49 mg, yield 62%), which was directly used in the next step; LCMS: (M+1)⁺ 831.27 (theoretical value: 830.32).

In a reaction flask, DCM (2 mL), **4b** (49 mg, 0.059 mmol), bromoacetamidoethoxyethoxypropionic acid (17.6 mg, 0.059 mmol) and DIC (7.43 mg, 0.059 mmol) were added in sequence. The mixture was stirred at room temperature for 90 min. The reaction solution was injected into a 25 g C18 pre-column (pre-equilibrated with acetonitrile, then with water, the water phase contains 0.1% TFA), then eluted via medium-pressure reverse-phase C18 chromatography (gradient: 5% to 40% acetonitrile in water over 30 min), lyophilized to afford **4** as a yellow solid (15 mg, yield 22%); LCMS: (M+1)⁺ 1110.28 (theoretical value: 1109.33).

ADC-4a: Compound **4** and HER2 monoclonal antibody HS627 form an antibody-drug conjugate

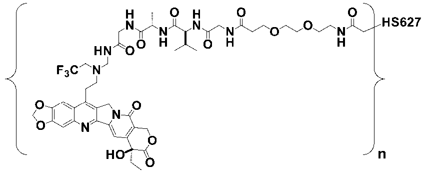

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **4** (0.89mg, 0.8mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose, 0.2g/L polysorbate 20, to affordADC-4a (3.1mg/mL, 2mL).

UV-HPLC calculated average value: n=6.3

ADC-4b: Compound 4 and 5T4 monoclonal antibody IP140B form an antibody-drug conjugate

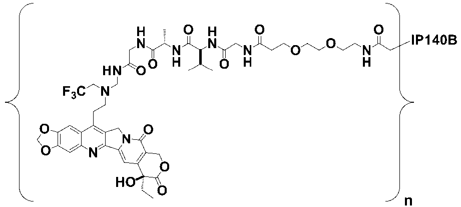

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **4** (0.89mg, 0.8mmol) was dissolved in 0.09 mL of DMA, and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH5.7, to afford ADC-4b (3.3mg/mL, 2mL).

UV-HPLC calculated average value: n=6.6

### Example 5: Bromoacetamidoethoxyethoxypropionyl-Gly-Leu-ethylenediamine(Ac)-methyleneoxyacetylexatecan (5)

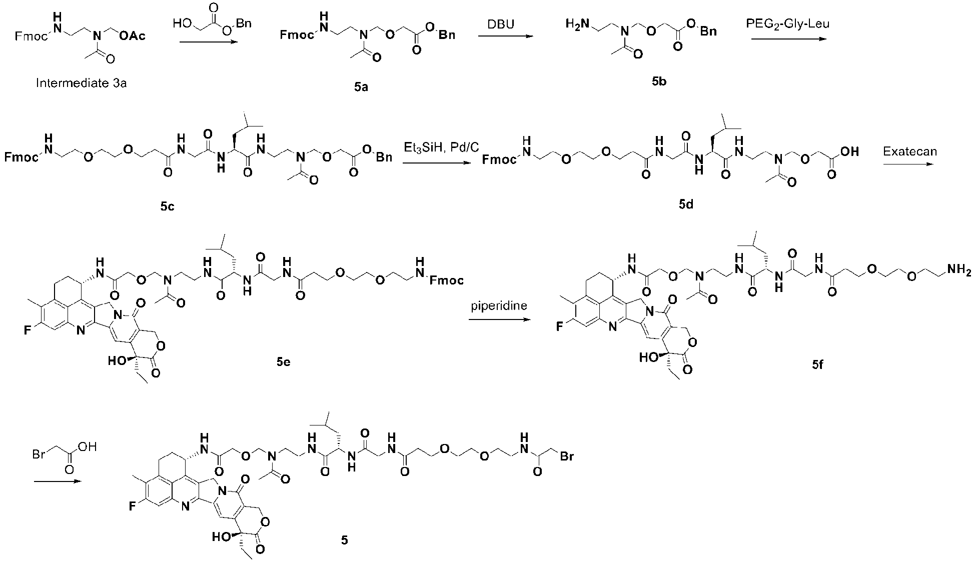

In a reaction flask, Intermediate 3a (1.95 g, 4.92 mmol), DCM (25 mL), benzyl hydroxyacetate (8.17 g, 49.2 mmol), and PPTS (123.6 mg, 0.492 mmol) were added. The reaction solution was heated to reflux under stirring overnight, then EtOAc (200 mL) was added. The mixture was washed with brine (3 x 200 mL), dried over anhydrous MgSO₄, filtered, and concentrated under vacuum, and purified by silica gel column chromatography to afford **5a** as a white powder. (2.43 g, yield 98.3%); LCMS: (M+1)⁺ 503.21 (theoretical value: 502.21).

In a reaction flask, **5a** (200 mg, 0.398 mmol) and DBU (30.3 mg, 0.199 mmol) were added in sequence. The mixture was stirred at room temperature for 1 h to afford **5b,** which was directly used in the next step. To the reaction solution **of 5b,** PPTS (50 mg, 0.298 mmol), DIPEA (60.9 mg, 0.471 mmol), PEG2-Gly-Leu (224 mg, 0.392 mmol), and HATU (298.4 mg, 0.785 mmol) were added. The mixture was stirred to react at room temperature for 2 h, and the solvent was removed under reduced pressure to afford **5c** as a solid (236.8 mg, yield 72.5%); LCMS: (M+1)⁺ 832.41 (theoretical value: 831.41).

Compound **5c** (0.3 g, 0.36 mmol), prepared as described above was dissolved in MeOH (5 mL). After adding palladium on carbon catalyst (90 mg), the mixture was stirred at room temperature. Triethylsilane (0.42 g, 3.61 mmol) was then added, and the mixture was stirred at room temperature for 0.5 h. The insoluble material was removed by filtration through diatomaceous earth, and the solvent was removed from the filtrate under reduced pressure to afford **5d** as a solid (267.5 mg, yield 100%); LCMS: (M+1)⁺ 742.36 (theoretical value: 741.36).

Exatecan (100 mg, 0.188 mmol) was dissolved in DMF (2 mL), then DIPEA (48.6 mg, 0.376 mmol), **5d** (167.5 mg, 0.226 mmol), and HATU (143 mg, 0.376 mmol) were added in sequence. The mixture was stirred to react at room temperature for 2 h, and the solvent was removed under reduced pressure to afford **5e** as a solid (196.4 mg, yield 90.1%); LCMS: (M+1)⁺ 1159.51 (theoretical value: 1158.51).

In a reaction flask, **5e** (196.4 mg, 0.17 mmol) and piperidine (144.4 mg, 1.7 mmol) were added in sequence. The mixture was stirred to react at room temperature for 0.5 h, methyl tert-butyl ether (30 mL) was added, and the mixture was centrifuged. The supernatant was removed, and the solvent was removed under reduced pressure to afford **5f** as a solid (135.6 mg, yield 85.4%), which was directly used in the next step; LCMS: (M+1)⁺ 937.44 (theoretical value: 936.44).

In a reaction flask, DCM (2mL), **5f** (100 mg, 0.107 mmol), bromoacetic acid (17.8 mg, 0.128 mmol) and DIC (16.2 mg, 0.128 mmol) were added in sequence. The mixture was stirred to react at room temperature for 90 min, followed by the addition of TFA (0.1 mL), and the mixture was continued to react for 30 min. The reaction solution was injected into a 25 g C18 pre-column (pre-equilibrated with acetonitrile, then with water, the water phase contains 0.1% TFA), then eluted via medium-pressure reverse-phase C18 chromatography (gradient: 5% to 40% acetonitrile in water over 30 min), lyophilized to afford **5** as a yellow solid (30.1 mg, yield 26.6%); LCMS: (M+1)⁺ 1058.26 (theoretical value: 1056.36).

ADC-5a: Compound **5** and HER2 monoclonal antibody HS627 form an antibody-drug conjugate

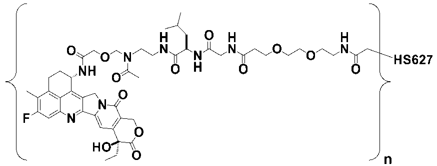

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **5** (0.85mg, 0.8mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose, 0.2g/L polysorbate 20, to affordADC-5a (3.1mg/mL, 2mL).

UV-HPLC calculated average value: n=6.6

ADC-5b: Compound **5** and 5T4 monoclonal antibody IP140B form an antibody-drug conjugate

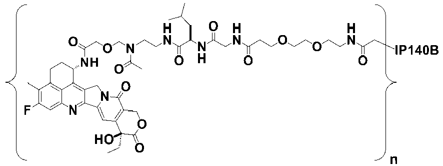

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **5** (0.85mg, 0.8mmol) was dissolved in 0.09 mL of DMA, and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH5.7, to afford ADC-5b (3.3mg/mL, 2mL).

UV-HPLC calculated average value: n=6.5

### Example 6: bromoacetamidoethoxyethoxypropionyl-Gly-Leu-ethylenediamine(Ms)-methyleneoxyacetylexatecan (6)

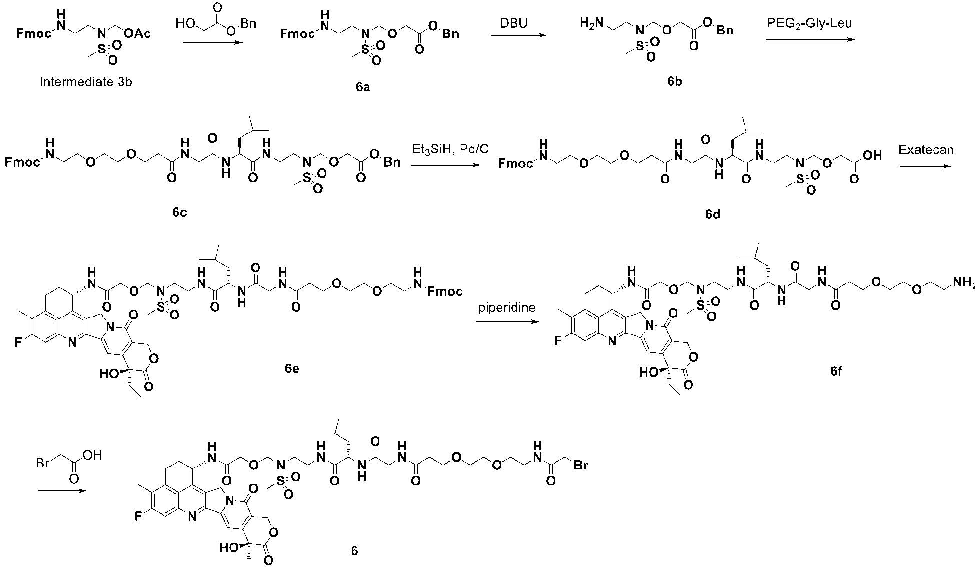

In a reaction flask, Intermediate **3b** (0.5 g, 1.16 mmol), DCM (5 mL), benzyl hydroxyacetate (8.17 g, 49.2 mmol), and PPTS (29.1 mg, 0.116 mmol) were added. The reaction solution was stirred under reflux overnight, cooled to room temperature, and EtOAc (200 mL) was added. The mixture was washed with water (3 x 200 mL), dried over anhydrous MgSO₄, filtered, and concentrated under vacuum, and purified by silica gel column chromatography to afford **6a** as a white powder (536.8 mg, yield 86.2%); LCMS: (M+1)⁺ 539.18 (theoretical value: 538.18).

In a reaction flask, **6a** (200 mg, 0.371 mmol) and DBU (28.3 mg, 0.186 mmol) were added in sequence. The mixture was stirred to react at room temperature for 1 h to afford **6b.** PPTS (31.2 mg, 0.186 mmol), DIPEA (48 mg, 0.371 mmol), PEG₂-Gly-Leu (179.8 mg, 0.316 mmol), and HATU (141.2 mg, 0.371 mmol) were added, and the mixture was stirred to react at room temperature for 2 h. The solvent was removed under reduced pressure to afford **6c** as a solid (215.6 mg, yield 66.9%); LCMS: (M+1)⁺ 868.37 (theoretical value: 867.37).

Compound **6c** (0.15 g, 0.173 mmol), prepared as described above, was dissolved in MeOH (5 mL). Palladium on carbon catalyst (50 mg) was added, and the mixture was stirred at room temperature. Triethylsilane (0.2 g, 1.73 mmol) was then added, and the mixture was continued to stir at room temperature for 0.5 h. The insoluble material was removed by filtration through diatomaceous earth, and the solvent was removed from the filtrate under reduced pressure to afford **6d** as a solid (134.43 mg, yield 100%); LCMS: (M+1)⁺ 778.33 (theoretical value: 777.33).

Exatecan (110 mg, 0.207 mmol) was dissolved in DMF (2 mL), then DIPEA (80.2 mg, 0.62 mmol), **6d** (161 mg, 0.207 mmol), and HATU (157.4 mg, 0.414 mmol) were added in sequence. The mixture was stirred to react at room temperature for 2 h, and the solvent was removed under reduced pressure to afford **6e** as a solid (213.5 mg, yield 86.3%); LCMS: (M+1)⁺ 1195.33 (theoretical value: 1194.47).

In a reaction flask, **6e** (140 mg, 0.117 mmol) and piperidine (99.7 mg, 1.17 mmol) were added in sequence. The mixture was stirred to react at room temperature for 0.5 h, methyl tert-butyl ether (30 mL) was added, and the mixture was centrifuged. The supernatant was removed, and the solvent was removed under reduced pressure to afford **6f** as a solid (80 mg, yield 70.2%) which was directly used in the next step; LCMS: (M+1)⁺ 973.41 (theoretical value: 972.41).

In a reaction flask, DCM (2 mL), **6f** (80 mg, 0.082 mmol), bromoacetic acid (13.7 mg, 0.099 mmol) and DIC (12.5 mg, 0.099 mmol) were added in sequence. The mixture was stirred to react at room temperature for 90 min, followed by the addition of 0.1 mL of TFA, and the mixture was continued to react for 30 min. Finally, the reaction solution was injected into a 25 g C18 pre-column (pre-equilibrated with acetonitrile, then with water, the water phase contains 0.1% TFA), then eluted via medium-pressure reverse-phase C18 chromatography (gradient: 5% to 40% acetonitrile in water over 30 min), lyophilized to afford **6** as a yellow solid (30.1 mg, yield 26.6%); LCMS: (M+1)⁺ 1094.33 (theoretical value: 1092.33).

ADC-6a: Compound **6** and HER2 monoclonal antibody HS627 form an antibody-drug conjugate

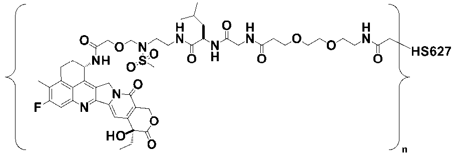

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **6** (0.87mg, 0.8mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose, 0.2g/L polysorbate 20, to affordADC-6a (3.2mg/mL, 2mL).

UV-HPLC calculated average value: n=6.3

ADC-6b: Compound **6** and 5T4 monoclonal antibody IP140B form an antibody-drug conjugate

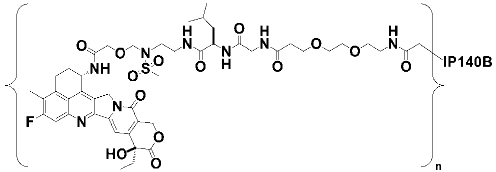

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **6** (0.87mg, 0.8mmol) was dissolved in 0.09 mL of DMA, and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH5.7, to affordADC-6b (3.3mg/mL, 2mL).

UV-HPLC calculated average value: n=6.6

### Example 7: 7-(N-(2,2,2-trifluoroethyl)-N-(bromoacetamidoethoxyethoxypropionyl-Gly-Phe-Gly-methylene)amino)ethyl-10,11-methylenedioxycamptothecin (7)

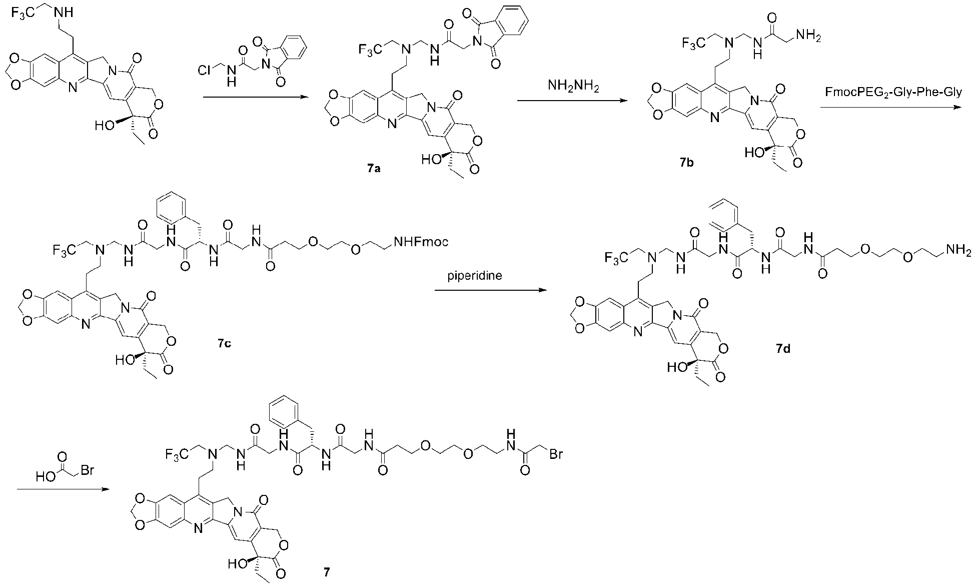

To a reaction flask, anhydrous DCM (2 mL), 7-(N-(2,2,2-trifluoroethyl)amino)ethyl-10,11-methylenedioxycamptothecin (100 mg, 162.5 µmol), Intermediate **5a** (61.58 mg, 243.75 µmol), and DIPEA (42.01 mg, 325 µmol) were added. The reaction was stirred at 40°C for 12 h, the solvent was removed under reduced pressure, and the residue was purified by silica gel column chromatography to afford Compound **7a** (100 mg, yield 83.9%); LCMS: (M+1)⁺ 734.41 (theoretical value: 733.41).

In a reaction flask, anhydrous methanol (2 mL), Intermediate **7a** (100 mg, 136.3 µmol), and hydrazine hydrate (40.94 mg, 817.82 µmol) were added. The reaction was stirred at 60°C for 12 h. Saturated brine (10 mL) was added to the reaction solution, and the mixture was extracted four times with DCM/i-PrOH = 4:1, dried over anhydrous sodium sulfate, and the solvent was removed under reduced pressure to afford Compound **2b** (70 mg, yield 85%, HPLC 94%); LCMS: (M+1)⁺ 604.16 (theoretical value: 603.16).

In a single-necked flask charged with anhydrous DMF (1.5 mL), FmocPEG₂-Gly-PheOH (70.01 mg, 115.98 µmol), HATU (66.15 mg, 173.97 µmol), and TEA (22.48 mg, 173.97 µmol) were added. The mixture was stirred at room temperature for 5 min, followed by the addition of Compound **2b** (70 mg, 115.98 µmol). The mixture was continued to stri at room temperature for 2 h. Saturated brine (10 mL) was added to the reaction solution, and the mixture was extracted four times with DCM/i-PrOH (4:1), dried over anhydrous sodium sulfate, filtered, and the solvent was removed under reduced pressure to afford Compound **7c** (90 mg, yield 65.3%); LCMS: (M+1)⁺ 1189.42 (theoretical value: 1188.42).

To a reaction flask, Compound **7c** (90 mg, 75.68 µmol), anhydrous DCM (1.5 mL), and piperidine (64.44 mg, 756.8 µmol) were added. The mixture was stirred at room temperature for 30 min, methyl tert-butyl ether was added to the reaction solution, and the mixture was centrifuged to separate the solid. This process was repeated twice, and the white precipitate was dried to afford Compound **7d** (45 mg, yield 61.5%, HPLC 94%); LCMS: (M+1)⁺ 967.23 (theoretical value: 966.23).

In a flask charged with anhydrous DCM (1.5 mL), bromoacetic acid (9.7 mg, 69.81 µmol) and DIC (8.81 mg, 69.81 µmol) were added. The mixture was stirred at room temperature for 5 min, followed by the addition of Compound **7d** (45 mg, 46.54 µmol). The mixture was continued to stri to react at room temperature for 1 h, and the residue was purified by silica gel column chromatography to afford **7** (27 mg, yield 53.3%, HPLC 97%); LCMS: (M+1)⁺1087.35 (theoretical value: 1086.35).

ADC-7a: Compound **7** and HER2 monoclonal antibody HS627 form an antibody-drug conjugate

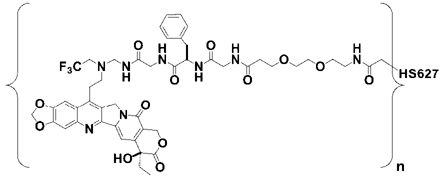

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **7** (0.87mg, 0.8mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose, 0.2g/L polysorbate 20, to affordADC-7a (3.1mg/mL, 2mL).

UV-HPLC calculated average value: n=7.5

ADC-7b: Compound **7** and 5T4 monoclonal antibody IP140B form an antibody-drug conjugate

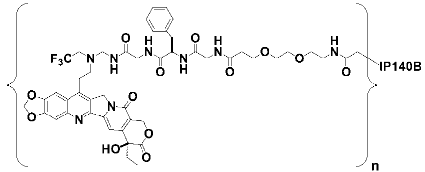

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound 7 (0.87mg, 0.8mmol) was dissolved in 0.09 mL of DMA, and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH5.7, to afford ADC-7b (3.3mg/mL, 2mL).

UV-HPLC calculated average value: n=7.6

### Example 8: 7-(N-(2,2,2-trifluoroethyl)-N-(bromoacetamidoethoxyethoxypropionyl-Gly-Gly-Val-methylene)amino)ethyl-10,11-methylenedioxycamptothecin (8)

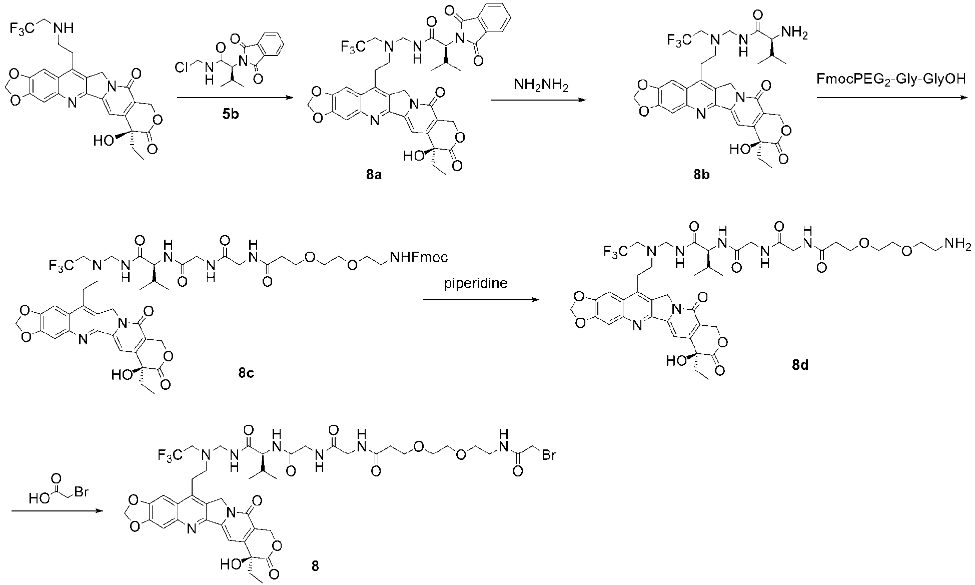

To a reaction flask, anhydrous DCM (2 mL), 7-(N-(2,2,2-trifluoroethyl)amino)ethyl-10,11-methylenedioxycamptothecin (90 mg, 146.2 µmol), Intermediate **5b** (64.64 mg, 219.3 µmol), and DIPEA (37.79 mg, 292.4 µmol) were added. The reaction was stirred at 40°C for 12 h, the solvent was removed under reduced pressure, and the residue was purified by silica gel column chromatography to afford Compound **8a** (100 mg, yield 88.2%, HPLC 95%); LCMS: (M+1)⁺ 776.25 (theoretical value: 775.25).

In a reaction flask, anhydrous methanol (2 mL) was add, then Compound **8a** (100 mg, 128.91 µmol), and hydrazine hydrate (38.72 mg, 773.46 µmol) were added. The reaction solution was stirred at 60°C for 12 h, saturated brine (10 mL) was added to the reaction solution, and the mixture was extracted four times with DCM/i-PrOH (4:1), dried over anhydrous sodium sulfate, filtered, and the solvent was concentrated to afford Compound **8b** (75 mg, yield 90%, HPLC 94%); LCMS: (M+1)⁺646.3 (theoretical value: 645.3).

In a reaction flask, anhydrous DMF (1.5 mL), FmocPEG₂-GLy-Gly-OH (59.65 mg, 116.16 µmol), HATU (66.26 mg, 174.24 µmol), and TEA (17.63 mg, 174.24 µmol) were added. The reaction solution was stirred at room temperature for 5 min, followed by the addition of Compound **8b** (75 mg, 116.16 µmol). The mixture was continued to react at room temperature for 2 h. Saturated brine (10 mL) was added to the reaction solution, and the mixture was extracted four times with DCM/i-PrOH (4: 1), dried over anhydrous sodium sulfate, filtered, and the solvent was concentrated to afford Compound **8c** (82 mg, yield 61.8%, HPLC 96%); LCMS: (M+1)⁺1141.41 (theoretical value: 1140.41).

To a reaction flask, Compound **8c** (82 mg, 71.85 µmol), anhydrous DCM (1.5 mL), and piperidine (61.18 mg, 718.5 µmol) were added. The reaction solution was stirred at room temperature for 30 min, methyl tert-butyl ether was added to the reaction solution, and the mixture was centrifuged to seperate the solid. This process was repeated twice, and the white precipitate was dried to afford Compound **8d** (52 mg, yield 86.1%, HPLC 94%); LCMS: (M+1)⁺919.2 (theoretical value: 918.2).

In a reaction flask, anhydrous DCM (1.5 mL), bromoacetic acid (11.79 mg, 84.88 µmol), and DIC (10.71 mg, 84.88 µmol) were added. The reaction solution was stirred at room temperature for 5 min, followed by the addition of Compound **8d** (52 mg, 56.58 µmol).The reaction solution was continued to react at room temperature for 1 h, the solvent was concentrated under reduced pressure, and the residue was purified by silica gel column chromatography to afford **8** (42 mg, yield 71.4%, HPLC 98%); LCMS: (M+1)⁺1039.23 (theoretical value: 1038.23).

ADC-8a: Compound **8** and HER2 monoclonal antibody HS627 form an antibody-drug conjugate

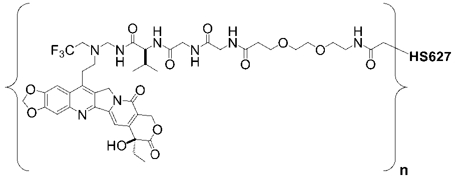

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **8** (0.83mg, 0.8mmol) was dissolved in 0.08 mL of DMA, and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose, 0.2g/L polysorbate 20, to affordADC-8a (3.1mg/mL, 2mL).

UV-HPLC calculated average value: n=7.6

ADC-8b: Compound **8** and 5T4 monoclonal antibody IP140B form an antibody-drug conjugate

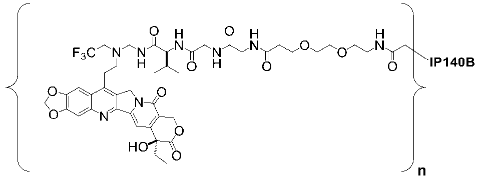

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **8** (0.83mg, 0.8mmol) was dissolved in 0.08 mL of DMA, and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH5.7, to afford ADC-8b (3.3mg/mL, 2mL).

UV-HPLC calculated average value: n=7.6

### Example 9: 7-(N-(2,2,2-trifluoroethyl)-N-(bromoacetamidoethoxyethoxypropionyl-Gly-Val-Ala-methylene)amino)ethyl-10,11-methylenedioxycamptothecin (9)

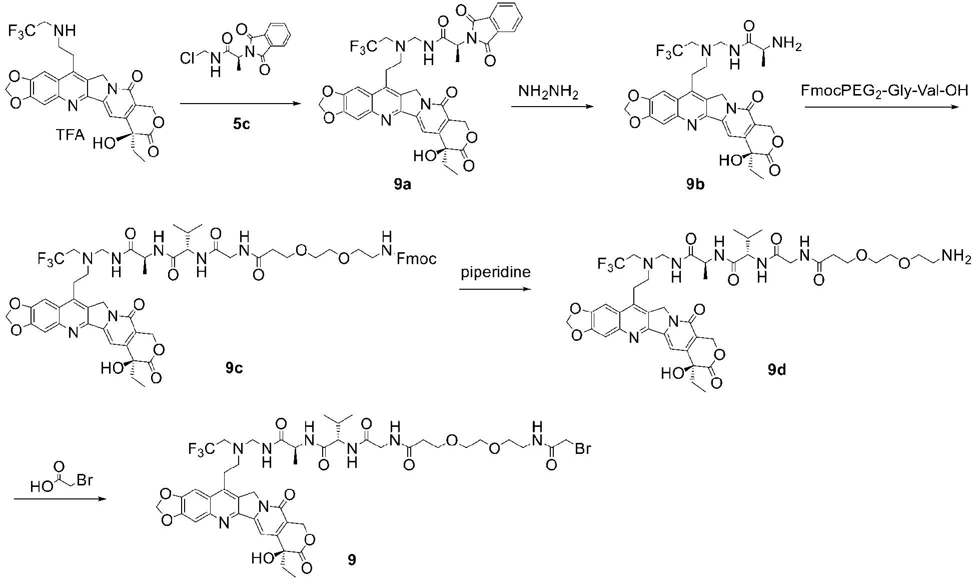

To a reaction flask, anhydrous DCM (2 mL), 7-(N-(2,2,2-trifluoroethyl)amino)ethyl-10,11-methylenedioxycamptothecin (90 mg, 146.2 µmol), Intermediate **5c** (61.34 mg, 219.3 µmol), and DIPEA (37.79 mg, 292.4 µmol) were added. The reaction was stirred at 40°C for 12 h, the solvent was removed under reduced pressure, and the residue was purified by silica gel column chromatography to afford Compound **9a** (95 mg, yield 89.2%, HPLC 95%); LCMS: (M+1)⁺748.22 (theoretical value: 747.22).

To a single-necked flask charged with anhydrous methanol (2 mL), Compound **9a** (95 mg, 128.91 µmol) and hydrazine hydrate (38.72 mg, 773.46 µmol) were added. The reaction solution was stirred at 60°C for 12 h, then saturated brine (10 mL) was added to the reaction solution. The mixture was extracted four times with DCM/i-PrOH (4:1), dried over anhydrous sodium sulfate, filtered, and concentrated to afford Compound **9b** (75 mg yield 88.5, HPLC 93%); LCMS: (M+1)⁺618.56 (theoretical value: 617.56).

To a single-necked flask charged with anhydrous DMF (1.5 mL), FmocPEG₂-GLy-Gly-OH (59.65 mg, 116.16 µmol), HATU (66.26 mg, 174.24 µmol), and TEA (17.63 mg, 174.24 µmol) were added. The reaction solution was stirred at room temperature for 5 min, followed by the addition of Compound **9b** (75 mg, 116.16 µmol). The mixture was continued to react at room temperature for 2 h. Saturated brine (10 mL) was added to the reaction solution, and the mixture was extracted four times with DCM/i-PrOH (4:1), dried over anhydrous sodium sulfate, and concentrated to afford Compound 9c (82 mg, yield 78.6%, HPLC 94%); LCMS: (M+1)⁺1155.43 (theoretical value: 1154.43).

To a reaction flask, Compound **9c** (82 mg, 71.85 µmol), anhydrous DCM (1.5 mL), and piperidine (61.18 mg, 718.5 µmol) were added. The mixture was stirred to react at room temperature for 30 min, and methyl tert-butyl ether was added to afford a white precipitate. The mixture was centrifuged to separate the solid, and this process was repeated twice. The white precipitate was dried to afford Compound **9d** (52 mg, yield 85.6%, HPLC 95%); LCMS: (M+1)⁺933.35 (theoretical value: 932.35).

To a single-necked flask charged with anhydrous DCM (1.5 mL), bromoacetic acid (11.79 mg, 84.88 µmol) and DIC (10.71 mg, 84.88 µmol) were added. The mixture was stirred at room temperature for 5 min, followed by the addition of Compound **9d** (52 mg, 56.58 µmol). The mixture was continued to stir to react at room temperature for 1 h, then purified by silica gel column chromatography to afford 9 (50 mg, yield 85.5%, HPLC 96%); LCMS: (M+1)⁺1055.27 (theoretical value: 1054.27).

ADC-9a: Compound **9** and HER2 monoclonal antibody HS627 form an antibody-drug conjugate

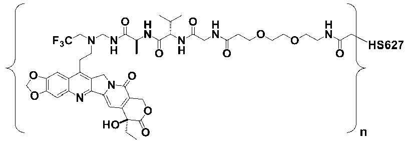

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **9** (0.84mg, 0.8mmol) was dissolved in 0.08 mL of DMA, and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose, 0.2g/L polysorbate 20, to affordADC-9a (3.1mg/mL, 2mL).

UV-HPLC calculated average value: n=7.7

ADC-9b: Compound **9** and 5T4 monoclonal antibody IP140B form an antibody-drug conjugate

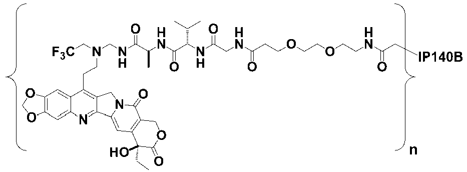

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **9** (0.84mg, 0.8mmol) was dissolved in 0.08 mL of DMA, and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH5.7, to afford ADC-9b (3.2mg/mL, 2mL).

UV-HPLC calculated average value: n=7.6

### Example 10: 10-(bromoacetamidoethoxyethoxypropionyl-Gly-Leu-ethylenediamine(Ms)-methylene)oxy-7-ethylcamptothecin(10)

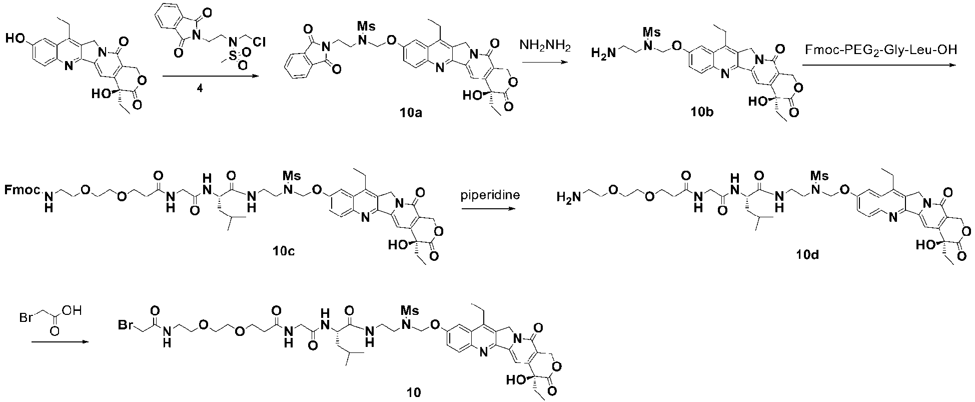

In a reaction flask, 7-ethyl-10-hydroxycamptothecin (0.1 g, 0.255 mmol), DCM (5 mL), DIEA (65.9 mg, 0.51 mmol), and Intermediate 4 (84.8 mg, 0.268 mmol) were added. The reaction solution was stirred to react at 40°C for 1 h, the solvent was removed under reduced pressure, and the residue was purified by silica gel column chromatography to afford Compound **10a** (145.1 mg, yield 84.6%); LCMS: (M+1)⁺ 673.71 (theoretical value: 672.71).

To a reaction flask, Compound **10a** (0.1 g, 0.149 mmol), MeOH (5 mL), and 80% hydrazine hydrate (55.8 mg, 0.892 mmol) were added. The reaction solution was stirred to react at 60°C for 2 h, then saturated brine (20 mL) was added. The mixture was extracted three times with dichloromethane (10 mL x 3), and the combined organic phases were dried. The solvent was removed under reduced pressure to afford Compound **10b** (73.2 mg, yield 90.8%); LCMS: (M+1)⁺ 543.61 (theoretical value: 542.61).

Compound 10b (100 mg, 0.184 mmol), prepared as described above, was dissolved in DMF (2 mL), then DIPEA (47.6 mg, 0.369 mmol), Fmoc-PEG₂-GLy-Leu-OH (105 mg, 0.184 mmol), and HATU (70.1 mg, 0.184 mmol) were added in sequence. The reaction solution was stirred to react at room temperature for 2 h, the solvent was removed under reduced pressure, and the residue was purified by silica gel column chromatography to afford Compound **10c** (184.5 mg, yield 91.5%); LCMS: (M+1)⁺ 1195.25 (theoretical value: 1194.25).

In a reaction flask, Compound **10c** (100 mg, 0.091 mmol) and piperidine (77.8 mg, 0.914 mmol) were added in sequence. The mixture was stirred to react at room temperature for 0.5 h, methyl tert-butyl ether (30 mL) was added, and the mixture was centrifuged. The supernatant was removed, and the process was repeated three times to afford Compound **10d** (80 mg, yield 100%) LCMS: (M+1)⁺ 873.01 (theoretical value: 872.01).

In a reaction flask, DCM (2 mL), Compound **10d** (70 mg, 0.08 mmol), bromoacetic acid (22.3 mg, 0.16 mmol), and DIC (20.3 mg, 0.16 mmol) were added in sequence. The mixture was stirred to react at room temperature for 90 min, the solvent was removed under reduced pressure, and the residue was purified by silica gel column chromatography to afford **10** as a light yellow solid (65.3 mg, yield 81.9%); LCMS: (M+1)⁺ 994.33 (theoretical value: 992.94).

ADC-10a: Compound **10** and HER2 monoclonal antibody HS627 form an antibody-drug conjugate

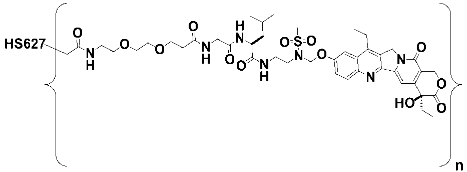

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **10** (0.79mg, 0.8mmol) was dissolved in 0.08 mL of DMA, and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose, 0.2g/L polysorbate 20, to afford ADC-10a (3.2mg/mL, 2mL).

UV-HPLC calculated average value: n=7.1

ADC-10b: Compound **10** and 5T4 monoclonal antibody IP140B form an antibody-drug conjugate

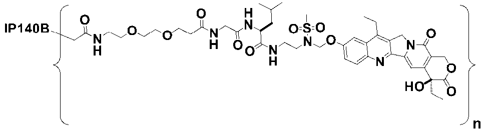

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **10** (0.79mg, 0.8mmol) was dissolved in 0.08 mL of DMA, and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH5.7, to afford ADC-10b (3.2mg/mL, 2mL).

UV-HPLC calculated average value: n=7.2

### Example 11: 2-(N-methyl-N-(bromoacetamidoethoxyethoxypropionyl-Gly-Leu-Gly-methylene)amino)oxyacety exatecan (11)

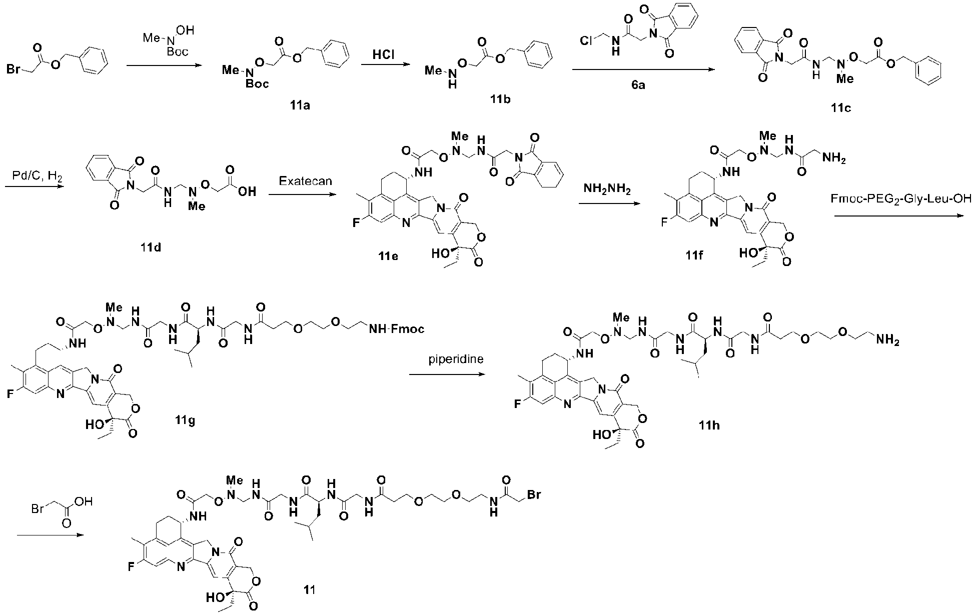

Boc-N-methylhydroxylamine hydrochloride (15 g, 101.9 mmol) was dissolved in isopropanol (100 mL), benzyl bromoacetate (27.709 g, 121.4 mmol) and DIPEA (15.7 g, 121.7 mmol) were added. The mixture was stirred under argon atmosphere and warmed to 85°C, and reacted for 3 h. The reaction solution was concentrated, dissolved by adding ethyl acetate (100 mL) washed with saturated brine (30mL*3), dried over anhydrous sodium sulfate, and purified by silica gel column chromatography to afford Intermediate **11a** (24 g, yield 80%, HPLC 98%); LCMS: (M+1)+ 296.08 (theoretical value: 295.14).

In a 100 mL single-necked flask, dichloromethane (20 mL) and **11a** (2 g, 6.8 mmol) were added. After stirring uniformly, a solution of 4N hydrochloric acid in ethyl acetate (10 mL) was added dropwise. The mixture was stirred to react at room temperature overnight, concentrated to afford **11b** (1.32 g, yield 100%, HPLC 96%); LCMS: (M+1)⁺ 195.99 (theoretical value: 195.06).

**5a** (1 g, 3.9 mmol) was dissolved in DCM (5 mL), then **11b** (0.77 g, 3.9 mmol) and DIPEA (290.79 mg, 2.24 mmol) were added. The mixture was sealed in a tube, heated to 50°C for an overnight reaction, cooled to room temperature, concentrated, and purified by silica gel column chromatography to afford **11c** (1.2 g, yield 75%, HPLC 91%); LCMS: (M+1)⁺412.06 (theoretical value: 411.14).

In a 100 mL single-necked flask, **11c** (1.2 g, 2.9 mmol), methanol (5 mL), and 10% palladium on carbon (0.1 g) were added. After purging with hydrogen, the mixture was reacted at room temperature and atmospheric pressure for 2 h, filtered, and concentrated to afford **11d** (0.91 g, yield 97%, HPLC 93%); LCMS: (M+1)+ 322.01 (theoretical value: 321.10).

To a reaction flask, exatecan mesylate (0.26 g, 0.5 mmol), DMF (2 mL), HATU (0.19 g, 0.5 mmol), DIPEA (0.129 g, 1 mmol), and 11d (0.176 g, 0.55 mmol) were added. The mixture was stirred to react at room temperature for 15 h, diluted with DCM (200 mL), washed with saturated sodium chloride solution, dried over anhydrous sodium sulfate, and purified by silica gel column chromatography to afford **11e** as a yellow solid (0.46 g, yield 66%, HPLC 92%); LCMS: (M+1)⁺ 739.11 (theoretical value: 738.24).

**11e** (0.1 g, 0.12 mmol) was dissolved in MeOH (5 mL), then hydrazine hydrate (66 mg, 1.32 mmol) was added. The mixture was sealed in a tube, heated to 60°C and reacted for 2 h, and cooled to room temperature. The reaction solution was poured into saturated brine (100 mL) and extracted with ethyl acetate (200 mL), then concentrated to afford **11f** (0.06 g, yield 72%, HPLC 94%); LCMS: (M+1)⁺ 609.14 (theoretical value: 608.24).

**11f** (60 mg, 0.098 mmol) was weighted and dissolved in DMF (2 mL), then DIPEA (25 mg, 0.197 mmol) was added. Under stirring, Fmoc-PEG₂-Gly-Leu-OH (55.76 mg, 0.098 mmol) and HATU (37.5 mg, 0.098 mmol) were added in sequence. The mixture was stirred to react at room temperature for 1 h, concentrated, and purified by silica gel column chromatography to afford Compound **11g** (85 mg, yield 75%, HPLC 91%); LCMS: (M+1)⁺ 1160.27 (theoretical value: 1159.50).

To a 10 mL single-necked flask, **11g** (85 mg, 0.069 mmol) was added and dissolved in DMF (1 mL). Piperidine (100 µL) was then added, and the mixture was reacted at room temperature for 1 h. Methyl tert-butyl ether (20 mL) was added, the mixture was centrifuged, the supernatant was removed, and the residue was dried to afford **11h** (31 mg, yield 46%, HPLC 93%); LCMS: (M+1)⁺ 938.02 (theoretical value: 937.43).

In a reaction flask, DCM (3 mL), **11h** (31 mg, 0.032 mmol), bromoacetic acid (8.9 mg, 0.064 mmol), and DIC (8.01 mg, 0.064 mmol) were added in sequence. The mixture was stirred to react at room temperature for 90 min, concentrated, and purified by silica gel column chromatography to afford **11** as a yellow solid (25 mg, yield 63%, HPLC 95%); LCMS: (M+1)⁺ 1058.24 (theoretical value: 1057.36).

ADC-11a: Compound **11** and HER2 monoclonal antibody HS627 form an antibody-drug conjugate

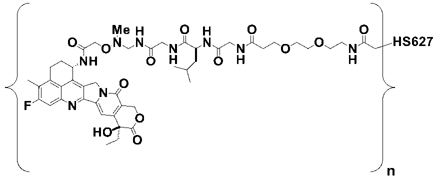

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **11** (0.85mg, 0.8mmol) was dissolved in 0.08 mL of DMA, and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose, 0.2g/L polysorbate 20, to afford ADC-11a (3.3mg/mL, 2mL).

UV-HPLC calculated average value: n=7.5

ADC-11b: Compound **11** and 5T4 monoclonal antibody IP140B form an antibody-drug conjugate

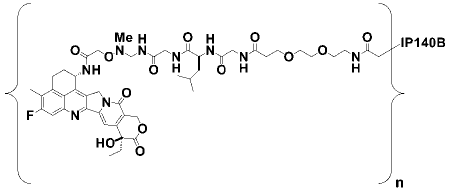

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **11** (0.85mg, 0.8mmol) was dissolved in 0.08 mL of DMA, and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH5.7, to afford ADC-1 1b (3.2mg/mL, 2mL).

UV-HPLC calculated average value: n=7.6

### Example 12: 2-(N-methyl-N-(bromoacetamidoethoxyethoxypropionyl-Gly-Val-Ala-methylene)amino)oxyacety exatecan (12)

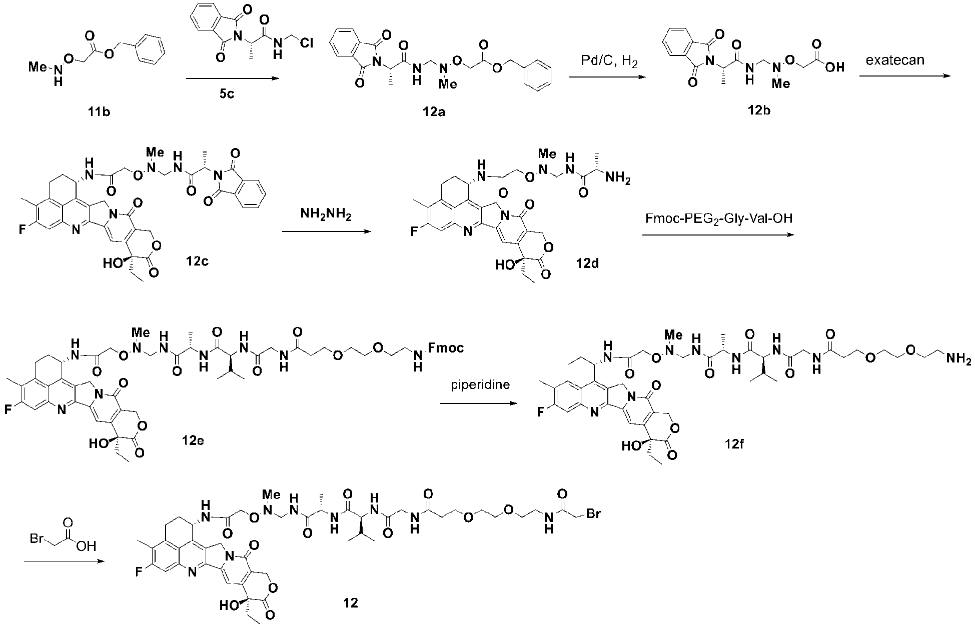

**5c** (0.2 g, 0.75 mmol) was taken and dissolved in DCM (5 mL), then **11b** (146 mg, 0.75 mmol) and DIPEA (290.79 mg, 2.24 mmol) were added. The mixture was sealed in a tube, heated to 50°C and reacted overnight, cooled to room temperature, and purified by silica gel column chromatography to afford the product **12a** (0.27 g, yield 84%, HPLC 95%); LCMS: (M+1)⁺ 426.21 (theoretical value: 425.36).

In a 100 mL single-necked flask, **12a** (0.27 g, 0.67 mmol), methanol (5 mL), and 10% palladium on carbon (0.1 g) were added. After purging with hydrogen, the mixture was reacted at room temperature and atmospheric pressure for 2 h, filtered, and concentrated to afford **12b** (0.20 g, yield 95%, HPLC 93%); LCMS: (M+1)⁺ 336.08 (theoretical value: 335.11).

To a reaction flask, exatecan mesylate (0.26 g, 0.5 mmol), DMF (2 mL), HATU (0.19 g, 0.5 mmol), DIPEA (0.129 g, 1 mmol), and **12b** (200 mg, 0.59 mmol) were added. The mixture was stirred to react at room temperature for 15 h, DCM (200 mL) was added, then the mixture was washed with saturated sodium chloride solution, dried over anhydrous sodium sulfate, and purified by silica gel column chromatography to afford **12c** as a yellow solid (0.3 g, yield 81%, HPLC 92%); ¹H NMR (500 MHz, DMSO-*d*₆) δ 8.72-8.66 (m, 1H), 8.49 (d, *J*=8.8 Hz, 1H), 7.79-7.71 (m, 1H), 7.70 (s, 3H), 7.28 (s, 1H), 6.51 (s, 1H), 5.39 (d, *J*=17.2 Hz, 3H), 5.23-5.19 (s, 1H), 5.00 (s, 1H), 4.68 (q, *J*=7.3 Hz, 1H),4.18 (s, 3H), 4.12-4.01 (m, 1H), 3.18 (d, *J*=17.1 Hz, 1H), 3.07 (d, *J*=17.6 Hz, 1H), 2.37 (s, 3H), 2.00-1.92 (m, 2H), 1.87-1.80 (m, 2H), 1.29 (m, *J*=7.2 Hz, 3H), 1.25-1.05 (m, 1H), 0.84 (q, *J*=13.5, 7.5 Hz, 3H); LCMS: (M+1)⁺753.12 (theoretical value: 752.26).

**12c** (0.1 g, 0.13 mmol) was dissolved in MeOH (5 mL), then hydrazine hydrate (39.9 mg, 0.79 mmol) was added. The mixture was sealed in a tube, heated to 60°C and react for 2 h, and cooled to room temperature. The reaction solution was poured into saturated brine (100 mL) and extracted with ethyl acetate (200 mL), then concentrated to afford **12d** (0.07 g, 77%, HPLC 92%); ¹H NMR (500 MHz, DMSO-*d₆*) δ 8.91 (t, *J*=6.0 Hz, 1H), 8.58 (d, *J*=8.9 Hz, 1H), 8.17 (s, 3H), 7.66 (d, *J*=10.8 Hz, 1H), 7.27 (s, 1H), 6.52 (s, 1H), 5.58 (td, *J*=8.3, 7.9, 4.8 Hz, 1H), 5.39 (s, 2H), 5.19 (d, *J*=19.0 Hz, 1H), 5.01 (d, *J*=18.9 Hz, 1H), 4.35 (dd, *J*=13.2, 6.3 Hz, 1H), 4.25 (dd, *J*=12.1, 4.7 Hz, 1H), 4.20 (d, *J*=2.3 Hz, 2H), 3.97-3.88 (m, 1H), 3.18 (dt, *J*=17.1, 5.9 Hz, 1H), 3.13-3.03 (m, 1H), 2.53 (s, 3H), 2.31 (d, *J*=1.9 Hz, 3H), 2.26 - 2.09 (m, 2H), 1.93-1.77 (m, *J*=7.2 Hz, 2H), 1.37 (d, *J*=6.9 Hz, 3H), 0.86 (t, *J*=7.3 Hz, 3H); LCMS: (M+1)⁺623.15 (theoretical value: 622.26).

**12d** (70 mg, 0.112 mmol) was weighted and dissolved in DMF (2 mL), then DIPEA (29 mg, 0.224 mmol) was added. Under stirring, Fmoc-PEG₂-Gly-Val-OH (62.16 mg, 0.112 mmol) and HATU (42.56 mg, 0.112 mmol) were added in sequence. The mixture was stirred to react at room temperature for 1 h, concentrated, and purified by silica gel column chromatography to afford Compound **12e** (81 mg, yield 62%, HPLC 91%); ¹H NMR (500 MHz, DMSO-*d₆*) δ 8.50 (d, *J*=8.9 Hz, 1H), 8.24 (t, *J*=6.3 Hz, 1H), 8.11 (t, *J*=5.7 Hz, 1H), 7.99 (d, *J*=7.2 Hz, 1H), 7.86 (d, *J*=7.5 Hz, 2H), 7.74 (dd, *J*=9.8, 7.4 Hz, 2H), 7.67 (d, *J=*7.5 Hz, 2H), 7.39 (t, *J*=7.4 Hz, 2H), 7.35-7.26 (m, 4H), 6.52 (s, 1H), 5.75 (s, 2H), 5.59 (dt, *J*=9.2, 6.1 Hz, 1H), 5.41 (d, *J*=2.0 Hz, 2H), 5.25 (d, *J*=18.9 Hz, 1H), 5.09 (d, *J*=18.8 Hz, 1H), 4.33-3.99 (m, 8H), 3.70 (m, *J*=16.6, 5.7 Hz, 2H), 3.57 (t, *J*=6.5 Hz, 2H), 3.46 (s, 4H), 3.39 (d, *J*=6.0 Hz, 2H), 3.26-3.06 (q, *J*=6.1 Hz, 5H), 2.48 (d, *J*=2.4 Hz, 3H), 2.41-2.29 (m, 5H), 2.17 (q, *J*=6.5 Hz, 2H), 1.93-1.77 (m, 3H), 1.14 (d, *J*=7.1 Hz, 3H), 0.86 (t, *J*=7.3 Hz, 3H), 0.82-0.66 (m, 6H); LCMS: (M+1)⁺ 1160.24 (theoretical value: 1159.50).

In a reaction flask, **12e** (81 mg, 0.069 mmol) was added, DMF (1 mL) and piperidine (100 µL) was then added, and the mixture was reacted at room temperature for 1 h. Methyl tert-butyl ether (20 mL) was added, the mixture was centrifuged, the supernatant was removed, and the residue was dried to afford **12f** (32 mg, yield 49%, HPLC 93%); ¹H NMR (500 MHz, DMSO-*d*₆) δ 8.53 (d, *J*=8.9 Hz, 1H), 8.28 (t, *J*=6.3 Hz, 1H), 8.13 (t, *J*=5.7 Hz, 1H), 8.01 (d, *J*=7.2 Hz, 1H), 7.77 (t, *J*=10.2 Hz, 5H), 7.30 (s, 1H), 6.53 (s, 1H), 5.60 (dt, *J*=8.9, 6.0 Hz, 1H), 5.41 (s, 2H), 5.27 (d, *J*=18.9 Hz, 1H), 5.13 (d, *J*=19.0 Hz, 1H), 4.28 (dd, *J*=13.2, 6.7 Hz, 1H), 4.24-4.12 (m, 3H), 4.12-4.03 (m, 2H), 3.79-3.64 (m, 2H), 3.63-3.48 (m, 8H), 3.27-3.08 (m, 2H), 2.97 (h, *J*=5.7 Hz, 2H), 2.47 (s, 3H), 2.37 (dd, *J*=7.8, 4.3 Hz, 5H), 2.18 (q, *J*=6.4 Hz, 2H), 1.85 (qq, *J*=14.0, 7.2 Hz, 3H), 1.15 (d, *J*=7.1 Hz, 3H), 0.86 (t, *J*=7.3 Hz, 3H), 0.71 (dd, *J*=13.3, 6.8 Hz, 6H); LCMS: (M+1)⁺ 938.02 (theoretical value: 937.43).

In a reaction flask, DCM (3 mL), **12f** (32 mg, 0.034 mmol), bromoacetic acid (9.5 mg, 0.068 mmol), and DIC (8.6 mg, 0.068 mmol) were added in sequence. The mixture was stirred to react at room temperature for 90 min, concentrated, and purified by silica gel column chromatography to afford **12** as a yellow solid (22 mg, yield 61%, HPLC 95%); ¹H NMR (500 MHz, DMSO-*d*₆) δ 8.49 (d, *J*=8.9 Hz, 1H), 8.33 (t, *J*=5.7 Hz, 1H), 8.24 (t, *J*=6.2 Hz, 1H), 8.11 (t, *J*=5.8 Hz, 1H), 7.98 (d, *J*=7.1 Hz, 1H), 7.75 (d, *J*=8.5 Hz, 1H), 7.70 (d, *J*=10.8 Hz, 1H), 7.28 (s, 1H), 5.64-5.54 (m, 1H), 5.40 (s, 2H), 5.23 (d, *J*=18.9 Hz, 1H), 5.04 (d, *J*=18.9 Hz, 1H), 4.33-3.97 (m, 6H), 3.85 (s, 2H), 3.70 (qd, *J*=16.5, 5.7 Hz, 3H), 3.57 (t, *J*=6.5 Hz,4H), 3.52-3.37 (m, 4H), 3.26-3.17 (m, 4H), 3.14-3.06 (m, 1H), 2.47 (s, 3H), 2.41-2.28 (m, 5H), 2.24-2.11 (m, *J*=6.9, 6.0 Hz, 2H), 1.84 (dh, *J*=20.8, 7.0 Hz, 3H), 1.14 (d, *J*=7.1 Hz, 3H), 0.85 (t, *J*=7.3 Hz, 3H), 0.70 (dd, *J*=11.4, 6.8 Hz, 6H); LCMS: (M+1)⁺ 1058.24 (theoretical value: 1057.36).

ADC-12a: Compound **12** and HER2 monoclonal antibody HS627 form an antibody-drug conjugate

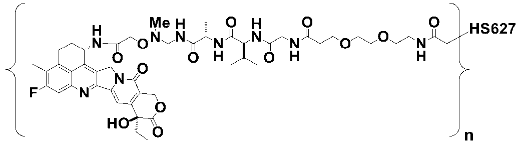

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **12** (0.85mg, 0.8mmol) was dissolved in 0.08 mL of DMA, and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose, 0.2g/L polysorbate 20, to afford ADC-12a (3.3mg/mL, 2mL).

UV-HPLC calculated average value: n=7.6

ADC-12b: Compound **12** and 5T4 monoclonal antibody IP140B form an antibody-drug conjugate

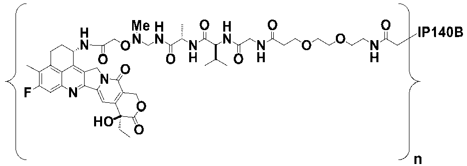

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **12** (0.85mg, 0.8mmol) was dissolved in 0.08 mL of DMA, and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH5.7, to afford ADC-12b (3.2mg/mL, 2mL).

UV-HPLC calculated average value: n=7.7

### Example 13: 2-(N-methyl-N-(bromoacetamidoethoxyethoxypropionyl-Gly-Gly-Val-methylene)amino)oxyacety exatecan (13)

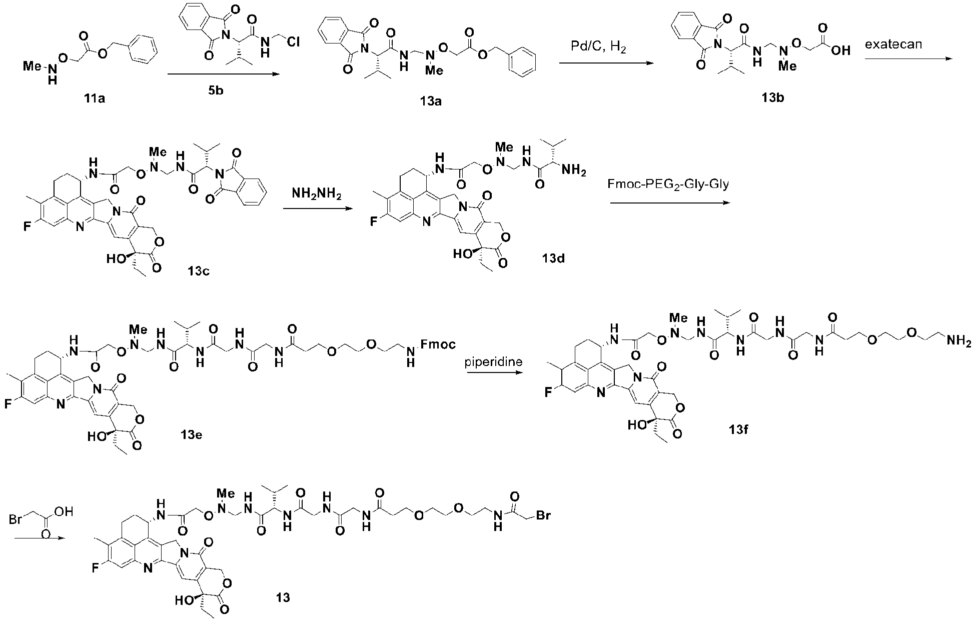

**5b** (0.2 g, 0.68 mmol) was dissolved in DCM (5 mL), then **11a** (133 mg, 0.68 mmol) and DIPEA (175 mg, 1.36 mmol) were added. The mixture was sealed in a tube, heated to 50°C and reacted overnight, cooled to room temperature, concentrated, and purified by silica gel column chromatography to afford the product **13a** (0.27 g, yield 90%, HPLC 93%); LCMS: (M+1)⁺ 454.11 (theoretical value: 453.19).

In a reaction flask, **13a** (0.27 g, 0.59 mmol), methanol (5 mL), and 10% palladium on carbon (0.1 g) were added. After purging with hydrogen, the mixture was reacted at room temperature and atmospheric pressure for 2 h, filtered, and concentrated to afford the product **13b** (0.20 g, yield 95%, HPLC 91%); LCMS: (M+1)+ 364.03 (theoretical value: 363.14).

Exatecan mesylate (0.26 g, 0.5 mmol) was taken and dissolved in DMF (2 mL), then HATU (0.19 g, 0.5 mmol), DIPEA (0.129 g, 1 mmol), and **13b** (0.2 g, 0.55 mmol) were added. The mixture was stirred to react at room temperature for 15 h, diluted with DCM (200 mL) washed with saturated sodium chloride solution, dried over anhydrous sodium sulfate, and purified by silica gel column chromatography to afford **13c** as a yellow solid (0.32 g, yield 84%, HPLC 94%); LCMS: (M+1)⁺781.12 (theoretical value: 780.29).

**13c** (0.1 g, 0.12 mmol) was dissolved in MeOH (5 mL), then hydrazine hydrate (66 mg, 1.32 mmol) was aadded. The mixture was sealed in a tube, heated to 60°C and reacted for 2 h, and cooled to room temperature. The reaction solution was poured into saturated brine (100 mL) and extracted with ethyl acetate (200 mL), then concentrated to afford **13d** (0.06 g, yield 72%, HPLC 90%); LCMS: (M+1)⁺ 651.06 (theoretical value: 650.29).

**13d** (60 mg, 0.092 mmol) was weighted and dissolved in DMF (2 mL), DIPEA (24 mg, 0.184 mmol) was added. Under stirring, Fmoc-PEG₂-Gly-Gly-OH (47.3 mg, 0.092 mmol) and HATU (35.07 mg, 0.092 mmol) were added in sequence. The mixture was stirred to react at room temperature for 1 h, concentrated, and purified by silica gel column chromatography to afford Compound **13e** (86 mg, yield 81%, HPLC 91%); LCMS: (M+1)⁺ 1146.28 (theoretical value: 1145.49).

To a reaction flask, **13e** (86 mg, 0.075 mmol), DMF (1 mL), and piperidine (100 µL) were added. The mixture was stirred to react at room temperature for 1 h, then methyl tert-butyl ether (20 mL) was added. The mixture was centrifuged, the supernatant was removed, and the residue was dried to afford **13f** (36 mg, yield 52%, HPLC purity 89%). LCMS: (M+1)⁺ 924.00 (theoretical value: 923.42).

To a reaction flask, DCM (3 mL), **13f** (36 mg, 0.039 mmol), bromoacetic acid (10.6 mg, 0.078 mmol), and DIC (9.8 mg, 0.078 mmol) were added in sequence. The mixture was stirred to react at room temperature for 90 min, concentrated, and purified by silica gel column chromatography to afford **13** as a yellow solid (17 mg, yield 42.5%, HPLC 95%); LCMS: (M+1)⁺ 1044.25 (theoretical value: 1043.34).

ADC-13a: Compound **13** and HER2 monoclonal antibody HS627 form an antibody-drug conjugate

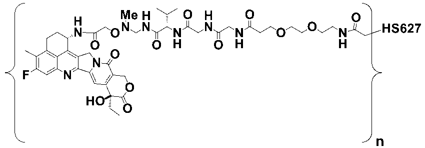

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **13** (0.83mg, 0.8mmol) was dissolved in 0.08 mL of DMA, and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose, 0.2g/L polysorbate 20, to afford ADC-13a (3.1mg/mL, 2mL).

UV-HPLC calculated average value: n=7.6

ADC-13b: Compound **13** and 5T4 monoclonal antibody IP140B form an antibody-drug conjugate

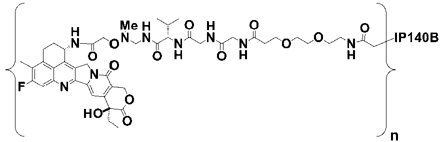

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **13** (0.83mg, 0.8mmol) was dissolved in 0.08 mL of DMA, and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH5.7, to afford ADC-13b (3.2mg/mL, 2mL).

UV-HPLC calculated average value: n=7.4

### Example 14: 2-(N-methyl-N-(bromoacetamidoethoxyethoxypropionyl-Gly-Leu-Val-methylene)amino)oxyacety exatecan (14)

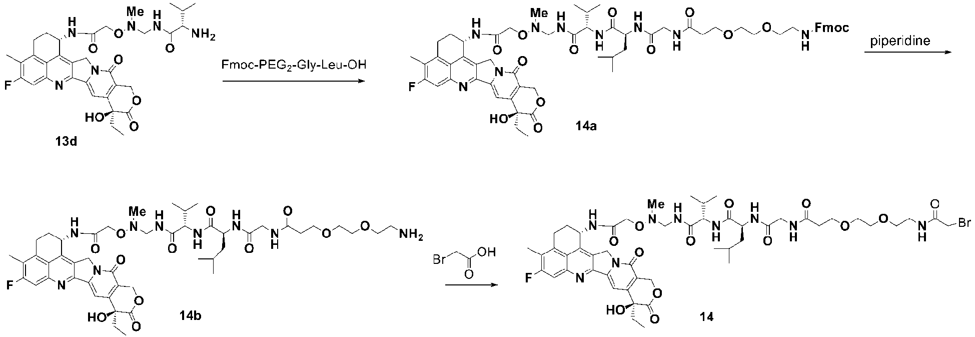

**13a** (0.10 g, 0.15 mmol) was taken and dissolved in DMF (2 mL), HATU (1.17 g, 0.31 mmol), DIPEA (0.4 g, 0.31 mmol), and Fmoc-PEG₂-Gly-Leu-OH (176 mg, 0.31 mmol) were added. The mixture was stirred to react at room temperature for 15 h, DCM (200 mL) was added. Then the mixture was washed with saturated sodium chloride solution, dried over anhydrous sodium sulfate, and purified by silica gel column chromatography to afford **14a** as a yellow solid (150 mg, yield 83%, HPLC 96%); LCMS: (M+1)⁺ 1202.35 (theoretical value: 1201.55).

**14a** (150 mg, 0.12 mmol) was dissolved in DMF (0.5 mL), then piperidine (90 mg, 1.2 mmol) was added. The mixture was stirred to react at room temperature for 1 h, then methyl tert-butyl ether (30 mL) was added. The mixture was centrifuged, the supernatant was removed, the solvent was evaporated under reduced pressure, and the residue was dried to afford **14b** as a solid (63 mg, yield 49%, HPLC 93%) which was directly used in the next step; LCMS: (M+1)⁺980.11 (theoretical value: 979.48).

To a reaction flask, DCM (3 mL), **14b** (63 mg, 0.064 mmol), bromoacetic acid (17 mg, 0.128 mmol), and DIC (16.2 mg, 0.128 mmol) were added in sequence. The mixture was stirred to react at room temperature for 90 min, concentrated, and purified by silica gel column chromatography to afford 14 as a yellow solid (34 mg, yield 49%, HPLC 95%); LCMS: (M+1)⁺ 1100.04 (theoretical value: 1099.40).

ADC-14a: Compound **14** and HER2 monoclonal antibody HS627 form an antibody-drug conjugate

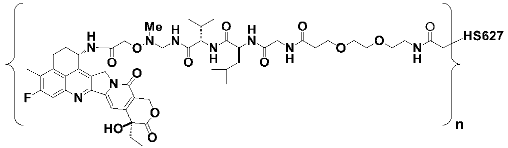

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **14** (0.88mg, 0.8mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose, 0.2g/L polysorbate 20, to afford ADC-14a (3.2mg/mL, 2mL).

UV-HPLC calculated average value: n=7.7

ADC-14b: Compound **14** and 5T4 monoclonal antibody IP140B form an antibody-drug conjugate

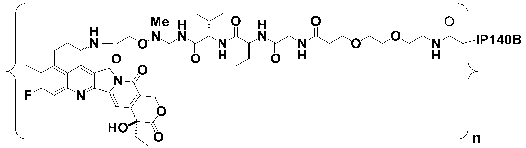

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **14** (0.88mg, 0.8mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH5.7, to afford ADC-14b (3.2mg/mL, 2mL).

UV-HPLC calculated average value: n=7.4

### Example 15: 2-(N-methyl-N-(bromoacetamidoethoxyethoxypropionyl-Gly-Phe-Gly-methylene)amino)oxyacety exatecan (15)

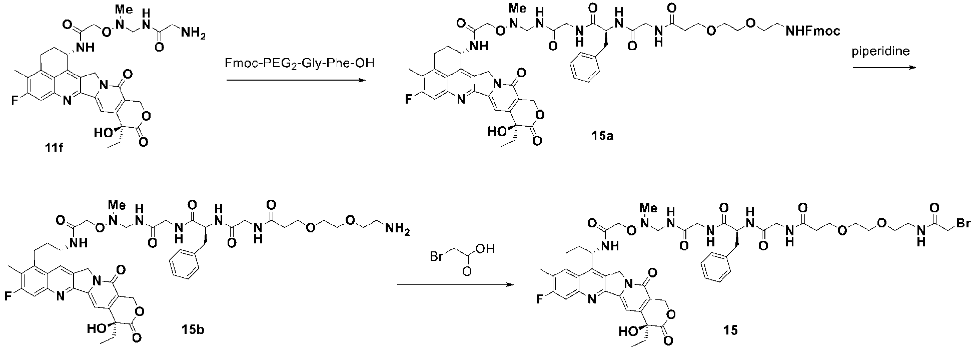

**11f** (60 mg, 0.098 mmol) was weighted and dissolved in DMF (2 mL), DIPEA (25 mg, 0.197 mmol) wasa added. Under stirring, Fmoc-PEG₂-Gly-Phe-OH (59.02 mg, 0.098 mmol) and HATU (37.5 mg, 0.098 mmol) were added in sequence. The reaction solution was stirred to react at room temperature for 1 h, concentrated, and purified by silica gel column chromatography to afford Compound **15a** (92 mg, yield 79%, HPLC 93%); LCMS: (M+1)⁺ 1194.36 (theoretical value: 1193.49).

To a reaction flask, **15a** (92 mg, 0.077 mmol), DMF (1 mL), and piperidine (100 µL) were added. The reaction solution was stirred to react at room temperature for 1 h, then methyl tert-butyl ether (20 mL) was added. The mixture was centrifuged, the supernatant was removed, and the residue was dried to afford **15b** (45 mg, yield 60%, HPLC 92%); LCMS: (M+1)⁺972.38 (theoretical value: 971.42).

To a reaction flask, DCM (3 mL), **15b** (45 mg, 0.046 mmol), bromoacetic acid (12.60 mg, 0.092 mmol), and DIC (11.67 mg, 0.092 mmol) were added in sequence. The reaction solution was stirred to react at room temperature for 90 min, concentrated, and purified by silica gel column chromatography to afford **15** as a yellow solid (15 mg, yield 30%, HPLC 94%); LCMS: (M+1)⁺ 1092.28 (theoretical value: 1091.34).

ADC-15a: Compound **15** and HER2 monoclonal antibody HS627 form an antibody-drug conjugate

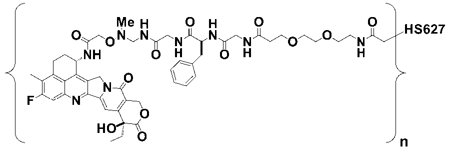

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **15** (0.87mg, 0.8mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose, 0.2g/L polysorbate 20, to afford ADC-15a (3.2mg/mL, 2mL).

UV-HPLC calculated average value: n=7.7

ADC-15b: Compound **15** and 5T4 monoclonal antibody IP140B form an antibody-drug conjugate

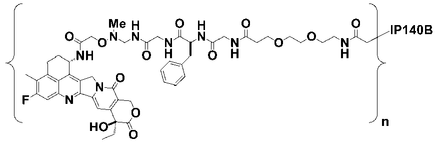

IP140B antibody (20.0mg/mL, 10mg, 0.066mmol),was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **15** (0.87mg, 0.8mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH 5.7, to afford ADC-15b (3.2mg/mL, 2mL).

UV-HPLC calculated average value: n=7.6

### Example 16: 2-(N-methyl-N-(bromoacetamidoethoxyethoxypropionyl-Gly-Val-Ala-Gly-methylene)amino)oxy acetyexatecan (16)

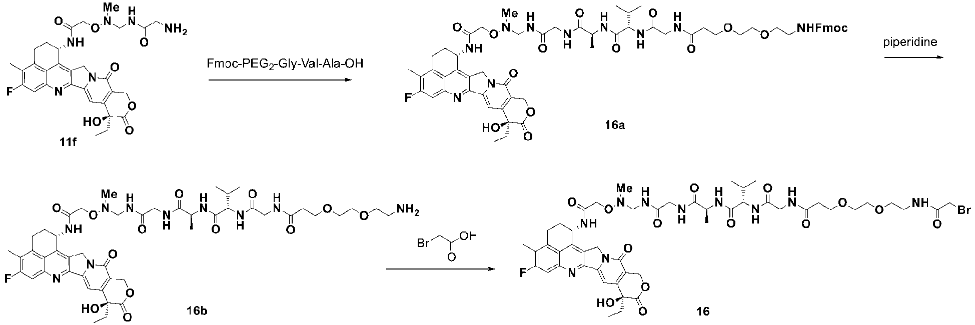

**11f** (50 mg, 0.082 mmol) was weighted and dissolved in DMF (2 mL), DIPEA (10.578 mg, 0.082 mmol) was added. After the mixture became clear under stirring, Fmoc-PEG₂-Gly-Val-Ala-OH (51.41 mg, 0.082 mmol) and HATU (32 mg, 0.082 mmol) were added in sequence. The mixture was stirred to react at room temperature for 1 h and purified by silica gel column chromatography to afford Compound **16a** (79 mg, yield 78%, HPLC 96%); LCMS: (M+1)⁺ 1216.52 (theoretical value: 1217.32).

In a 10 mL single-necked flask, **16a** (79 mg, 0.064 mmol) was dissolved in DMF (1 mL), 100 µL of piperidine was added, and the mixture was reacted at room temperature for 1 h. Methyl tert-butyl ether (20 mL) was then added, and the mixture was centrifuged. The supernatant was removed, and the resulting solid was dissolved in a small amount of methanol, dried to afford **16b** (26 mg, yield 45%, HPLC 95%); LCMS: (M+1)⁺994.46 (theoretical value: 995.08).

In a 10 mL single-necked flask, DCM (3 mL), **16b** (26 mg, 0.026 mmol), bromoacetic acid (7.2 mg, 0.052 mmol), and DIC (6.6 mg, 0.052 mmol) were added in sequence. The mixture was stirred to react at room temperature for 90 min, concentrated, and purified by silica gel column chromatography to afford **16** as a yellow solid (12 mg, yield 41%, HPLC 98.5%); LCMS: (M+1)⁺ 1114.38 (theoretical value: 1115.01).

ADC-16a: Compound **16** and HER2 monoclonal antibody HS627 form an antibody-drug conjugate

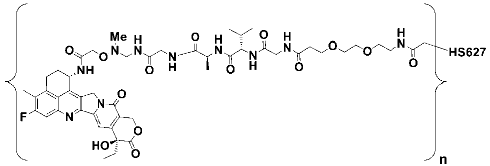

HS627 antibody (10.0 mg/mL, 10 mg, 0.066mmol) was taken and adjusted pH to 7.2 with 1 M Na₂HPO₄ solution. Then, a solution of 5 mM disodium ethylenediaminetetraacetate (20 mg/mL, 84 µL) was added, then a solution of 6eq prepared TCEP·HCl (tris(2-carboxyethyl)phosphine hydrochloride) (10 mM, 0.04 mL) was added. The mixture was rotated to react at 25°C for 90 min.

Compound **16** (0.89 mg, 0.8 mmol) was dissolved in DMA (0.09 mL), and added to the above solution system. After mixing well, the mixture was rotated to react at room temperature for 16 h. After the reaction was completed, the buffer was exchanged using a NAP-5 gel column (Cytiva) into 20 mM L-histidine acetate buffer, pH 6.0, 120 mM sucrose, 0.02% Tween 80, to afford ADC-16a (3mg/mL, 2mL).

UV-HPLC calculated average value: n=7.80

ADC-16b: Compound **16** and 5T4 monoclonal antibody IP140B form an antibody-drug conjugate

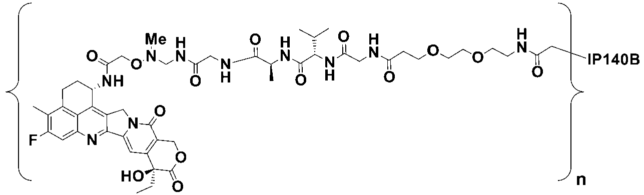

IP140B antibody (10.0 mg/mL, 10mg, 0.066 mmol) was taken and adjusted pH to 7.2 with 1 M Na₂HPO₄ solution. Then, a solution of 5 mM disodium ethylenediaminetetraacetate (20 mg/mL, 84 µL) was added, then a solution of 6eq prepared TCEP·HCl (tris(2-carboxyethyl)phosphine hydrochloride) (10 mM, 0.04 mL) was added. The mixture was rotated to react at 25°C for 90 min.

Compound **16** (0.89 mg, 0.8 mmol) was dissolved in DMA (0.09 mL), and added to the above solution system. After mixing well, the mixture was rotated to react at room temperature for 16 h After the reaction was completed, the buffer was exchanged using a NAP-5 gel column (Cytiva) into 20 mM L-histidine acetate buffer, pH 6.0, 120 mM sucrose, 0.02% Tween 80, to afford ADC-16b (2.9mg/mL, 2mL).

UV-HPLC calculated average value: n=7.78

### Assay 1: In vivo inhibitory activity of ADC against tumor growth

Method for *in vivo* inhibitory activity assay of ADC: Human non-small cell lung cancer Calu-6, Human lung cancer cell NCI-H1975, OE-21 esophageal adenocarcinoma, Human esophageal squamous cell carcinoma KYSE-150, Human ovarian cancer cell ES-2 and Human breast cancer cell MDA-MB-231 were cultured *in vitro* as a monolayer. When the cell saturation was 80%-90%, they were digested with trypsin-EDTA, centrifuged, and the supernatant was discarded. The cells were resuspended with PBS, and the cell suspension was adjusted to a suitable concentration. Calu-6, NCI-H1975, KYSE-150, OE-21, and MDA-MB-231 cells (2-10 x 10⁶ cells/0.1 mL) were subcutaneously inoculated into BALB/c nude mice, ES-2 cells were subcutaneously inoculated into NOD-SCID mice. The animals and the growth of transplanted tumors were regularly observed. When the tumor volume was grown to about 100-200 mm³, random grouping was performed according to tumor volume and body weight, namely the vehicle control group (normal saline) and the ADC administration group (dissolved in normal saline), with 6 animals per group. Administration was performed by intravenous injection at a dosing frequency of Q4D for a total of 2 times (the first administration was recorded as Day 1, and the second administration was on Day 5). Experimental grouping and dosing settings were shown in Table 1. Tumor major diameter a (mm), minor diameter b (mm), and mice body weight were measured with a vernier caliper twice a week. Tumor volume (V) was calculated according to the following formula: V = 1/2 x a x b²(mm³), wherein a and b represented the tumor length and width, respectively. Growth curves were plotted, and finally, tumors were excised and weighed. Statistical analysis was performed using GraphPad Prism software based on tumor volume and body weight data of tumor-bearing mice at the end of the trial to afford tumor inhibition results. In the figures, "\" in the control group indicates no results, with tumor status unmeasured; the experimental group had no corresponding tumors. "Tumor volume reduced to 0" and "tumor disappeared to 0" indicates no tumor residue was found in the corresponding dissected animals.

**Table 1: Mouse Grouping and Administration Settings**

| Cell Model | Group/Drug Injected | Administration Dose | Tumor Growth Curves | Photographs of Tumors after Dissection |
|---|---|---|---|---|
| NCI-H1975 | Vehicle control | - | Figure 1 | Figure 2 |
| | group | | | |
| | ADC Dxd | 3mg/kg weight | | |
| | | 10mg/kg weight | | |
| | ADC 3b | 3mg/kg weight | | |
| | | 6mg/kg weight | | |
| NCI-H1975 | Vehicle control group | - | Figure 3 | Figure 4 |
| | ADC Dxd | 2mg/kg weight | | |
| | | 5mg/kg weight | | |
| | ADC 11b | 2mg/kg weight | | |
| | | 5mg/kg weight | | |
| | ADC 15b | 2mg/kg weight | | |
| | | 5mg/kg weight | | |
| Calu-6 | Vehicle control group | - | Figure 5 | Figure 6 |
| | ADC Dxd | 3mg/kg weight | | |
| | | 10mg/kg weight | | |
| | ADC 16b | 3mg/kg weight | | |
| | | 10mg/kg weight | | |
| KYSE-150 | Vehicle control group | - | Figure 7 | Figure 8 |
| | ADC Dxd | 3mg/kg weight | | |
| | | 10mg/kg weight | | |
| | ADC 16b | 3mg/kg weight | | |
| | | 10mg/kg weight | | |
| KYSE-150 | Vehicle control group | - | Figure 9 | Figure 10 |
| | ADC Dxd | 2mg/kg weight | | |
| | | 5mg/kg weight | | |
| | ADC 11b | 2mg/kg weight | | |
| | | 5mg/kg weight | | |
| | ADC 15b | 2mg/kg weight | | |
| | | 5mg/kg weight | | |
| | ADC 2b | 5mg/kg weight | | |
| ES-2 | Vehicle control group | - | Figure 11 | Figure 12 |
| | ADC Dxd | 3mg/kg weight | | |
| | | 10mg/kg weight | | |
| | ADC 16b | 3mg/kg weight | | |
| | | 10mg/kg weight | | |
| OE-21 | Vehicle control group | - | Figure 13 | Figure 14 |
| | ADC Dxd | 2mg/kg weight | | |
| | | 5mg/kg weight | | |
| | ADC 7b | 2mg/kg weight | | |
| | | 5mg/kg weight | | |
| | ADC 9b | 2mg/kg weight | | |
| | | 5mg/kg weight | | |
| | ADC 8b | 2mg/kg weight | | |
| | | 5mg/kg weight | | |
| MDA-MB-231 | Vehicle control group | - | Figure 15 | Figure 16 |
| | ADC Dxd | 2mg/kg weight | | |
| | | 5mg/kg weight | | |
| | ADC 11b | 2mg/kg weight | | |
| | | 5mg/kg weight | | |
| | ADC 15b | 2mg/kg weight | | |
| | | 5mg/kg weight | | |
| | ADC 2b | 2mg/kg weight | | |
| | | 5mg/kg weight | | |
| | ADC 10b | 2mg/kg weight | | |
| | | 5mg/kg weight | | |

The above descriptions of specific exemplary embodiments of the present disclosure are for purposes of illustration and exemplification. These descriptions are not intended to limit the present disclosure to the precise forms disclosed, and it is apparent that many changes and modifications can be made in light of the above teachings. The purpose of selecting and describing exemplary embodiments is to explain the specific principles of the present disclosure and practical applications thereof, thereby enabling those skilled in the art to implement and utilize various exemplary embodiments of the present disclosure, along with various selections and modifications. The scope of the present disclosure is intended to be defined by the claims and equivalents thereof.

## Claims

1. An antibody-drug conjugate represented by Formula (I), 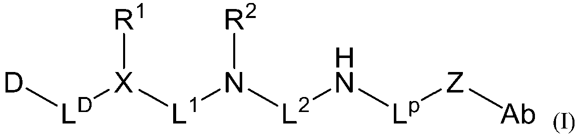 in the Formula,
D is a cytotoxic drug, Ab is an antibody;
L^{D} is selected from nonexistence or C₁-C₃ alkylene;
X is selected from N, O or S;
R¹ is selected from nonexistence, hydrogen, deuterium, C₁-C₆ alkyl or halogen-substituted C₁-C₆ alkyl;
R² is selected from hydrogen, deuterium, C₁-C₆ alkyl, C₁-C₆ alkoxyl, acyl, sulfonyl;
L¹ is selected from -L¹¹-L¹²-L¹³-; wherein, L¹¹, L¹², L¹³ are each independently selected from nonexistence, -C=O-, C₁-C₆ alkylene or -O-C₁-C₆ alkylene;
L² is selected from C₁-C₆ alkylene or C₁-C₆ acyl, the C₁-C₆ alkylene or C₁-C₆ acyl is optionally substituted with one or more R³;
R³ is selected from phenyl-substituted or unsubstituted C₁-C₆ alkyl, C₁-C₆ alkoxyl;
L^{P} is a peptide residue consisting of 2 to 7 amino acids;
Z is selected from -L^{z}-L^{j}-, wherein, L^{z} is selected from -C(=O)-C₁-C₈ alkylene or -C(=O)-(CH₂CH₂O)₂₋₆-CH₂CH₂NH-, L^{j} is a linker that can conjugate to an antibody.

2. The antibody-drug conjugate according to claim 1, wherein, R¹ is selected from nonexistence, hydrogen, deuterium, C₁-C₃ alkyl or halogen-substituted C₁-C₃ alkyl;
preferably, R¹ is selected from nonexistence, hydrogen, deuterium or halogen-substituted C₁-C₃ alkyl;
preferably, R¹ is selected from nonexistence, hydrogen, deuterium or a C₁-C₃ alkyl substituted by F, Cl, Br, I;
preferably, R¹ is selected from nonexistence, hydrogen, deuterium or F-substituted C₁-C₃ alkyl;
preferably, R¹ is selected from nonexistence, hydrogen, deuterium, fluoromethyl, difluoromethyl, trifluoromethyl, fluoroethyl, difluoroethyl or trifluoroethyl;
preferably, R¹ is selected from nonexistence, hydrogen, difluoroethyl or trifluoroethyl;
preferably, R² is selected from hydrogen, deuterium, C₁-C₆ alkyl, C₁-C₆ alkoxyl, -C(=O)C₁-C₆ alkyl or -S(=O)₂C₁-C₆ alkyl;
preferably, R² is selected from hydrogen, deuterium, C₁-C₆ alkyl, C₁-C₆ alkoxyl, -C(=O)C₁-C₆ alkyl or -S(=O)₂C₁-C₃ alkyl;
preferably, R² is selected from hydrogen, deuterium, C₁-C₃ alkyl, C₁-C₃ alkoxyl, -C(=O)C₁-C₃ alkyl or -S(=O)₂C₁-C₃ alkyl;
preferably, R² is selected from hydrogen, deuterium, methyl, ethyl, methoxy, ethoxy, formyl, acetyl, methanesulfonyl or ethylsulfonyl;
preferably, R² is selected from hydrogen, methyl, methoxy, formyl or methanesulfonyl;
preferably, R² is selected from hydrogen, methyl, formyl or methanesulfonyl.

3. The antibody-drug conjugate according to claim 1 or 2, wherein, L¹¹, L¹², L¹³ are each independently selected from nonexistence, -C=O-, C₁-C₂ alkylene or C₁-C₂ alkylene-O-, provided that , when L¹¹ is-C=O-, R² is not hydrogen;
preferably, L¹¹, L¹², L¹³ are each independently selected from nonexistence, -C=O-, -CH₂- or - OCH₂-;
preferably, L¹ is selected from -C(=O)CH₂OCH₂-, -C(=O)CH₂O- or -CH₂-;
preferably, L² is selected from C₁-C₃ alkylene or C₁-C₃ acyl, the C₁-C₃ alkylene or C₁-C₃ acyl is optionally substituted with one or more R³;
preferably, L² is selected from methylene, ethylene or -C(=O)CH₂-, the methylene, ethylene or - C(=O)CH₂-is optionally substituted with one or more R³;
preferably, R³ is selected from C₁-C₆ alkyl, C₁-C₆ alkoxyl or phenyl-substituted C₁-C₆ alkyl;
preferably, R³ is selected from C₁-C₆ alkyl or phenyl-substituted C₁-C₆ alkyl;
preferably, R³ is selected from C₁-C₄ alkyl or phenyl-substituted C₁-C₃ alkyl;
preferably, R³ is selected from methyl, ethyl, propyl, isopropyl, butyl, isobutyl, phenylmethyl or phenylethyl;
preferably, R³ is selected from methyl, isopropyl, isobutyl or phenylmethyl;
preferably, L² is selected from -CH₂-, -CH₂CH₂-, 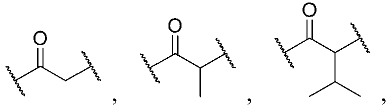 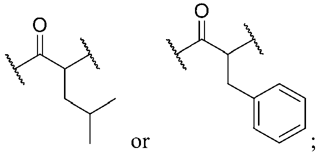
preferably, L² is selected from -CH₂-, -CH₂CH₂-, 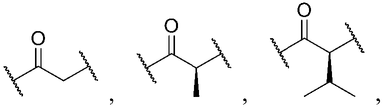 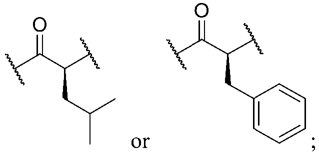
preferably, L² is selected from -CH₂-, -CH₂CH₂-, 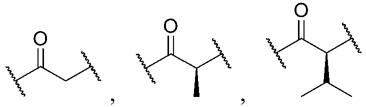 or

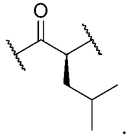

4. The antibody-drug conjugate according to any one of claims 1-3, wherein, the amino acid is selected from phenylalanine (Phe), glycine (Gly), valine (Val), alanine (Ala), leucine (Leu), lysine (Lys) or glutamate (Glu);
preferably, the amino acid is selected from phenylalanine (Phe), glycine (Gly), valine (Val), alanine (Ala) or leucine (Leu);
preferably, L^{p} is selected from peptide residues consisting of 2-5 amino acids selected from phenylalanine (Phe), glycine (Gly), valine (Val), alanine (Ala) or leucine (Leu);
preferably, L^{p} is selected from peptide residues consisting of 2, 3 or 4 amino acids selected from phenylalanine (Phe), glycine (Gly), valine (Val), alanine (Ala) or leucine (Leu);
preferably, L^{p} is selected from -Val-Cit-, -Gly-Lys-, -Gly-Leu-, -Val-Ala-, -Gly-Phe-, -GLy-Gly-Lys-, -Gly-Gly-Phe-, -Gly-Val-Ala-, -Gly-Gly-Val-, -Gly-Leu-Val-, -Gly-Phe-Gly- or -Gly-Gly-Leu-;
preferably, L^{p} is selected from -Gly-Leu-, -Gly-Phe-, -Gly-Gly-Phe-, -Gly-Val-Ala-, -Gly-Gly-Val-, -Gly-Leu-Val-, -Gly-Phe-Gly- or -Gly-Gly-Leu-;
preferably, L^{p} is selected from 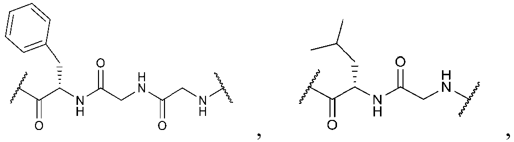 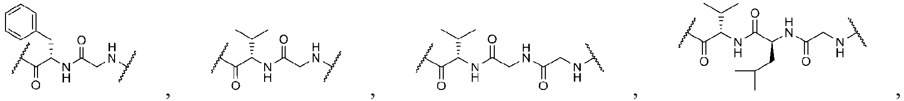 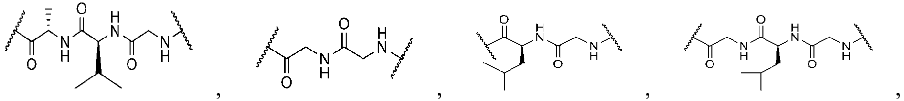 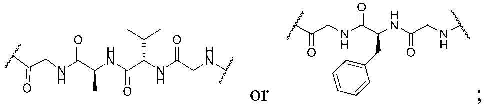
preferably, L^{j} is selected from 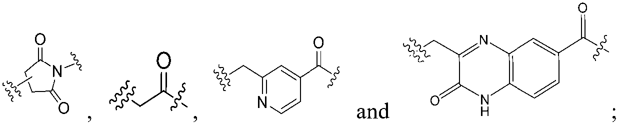 the position represented by 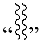 is linked to the antibody, and the position represented by 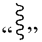 is linked to the L^{Z} group;
preferably, L^{z} is selected from -C(=O)-C₁-C₈ alkylene or -C(=O)-(CH₂CH₂O)₂₋₆-CH₂CH₂NH-;
preferably, L^{z} is selected from -C(=O)-(CH₂CH₂O)₂₋₆-CH₂CH₂NH-;
preferably, L^{z} is selected from -C(=O)-(CH₂CH₂O)₂₋₄-CH₂CH₂NH-;
preferably, L^{z} is selected from -C(=O)-(CH₂CH₂O)₂-CH₂CH₂NH-;
preferably, Z is selected from

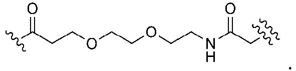

5. The antibody-drug conjugate according to any one of claims 1-4, the cytotoxic drug is selected from camptothecin and derivatives thereof;
preferably, the cytotoxic drug is selected from 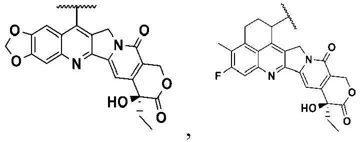 or 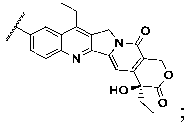
preferably, the antibody is an antibody against tumor-associated antigen;
preferably, the antibody against tumor-associated antigen is selected from anti-Her2 antibody, anti-Trop2 antibody, anti-B7H3 antibody, anti-5T4 antibody, anti-Nectin-4 antibody, anti-CD20 antibody, and anti-ROR1 antibody.

6. The antibody-drug conjugate according to any one of claims 1-5, the compound structure represented by Formula (I) is as represented by Formula (II), Formula (III), or Formula (IV): 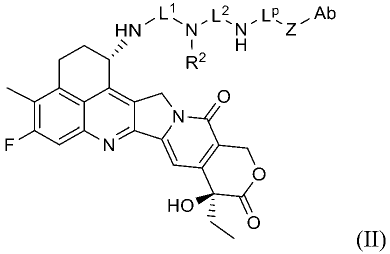
in Formula (II), R², L¹, L², L^{p}, Z, Ab are each defined the same as the antibody-drug conjugate of Formula (I);
preferably, L¹ is selected from -C(=O)CH₂OCH₂- or -C(=O)CH₂O-;
preferably, L² is selected from -CH₂- or -CH₂CH₂-;
preferably, 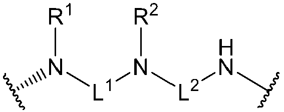 is selected from 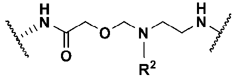 or 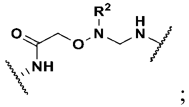 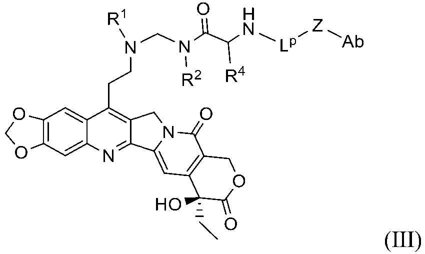
in Formula (III), R¹, R², R⁴, L^{p}, Z, Ab are each defined as the antibody-drug conjugate of Formula (I);
preferably, R⁴ is selected from hydrogen, phenyl-substituted or unsubstituted C₁-C₆ alkyl, C₁-C₆ alkoxyl;
preferably, R⁴ is selected from hydrogen, C₁-C₆ alkyl, C₁-C₆ alkoxyl or phenyl-substituted C₁-C₆ alkyl;
preferably, R⁴ is selected from hydrogen, C₁-C₆ alkyl or phenyl-substituted C₁-C₆ alkyl;
preferably, R⁴ is selected from hydrogen, C₁-C₄ alkyl or phenyl-substituted C₁-C₃ alkyl;
preferably, R⁴ is selected from hydrogen, methyl, ethyl, propyl, isopropyl, butyl, isobutyl, phenylmethyl or phenylethyl;
preferably, R⁴ is selected from hydrogen, methyl, isopropyl, isobutyl or phenylmethyl;
preferably, R⁴ is selected from hydrogen, methyl or isopropyl; 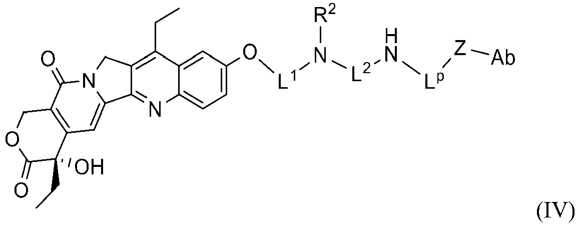
in Formula (IV), R², L¹, L², L^{p}, Z, Ab are each defined the same as the antibody-drug conjugate of Formula (I);
preferably, L¹, L² are each independently selected from C₁-C₃ alkylene;
preferably, L¹ is -CH₂-, L² is -CH₂CH₂-.

7. The antibody-drug conjugate according to any one of claims 1-6, wherein, 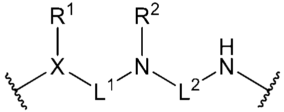 is selected from the following structure: 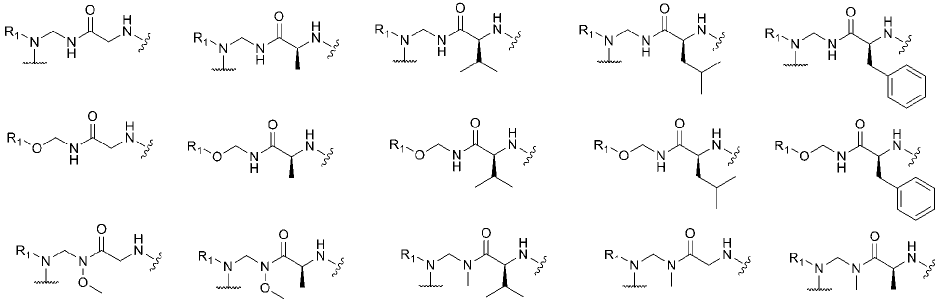 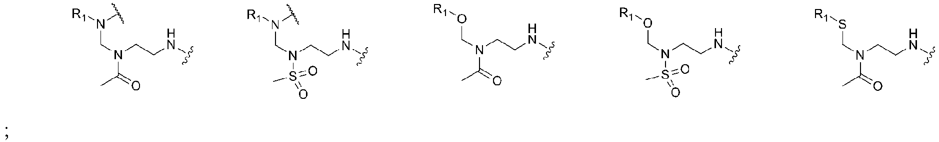 wherein, R¹ is defined the same as the antibody-drug conjugate of Formula (I).

8. The antibody-drug conjugate according to any one of claims 1-7, which is afforded by linking the following compound with an antibody: 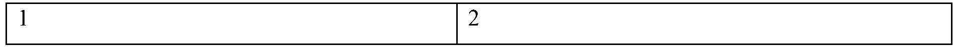 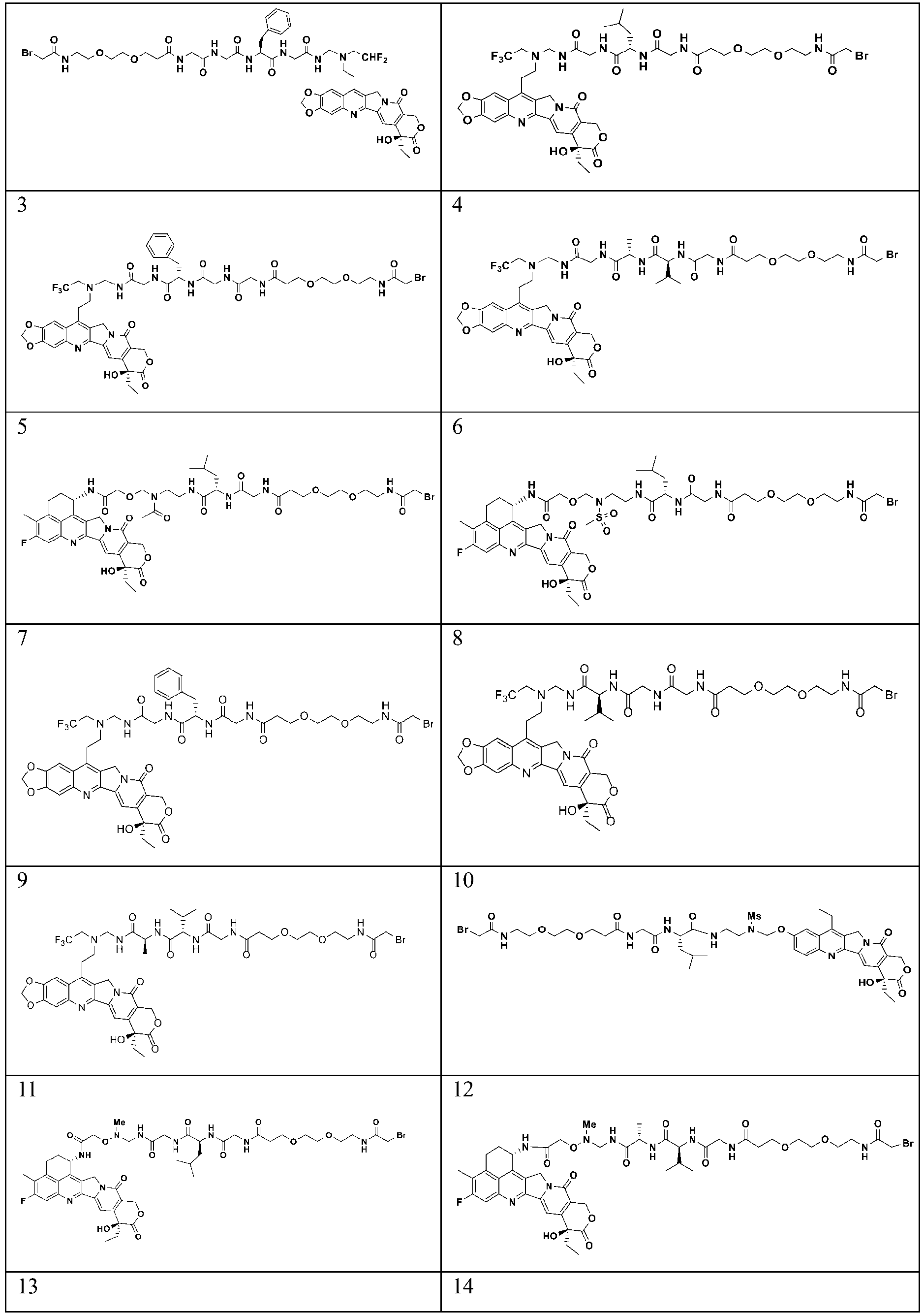 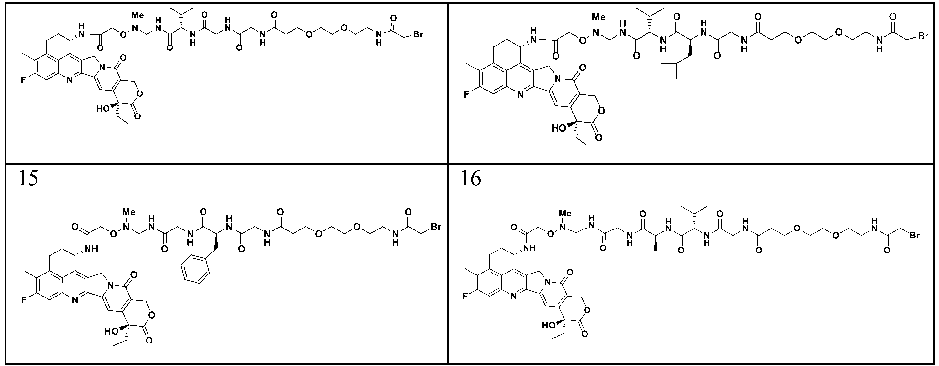 preferably, the antibody is HS627 antibody or IP140B antibody, the heavy chain amino acid sequence of HS627 is as set forth in SEQ ID NO: 1, and the light chain amino acid sequence thereof is as set forth in SEQ ID NO: 2; the heavy chain amino acid sequence of the IP140B antibody is as set forth in SEQ ID NO: 3, and the light chain amino acid sequence thereof is as set forth in SEQ ID NO: 4.

9. A pharmaceutical composition comprising the antibody-drug conjugate according to any one of claims 1-8 and a pharmaceutically acceptable carrier.

10. Use of the antibody-drug conjugate according to any one of claims 1-8 or the pharmaceutical composition according to claim 9 in the manufacture of an anti-tumor medicament;
preferably, the tumor is selected from a solid tumor, more preferably selected from lung cancer, non-small cell lung cancer, esophageal squamous cell carcinoma, ovarian cancer, esophageal adenocarcinoma or breast cancer.

11. A method for treating a tumor disease, comprising the step of administering to a patient in need thereof the antibody-drug conjugate according to any one of claims 1-8 or the pharmaceutical composition according to claim 9.
